# FASCICULE DE BREVET EUROPEEN

(11) **EP 2 521 530 B1**
(45) Date de publication et mention de la délivrance du brevet: **26.06.2019**
(21) Numéro de dépôt: 11704248.1
(22) Date de dépôt: 04.01.2011
(51) Int. Cl.: A61K 8/49, A61Q 5/04, A61Q 5/06, A61Q 5/12, C07D 239/47, A61Q 5/00

(54) **COMPOSITION COSMÉTIQUE, PROCÉDÉ DE TRAITEMENT COSMÉTIQUE, KIT ET COMPOSÉ**
KOSMETIKZUSAMMENSETZUNG, KOSMETISCHES BEHANDLUNGSVERFAHREN, KIT UND VERBINDUNG
COSMETIC COMPOSITION, COSMETIC TREATMENT METHOD, KIT, AND COMPOUND

(30) Priorité: 05.01.2010 US 292255 P; 04.01.2010 FR 1050003
(43) Date de publication de la demande: 14.11.2012
(73) Titulaire: L'Oréal, 75008 Paris (FR)
(72) Inventeur: DUBLANCHET, Anne-Claude, F-92160 Antony (FR); BLAISE, Christian, F-94160 Saint Mande (FR); MESSAVUSSU, Abel, F-94210 Fontenay Sous Bois (FR)
(74) Mandataire: Boubel, Thomas
(86) Numéro de dépôt international: PCT/FR2011/050005
(87) Numéro de publication internationale: WO 2011/080494

(56) Documents cités:
- EP-A2- 1 970 099
- FR-A1- 2 862 218
- JP-A- 2005 300 817
- US-A1- 2006 018 856
- US-A1- 2007 093 639
- US-A1- 2009 081 145
- US-A1- 2009 130 172
- US-B1- 6 320 018
- RIETH L R ET AL: "Polymerization of ureidopyrimidinone-functionalized olefines by using late-transition metal Ziegler-Natta catalysts: synthesis of thermoplastic elastomeric polyolefins", ANGEWANDTE CHEMIE. INTERNATIONAL EDITION, WILEY VCH VERLAG, WEINHEIM LNKD- DOI:10.1002/1521-3773(20010601)40:11<2153: :AID-ANIE2153>3.0.CO;2-W, vol. 40, no. 11, 1 janvier 2001 (2001-01-01), pages 2153-2156, XP002260388, ISSN: 1433-7851
- Rint P. Sijbesma et al.: "Reversible Polymers Formed from Self-Complementary Monomers Using Quadruple Hydrogen Bonding", Science, vol. 278 28 novembre 1997 (1997-11-28), pages 1601-1604, XP002605246, DOI: 10.1126/science.278.5343.1601 Extrait de l'Internet: URL:http://www.sciencemag.org/cgi/reprint/ 278/5343/1601.pdf [extrait le 2010-10-07]
- FELIX H. BEIJER, RINT P. SIJBESMA, HUUB KOOIJMAN, ANTHONY L. SPEK, AND E.W. MEIJER: "Strong Dimerization of Ureidopyrimidones via Quadruple Hydrogen Bonding", JOURNAL OF THE AMERICAN CHEMICAL SOCIETY, vol. 120, 27 juin 1998 (1998-06-27), pages 6761-6769, XP002605247,
- Bas W.T. Gruijters et al.: "Catalyst Recycling via Hydrogen-Bonding-Based Affinity Tags", Organic Letters, vol. 8, no. 15 22 juin 2006 (2006-06-22), 2006, pages 3163-3166, XP002605248, DOI: 10.1021/ol0607387 Extrait de l'Internet: URL:http://pubs.acs.org/doi/suppl/10.1021/ ol0607387 [extrait le 2010-10-06]
- Alfred Kreutzberger, Horst Schimmelpfennig: "2-Ureido-4(3H)-pyrimidinone", Archiv der Pharmazie, vol. 314, no. 1 1981, 1981, pages 34-41, XP002605249, DOI: 10.1002/ardp.19813140107 Extrait de l'Internet: URL:http://onlinelibrary.wiley.com/doi/10. 1002/ardp.19813140107/pdf [extrait le 2010-10-08]
- Franz Pohl: "Untersuchungen aus dem organ.-chem. Laboratorium der Technischen Hochschule zu Dresden, LXXXVII. Zur Kenntnis des Dicyandiamids", Journal für Praktische Chemie, vol. 77, no. 1 14 mai 1908 (1908-05-14), pages 533-548, XP002605250, DOI: 10.1002/prac.19080770142 Extrait de l'Internet: URL:http://onlinelibrary.wiley.com/doi/10. 1002/prac.19080770142/pdf [extrait le 2010-10-06]
- Guilhem Arrachart Dr. et al.: "Nanostructuring of Hybrid Silicas through a Self-Recognition Process", Chemistry, A European Journal, vol. 15, no. 20 11 mai 2009 (2009-05-11), pages 5002-5005, XP002605251, DOI: 10.1002/chem.200802748 Extrait de l'Internet: URL:http://onlinelibrary.wiley.com/doi/10. 1002/chem.200802748/pdf [extrait le 2010-10-08]
- Joong Tark Han, Dae Ho Lee, Chang Yeol Ryu, Kilwon Cho: "Fabrication of Superhydrophobic Surface from a Supramolecular Organosilane with Quadruple Hydrogen Bonding", , vol. 126 2004, pages 4796-4797, XP002605252, DOI: 10.1021/ja0499400 Extrait de l'Internet: URL:http://pubs.acs.org/doi/pdf/10.1021/ja 0499400 [extrait le 2010-10-05]
- Zhibin Guan, Jason T. Roland, Jane Z. Bai, Sharon X. Ma, Theresa M. McIntire, and Maianh Nguyen: "Modular Domain Structure: A Biomimetic Strategy for Advanced Polymeric Materials", , vol. 126, no. 7 29 janvier 2004 (2004-01-29), pages 2058-2065, XP002605253, DOI: 10.1021/ja039127p Extrait de l'Internet: URL:http://pubs.acs.org/doi/pdf/10.1021/ja 039127p [extrait le 2010-10-07]
- Shan Zou, Zhihong Zhang, Renate Förch, Wolfgang Knoll, Holger Schönherr and G. Julius Vancso: "Tunable Complex Stability in Surface Molecular Recognition Mediated by Self-Complementary Quadruple Hydrogen Bonds", Langmuir, vol. 19 19 septembre 2003 (2003-09-19), pages 8618-8621, XP002605254, DOI: 10.1021/la030127c Extrait de l'Internet: URL:http://pubs.acs.org/doi/abs/10.1021/la 030127c [extrait le 2010-10-11]
- Xiao-Zhong Wang, Xiao-Qiang Li, Xue-Bin Shao, Xin Zhao, Peng Deng, Xi-Kui Jiang, Zhan-Ting Li and Ying-Qi Chen: "Selective Rearrangements of Quadruply Hydrogen-Bonded Dimer Driven by Donor-Acceptor Interaction", , vol. 9, no. 12 6 juin 2003 (2003-06-06), pages 2904-2913, XP002605255, DOI: 10.1002/chem.200204513 Extrait de l'Internet: URL:http://onlinelibrary.wiley.com/doi/10. 1002/chem.200204513/pdf [extrait le 2010-10-15]
- DE GREEF ET AL: "The influence of ethylene glycol chains on the thermodynamics of hydrogen bonded supramolecular assemblies in apolar solvents", CHEMICAL COMMUNICATIONS, vol. 36, 16 July 2008 (2008-07-16), pages 4306-4308,
- WONG ET AL: "Generation-independent dimerization behavior of quadruple hydrogen-bond-containing oligoether dendrons", ORGANIC LETTERS, vol. 8, no. 9, 4 June 2006 (2006-06-04), pages 1811-1814,
- LAFITTE ET AL: "Ureidopyrimidinones incorporating a functionalizabe p-Aminophenyl electron donating group at C-6", JOURNAL OF ORGANIC CHEMISTRY, vol. 70, 3 September 2005 (2005-09-03), pages 2701-2707,
- LI ET AL: "Self-assembly of a new seris of quadruply hydrogen bonded heterotrimers driven by the donor-acceptor interaction", TETRAHEDRON, vol. 61, 10 August 2005 (2005-08-10), pages 9600-9610,

## Description

La présente invention concerne un procédé de traitement cosmétique des matières kératiniques, notamment des cheveux, à l'aide d'une composition comprenant des composés susceptibles de former des liaisons hydrogène, ainsi que les compositions cosmétiques ainsi préparées, et un kit comprenant cette composition.

En cosmétique, on cherche toujours à améliorer les propriétés des matières kératiniques et à lutter contre les dommages, tels que les agressions extérieures comme la pollution et le rayonnement ultraviolet ou les agressions chimiques comme celles provoquées par les traitements oxydants, réducteurs ou alcalins, de coloration ou de permanente. Parmi les dégâts subis par le cheveu, on peut notamment citer l'augmentation du caractère hydrophile, la perte ou le décollement d'une partie des écailles, les difficultés de démêlage.
Pour améliorer les propriétés des cheveux, il est connu d'utiliser des compositions contenant des actifs cosmétiques pour apporter aux matières kératiniques telles que le cheveu tous les bienfaits liés à ces actifs cosmétiques. Toutefois, la rémanence et donc l'efficacité de ces actifs ne sont pas suffisantes, car ils peuvent être aisément éliminés au shampoing, ou encore ils ne forment pas un dépôt homogène à la surface du cheveu.

La présente invention a pour but de proposer une composition cosmétique susceptible d'être utilisée pour le traitement cosmétique des cheveux, et de leur apporter des propriétés cosmétiques de manière durable.
On a en effet constaté que des composés comportant des entités capables de former des interactions physiques entre elles pouvaient apporter des qualités cosmétiques intéressantes au cheveu. Ces composés sont notamment caractérisés par la présence d'au moins une entité capable de donner au moins 3 liaisons hydrogène, en particulier 4 liaisons hydrogène, ainsi que par leur faible masse.

La présente invention a donc pour objet une composition cosmétique telle que définie à la revendication 1.
Un autre objet de l'invention est un procédé de traitement cosmétique des matières kératiniques tel que défini à la revendication 10.
Un autre objet de l'invention est un kit comprenant ladite composition tel que défini à la revendication 13.
Des composés particuliers, de formule (Ia) tels que définis à la revendication 14, constituent encore un autre objet de l'invention.

On a constaté que le fait de déposer sur la peau ou le cheveu ou de faire pénétrer dans le cheveu un composé selon l'invention permettait d'y apporter un effet bénéfique. Sans être tenu par la présente explication, on peut penser que les entités uréidopyrimidone sont capables de générer in situ dans ou sur le cheveu, un réseau réticulé, par associations physiques entre molécules. Déposé sur le cheveu ou la peau, ledit composé se trouve engagé et emprisonné dans un dépôt réticulé, ce qui accroît sa rémanence, en particulier au sébum, à l'eau et au shampooing.
Par ailleurs, comme la réticulation est une réticulation physique, la rémanence de l'effet est possible tout en permettant le démaquillage du composé. L'élimination du dépôt peut consister notamment en un rinçage par une composition nettoyante appliquée à température ambiante ou à une température supérieure à 25°C, ou par l'utilisation d'un démaquillant, ou en utilisant tout rupteur connu de liaison hydrogène.

De nombreux composés incorporant des motifs ureïdopyrimidones (UPY) ont été décrits dans la littérature et étudiés pour leur propriété d'auto-assemblage par des laboratoires de recherche fondamentale. Toutefois aucun de ces documents ne décrit l'application de ces composés sur les matières kératiniques, ni même leur possible utilisation pour renforcer lesdites matières.
Dans le domaine cosmétique, on peut citer WO02098377 qui décrit d'une manière générale des composés à motifs UPY pour des applications cosmétiques sur la peau et les cheveux. On peut aussi citer WO2003032929 qui décrit la préparation de polymères supramoléculaires et leur utilisation dans des applications capillaires. On peut également citer WO2004016598 qui décrit la préparation de polymères supramoléculaires et leur utilisation dans des applications variées incluant les applications cosmétiques. Ou encore WO2005042641 qui décrit la préparation de polymères supramoléculaires, notamment de type polyuréthannes, et leur utilisation dans des applications variées incluant les applications cosmétiques.
Dans l'ensemble de ces documents, les composés décrits sont des polymères, donc des composés de poids moléculaire élevé, qui ne vont donc pas pénétrer dans le cheveu pour lui apporter des propriétés bénéfiques, notamment en terme de protection du cheveu.

La présente invention peut également permettre d'hydrater et de renforcer voire réparer les matières kératiniques, et de leur apporter de la douceur de façon durable de façon à ce que l'effet reste perceptible après au moins un shampooing.
Par renforcement des matières kératiniques, on entend notamment une amélioration des propriétés mécaniques qui peut se traduire par :
- une augmentation de leur rigidité, ce qui leur procure davantage de résistance et de corps; ou bien
- une diminution de leur déformation en particulier en conditions humides, ce qui permet au cheveu de retrouver facilement sa forme initiale une fois séché et se traduit par une amélioration de la dynamique du cheveu; ou encore
- une meilleure résistance à des forces mécaniques de traction qui leur sont appliquées, par exemple lors d'un peignage, et qui peuvent entraîner la casse du cheveu;
- une diminution de sa porosité ou de son gonflement dans l'eau. En effet il est connu qu'un cheveu dégradé par des traitements oxydants, réducteurs ou alcalins est plus poreux qu'un cheveu non dégradé, ce qui se traduit par une diffusion plus rapide et plus à coeur de l'eau et a pour effet une augmentation du diamètre du cheveu en milieu humide (The Science of Hair Care, p.416, 2nd édition, ed C. Bouillon, J Wilkinson, 2005).

Par ailleurs, il est possible de générer un réseau in situ, dans le cheveu, ce qui va permettre d'apporter des propriétés de protection ou de réparation au cheveu, suite à un traitement de coloration, de décoloration, de permanente, de lissage ou de défrisage par exemple. Créer ce réseau réticulé dans le cheveu permet également d'éviter un dégorgement prématuré d'une teinture.
Les composés selon l'invention peuvent donc à la fois gainer le cheveu, et donc apporter notamment des propriétés de renforcement, et également pénétrer dans le cheveu et apporter in situ des propriétés de protection ou de réparation, notamment.
En outre, il a été constaté que l'utilisation de composés selon l'invention sur des cheveux défrisés par un traitement alcalin permettait de transformer le cheveu de façon rémanente aux shampooings, en d'autres termes d'augmenter les performances de défrisage, sans augmenter la porosité desdits cheveux; cet effet restant encore perceptible après 5 shampooings.
Il a également été constaté que l'application de certains composés selon l'invention sur des cheveux abimés, donc hydrophiles, avait pour effet de réparer la surface dudit cheveu en la rendant hydrophobe durablement, et d'en faciliter le démêlage en milieu humide.

Par ailleurs, on a constaté que l'association de certains composés selon l'invention, appliquée sous forme de 3 applications successives à faible concentration (0,1% MA) sur des cheveux abimés, préalablement réduits avec un traitement à l'acide thioglycolique 0,2 M, avait pour effet de rétablir le module de rigidité du cheveu.

Les composés selon l'invention peuvent donc être appliqués avant, pendant ou après un traitement cosmétique de coloration, de décoloration, de permanente, de défrisage, ou de lissage des cheveux, ou bien en traitement d'entretien des cheveux dégradés par les agressions extérieures telles que les UV, la pollution, les brossages répétés, l'eau chlorée.

Le composé susceptible d'être employé dans le cadre de l'invention répond donc à la formule (I), ses sels, ses sels d'addition, ses isomères, ses solvates notamment hydrates, les formes tautomères étant inclues : 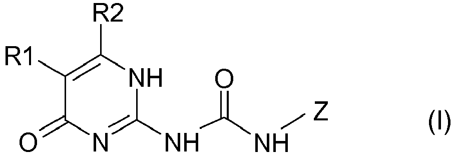 dans laquelle :
- R1 et R2, indépendamment l'un de l'autre, représentent H, -OH, -NRR' (avec R, R', identiques ou différents, étant H ou un radical alkyle, linéaire ou ramifié en C1-C12, de préférence en C1-C4 et mieux un radical méthyle ou éthyle); ou un groupe carboné, notamment alkyle, linéaire, ramifié et/ou cyclique, en C1-C18, de préférence C1-C12, saturé ou non, éventuellement aromatique, pouvant contenir un ou plusieurs hétéroatomes O, N et/ou S, notamment OH, COOH et/ou NRR'.

Les radicaux R1 et R2 peuvent notamment être choisis parmi :
- H;
- NH₂,
- un groupe alkyle en C1-C18, éventuellement substitué par une ou plusieurs fonctions NH₂, COOH et/ou OH;
- un groupe cycloalkyle en C4-C12, éventuellement substitué par une ou plusieurs fonctions NH₂, COOH et/ou OH;
- un groupe aryle en C4-C12, éventuellement substitué par une ou plusieurs fonctions NH₂, COOH et/ou OH; et/ou éventuellement substitué par un ou plusieurs groupements PEG de formule -(CH₂CH₂O)m- avec m=2 à 15;
- un groupe alcoxy en C1-C4;
- un groupe aryle (C1-C4)alcoxy; éventuellement substitué par une ou plusieurs fonctions NH₂, COOH et/ou OH.

De préférence R1 représente H.
De préférence, R2 représente H, CH₃, C₇H₁₅, C₁₃H₂₇ ou aryle.
Préférentiellement, R1=H et R2=méthyle.
- Z représente un radical monovalent choisi parmi :
   (i) un atome d'hydrogène,
   (ii) un radical alkyle (saturé) linéaire ou ramifié, en C1 à C32; notamment en C2-C24, voire C3-C18, encore mieux C4-C12;
   (iii) un radical alcène (insaturé) linéaire ou ramifié, en C2 à C32, comprenant une ou deux doubles liaisons C=C; notamment en C2-C24, voire en C3-C18;
   (iv) un radical alkyle en C1-C32, notamment C2-C24, voire C3-C18, encore mieux C4-C12, substitué par un aryle en C6-C10, notamment C6-C8; en particulier phényle;
lesdits radicaux pouvant éventuellement être substitués par 1 à 8, notamment 2 à 6, voire 3 à 5, groupements choisis parmi -OH, -OR, -SH, -SR, -SO₃H, -SO₃R,-SO₂NRR', -COOH, -COOR, -CONRR', -NR-C(O)-NRR', -NRR' et -N⁺RR'R", avec R, R' et R" = H ou alkyle en C1-C6, notamment méthyle; et les substituants de formule (a) à (h) ci-dessous : 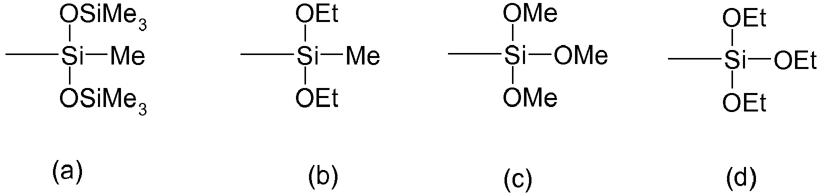 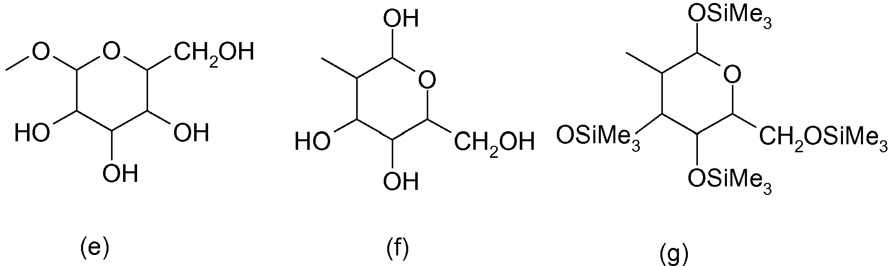 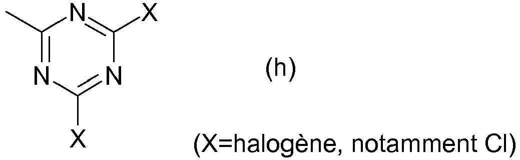 et/ou lesdits radicaux pouvant éventuellement comprendre 1 à 8, notamment 2 à 6, voire 3 à 5, groupements divalents choisis parmi, seuls ou en mélange, -S-, S(O), SO₂, -NH- (ou =NH), -O-, -C(O)-, -C(=NH)-, -N⁺(CH₃)₂-An⁻ (An⁻ : anion); ou bien-N= (trivalent).

Par souci de clarté, il est spécifié que le radical Z ne peut pas comprendre de groupement :

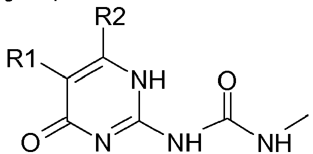

De préférence, le radical Z comprend 0 à 12 hétéroatomes, notamment 1 à 10, voire 2 à 6 hétéroatomes.
De préférence, le radical Z, lorsqu'il est substitué, l'est par 1 à 8, notamment 2 à 6, voire 3 à 5, groupements choisis parmi -OH, -SH, -SO₃H, -COOH, -NRR' et-N⁺RR'R", avec R, R' et R" = H ou alkyle en C1-C6, notamment méthyle; et les substituants de formule (a) à (h).
De préférence, le radical Z, lorsqu'il est interrompu, l'est par 1 à 8, notamment 2 à 6, voire 3 à 5, groupements divalents choisis parmi, seuls ou en mélange, -S-,-NH- (ou =NH), -O-, -C(O)-, -C(=NH)-, -N⁺(CH₃)₂-.

Parmi les composés de formule (I) particulièrement préférés, on peut citer les composés suivants :

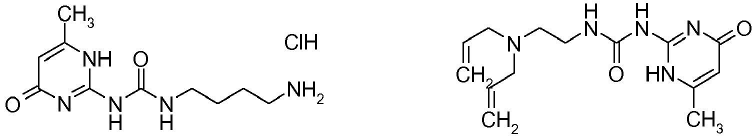

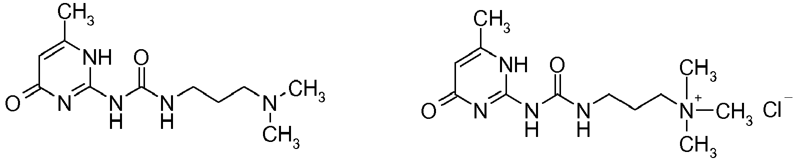

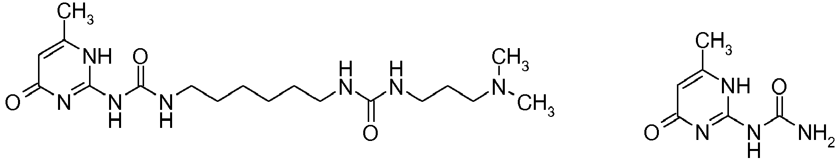

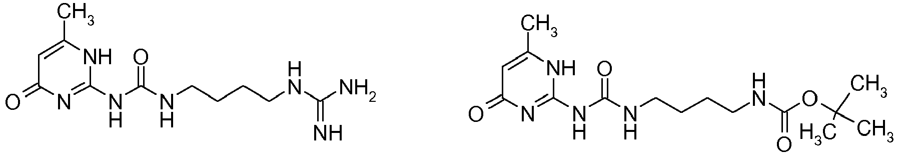

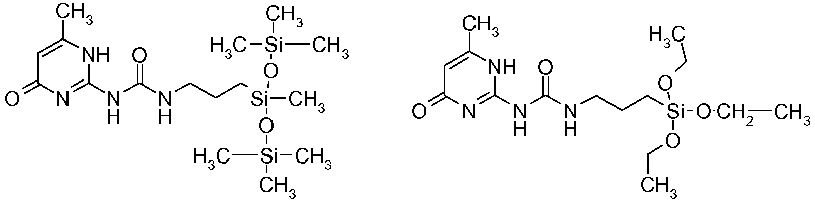

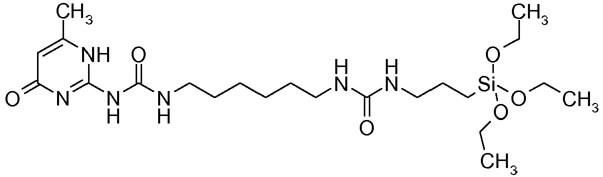

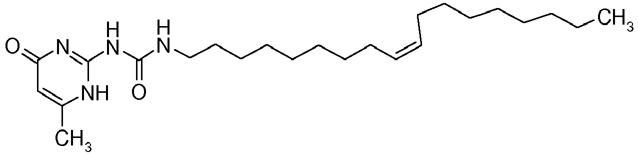

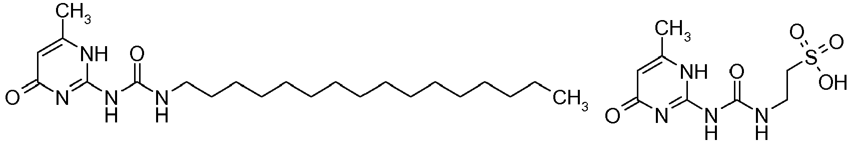

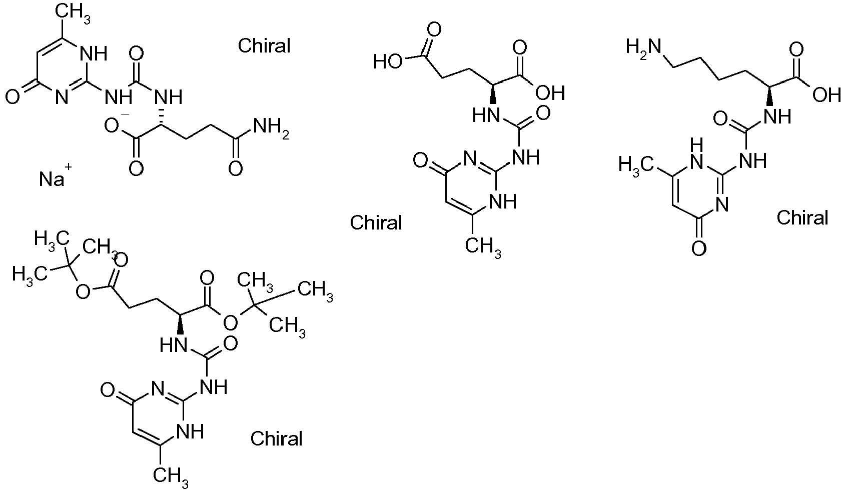

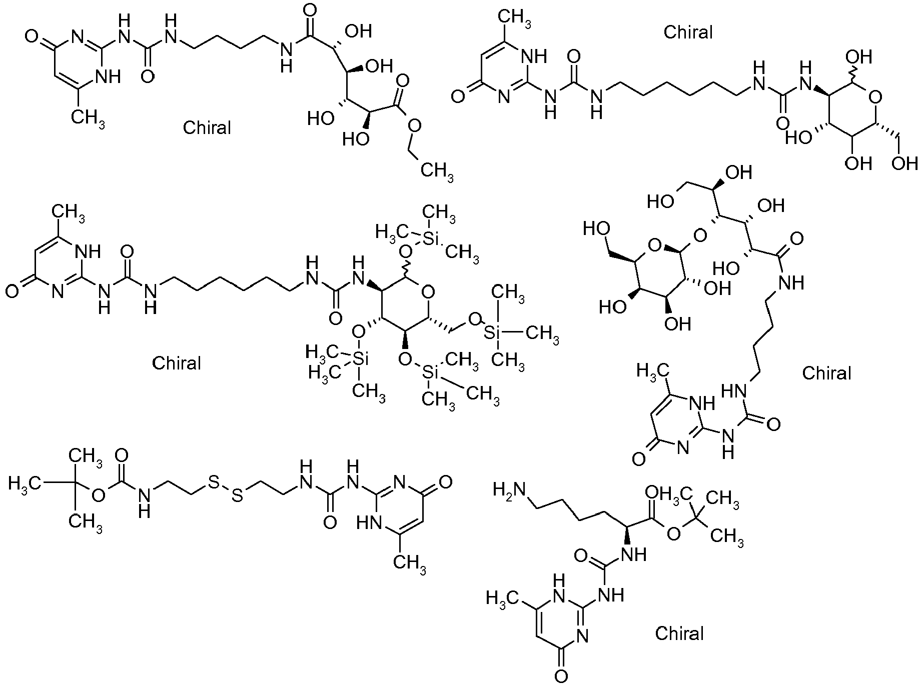

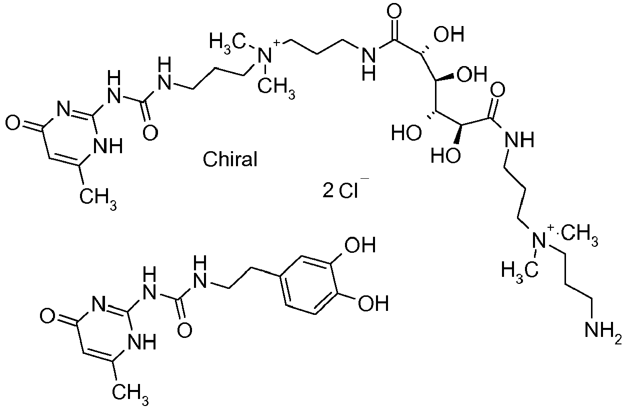

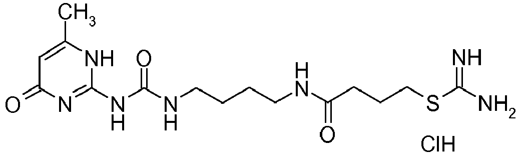

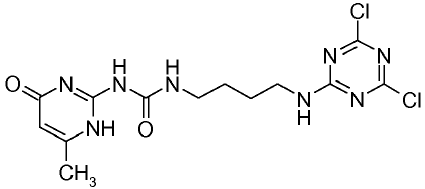

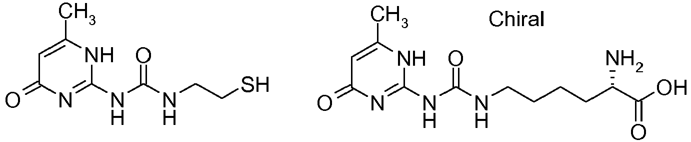

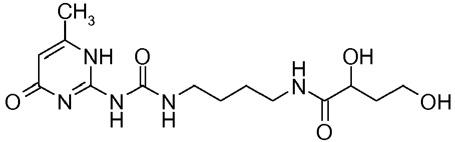

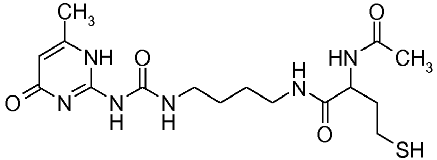

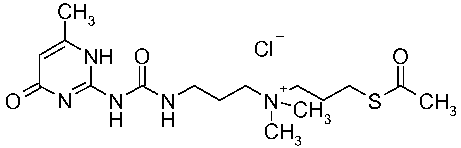

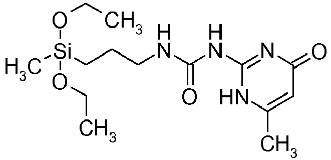

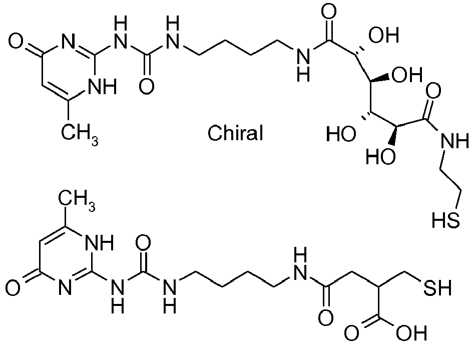

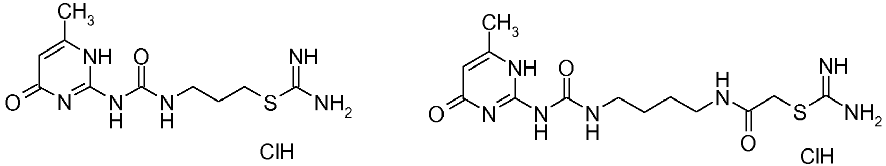

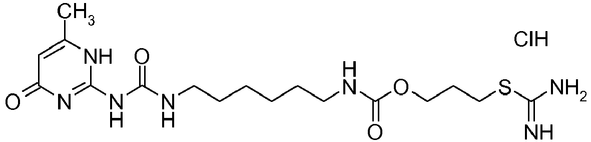

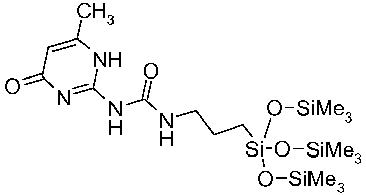

De préférence, le poids moléculaire en masse (Mw) du composé de formule (I) selon l'invention est inférieur ou égal à 1200 g/mol. Ce faible poids moléculaire favorise en particulier la pénétration des composés dans le cheveu.

La présente invention concerne également certains composés nouveaux, de formule (Ia), ainsi que leurs formes tautomères et leurs sels 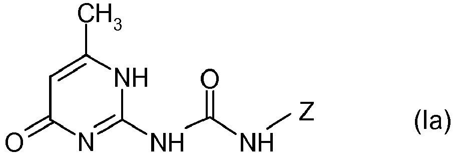 dans laquelle Z représente un radical monovalent choisi parmi :
(i) un radical hydrocarboné non cyclique saturé ou non, linéaire ou ramifié, en C1 à C32,
   - éventuellement substituée par 1 à 20 radicaux hydroxyles -OH, et/ou
   - éventuellement interrompu par 1 à 10 groupements identiques ou différents choisis parmi, seuls ou combinés entre eux, -S-, S(O), SO₂, -NH- (ou =NH), -N= (trivalent), -O-, -C(O)-,-CH(OH)-, -C(=NH)-, -N+(CH₃)₂-, radical aryle (cyclique aromatique), en C6-C10, notamment C6-C8. De préférence le groupement interrompant est choisi parmi les groupements -NH-, -C(O)-, -CH(OH)-, -N+(CH3)₂- ou est inexistant ; de préférence le radical hydrocarboné non cyclique est linéaire en C3 à C10;
   - terminé par un groupement choisi parmi -NR₁R₂, -N+R₁R₂R₃ An-, -NHC(O)OR₄,-CH(NH₂)CO₂R₄, -NHC(O)CH(0H)(CH₂)₂OH, dans lesquels
   R₁, R₃, représentent indépendamment un radical alkyle linéaire ou ramifié en C1-C6, un radical hydroxyalkyle ou un radical aminoalkyle,
   R₂ représente un radical alkyle linéaire ou ramifié en C1-C6, ou un radical aminoalkyle,
   R₄ représente un radical alkyle en C1-C6, linéaire ou ramifié, ou un radical hydroxyéthyle;
   An- désigne un anion ou un mélange d'anions cosmétiquement acceptable.
(ii) un radical de formule (A1) : -(CH₂)ₚ-(X)ₙ-(CH₂)_{q}-NR₆-C(=NH)-NR₇R₈
   dans laquelle X désigne un groupement choisi parmi -S-, S(O), SO₂, -NH- (ou =NH), -N= (trivalent), -O-, -C(O)-, -C(=NH)-, -C(O)O-,-OC(O)-,-NC(O)-, -NC(O)N-, NSO2N-, -NR6-C(=NH)-NR7-, -N+(CH3)2- An-, un radical aryle (cyclique aromatique), en C6-C10, notamment C6-C8. De préférence le groupement interrompant est égal à -NH-C(O)-NH-;
   An- désigne un anion ou un mélange d'anions cosmétiquement acceptables.
   n, p et q sont des nombres entiers et
   n = 0 à 8 ;
   p = 1 à 10 ;
   q=1 à 10,
   p+q ≠ 5.
   De préférence n = 0 ou 1, p + q compris entre 4 et 10, p+ q ≠ 5 ;
   Plus préférentiellement n = 0 et p + q = 4.
   R₆, R₇, R₈ désignent indépendamment un atome d'hydrogène H, un radical alkyle linéaire ou ramifié en C1-C6, notamment méthyle ou éthyle, ou un radical hydroxyalkyle notamment hydroxyéthyle.
   De préférence R₆, R₇, R₈ désignent indépendamment un atome d'hydrogène H, un radical méthyle ou éthyle ;
   Plus préférentiellement R₆, R₇, R₈ sont identiques et représentent un atome d'hydrogène H.
(iii) un radical de formule (A2) : -(CH₂)ᵣ-(T)ₛ-(CH₂)ₜ-(CHR₉)ᵤ-(CH₂)ᵥ- Si R₁₀R₁₁R₁₂ dans lequel T représente un groupement choisi parmi les groupements, -S-, S(O), SO2, -NH- (ou =NH), -N= (trivalent), -O-, -C(O)-, -C(=NH)-, -N+(CH3)2- An-, un radical aryle (cyclique aromatique), en C6-C10, notamment C6-C8, une double liaison carbone -carbone notamment -CH=CH-, une triple liaison carbone-carbone, et leurs combinaisons. De préférence T représente un groupement -NH-C(O)-NH- ou -NH-C(O)-O-.
   Lorsque s, respectivement u, est non nul, les groupements T adjacents, respectivement -CHR₉-adjacents, sont identiques ou différents.
   Rg désigne un radical choisi parmi les radicaux -OH, -SH, -SO₃H, -COOH, -COOR, -NHC(O)R, NHC(O)OR, -NRR' et -N⁺RR'R" An-, dans lesquels R, R' et R" , identiques ou différents représentent un atome d'hydrogène H ou un radical alkyle linéaire ou ramifié en C1-C6, notamment méthyle ;
   R₁₀, R₁₁, R₁₂ sont choisis indépendamment parmi les radicaux OR₁₃, alkyle linéaire ou ramifié en C1-C6 ;
   R₁₃ désigne un radical alkyle linéaire ou ramifié en C1-C6, ou un radical -(CH₂-CH₂-O)_{w}R₁₄ ou un radical -SiR₁₅R₁₆R₁₇.
   R₁₄ désigne un atome d'hydrogène H, ou un radical méthyle.
   R₁₅ , R₁₆, et R₁₇ désignent indépendamment un radical alkyle linéaire ou ramifié en C1-C4.
   De préférence R₁₃ représente un radical méthyle ou éthyle ou un radical SiMe₃. An- désigne un anion ou un mélange d'anions cosmétiquement acceptables.
   r, s, t, u, v, w sont des nombres entiers et
   r = 1 à 10 ;
   s, t, u, v = 0 à 10 ;
   w = 1 à 5 ;
   étant entendu que lorsque s=u=0 et R₁₀=R₁₁=R₁₂= OR₁₃, alors r+t+v ≠ 3.
   De préférence, s = 0 ou 1, u = 0, r+t+v est compris entre 1 et 10, plus préférentiellement entre 3 et 9.
(iv) un radical de formule (A3) : -(CH₂)ₓ-(U)_{y}-(CH₂)_{z}-CH₃
   dans laquelle U désigne un groupement choisi parmi les radicaux, -S-, S(O), SO₂, -NH- (ou =NH), -N= (trivalent), -O-, -C(O)-, -C(=NH)-, -N+(CH₃)₂- An-, un radical aryle (cyclique aromatique), en C6-C10, notamment C6-C8 , une double liaison carbone -carbone notamment -CH=CH-, une triple liaison carbone -carbone , ainsi que leurs combinaisons;
   An- désigne un anion ou un mélange d'anions cosmétiquement acceptables.
   x, y et z sont des nombres entiers, compris entre 0 et 10 inclus;
   étant entendu que lorsque y=0, x+z est supérieur ou égal à 10 et inférieur ou égal à 30 et différent de 18, notamment x+z est égal à 15 ;
   et que lorsque y ≠ 0, x est supérieur ou égal à 2 et inférieur ou égal à 25 et z est supérieur ou égal à 3 et inférieur ou égal à 25, notamment x+z est égal à 15 avec U égal à C=C et y =1 ;
(v) un radical de formule (A4) : -Alk1-(V)ₐ₁-(Alk2)ₐ₂-CH₂-W
   dans laquelle Alk1 et Alk2 désignent indépendamment un radical alkyle saturé ou non, linéaire ou ramifié, en C1 à C32. De préférence le radical alkyle est saturé, en C1 à C10.
   V désigne un groupement choisi parmi les radicaux, -S-, S(O), SO₂, -NH- (ou =NH), -N= (trivalent), -O-, -C(O)-, -C(=NH)-, -N+(CH₃)₂- An-, radical aryle (cyclique aromatique), en C6-C10, notamment C6-C8 , double liaison carbone -carbone notamment -CH=CH- , triple liaison carbone -carbone, ou leurs combinaisons. De préférence, V représente un groupement choisi parmi -NH-C(O)-, NH-C(O)-NH-, -NH-CO-O-, O-C(O)-, -N+(CH₃)₂- An-.
   An- désigne un anion ou un mélange d'anions cosmétiquement acceptables.
   W désigne un radical choisi parmi les radicaux -SR₁₅, -S(O)R₁₅, -SO₂R₁₅, -SO₃H,-OSO₃H. De préférence, W représente un radical -SR₁₅ ou -SO₃H.
   R₁₅ désigne un (hétéro)cycle aromatique en C5-C10 notamment C5-C6 et dont 3 atomes de carbone peuvent être remplacés par des atomes d'azote; un radical carbonyle -C(O)R₁₆ ; un radical -C(=NR₁₇)-NR₁₈R₁₉
   R₁₆ désigne un radical alkyle linéaire ou ramifié en C1 à C6
   R₁₇, R₁₈, R₁₉ désignent indépendamment un atome d'hydrogène H, un radical alkyle linéaire ou ramifié en C1-C6,
   R₁₇ et R₁₈ peuvent être reliés entre eux pour former avec les atomes d'azote auxquels ils sont rattachés un cycle ou un bicycle de 5 à 8 atomes, saturé ou non ;
   a1 et a2 sont des nombres entiers
   a1 = 0 à 8, de préférence 0 à 2.
   a2 = 0 ou 1.
(vi) un radical de formule (A5) : -Y-(K)j-G
   dans laquelle Y désigne un radical alkyle saturé ou non, linéaire ou ramifié, en C1 à C32, éventuellement interrompu par 1 à 8 groupements identiques ou non choisis parmi -S-, S(O), SO₂, -NH- (ou =NH), -N= (trivalent), -O-, -C(O)-, -C(=NH)-,-N+(CH₃)₂- An-, un radical aryle (cyclique aromatique), en C6-C10, notamment C6-C8, ou leur combinaison.
   De préférence, Y désigne un radical alkyle saturé, en C2 à C8, plus préférentiellement C2, C4, C6 ;
   K désigne un groupement choisi parmi -CO-, -NHC(O)- -CH(NRR')-, -CHR-,-C(=CHR)-. De préférence, K désigne un groupement -CO- ou -NHC(O)-, plus préférentiellement -NHC(O)- ;
   G désigne un groupement choisi parmi G1, G2 ou G3, avec:
   - G1 désigne un monosaccharide ou un polysaccharide jusqu'à 20 unités sucre, sous forme pyranose et/ou furanose et de séries L et/ou D, ledit mono- ou polysaccharide présentant éventuellement une ou plusieurs fonctions aminés, les fonctions hydroxyles et amines étant éventuellement protégées ; et leurs isomères optiques et/ou géométriques. De préférence, G1 représente un monosaccharide ou un polysaccharide contenant jusqu'à 6 unités sucre, sous forme pyranose et/ou furanose et de série L et/ou D. Avantageusement, les monosaccharides préférés sont choisis parmi la D-glucosamine, la D-galactosamine, le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, avec une préférence pour la D-glucosamine. Ces monosaccharides peuvent être protégés ou non sur les fonctions hydroxyles non engagées dans la liaison avec le radical H. Avantageusement encore, les polysaccharides préférés contenant jusqu'à 6 unités sucre sont choisis parmi le D-maltose, le D-lactose, le D-cellobiose, le D-maltotriose, un disaccharide associant un acide uronique choisi parmi l'acide D-iduronique, ou l'acide D-glucuronique avec une hexosamine choisi parmi la D-galactosamine, la D-glucosamine, la N-acétyl-D-galactosamine, la N-acétyl-D-glucosamine.
   - G2 désigne un radical acide aminé alpha avec une chaine latérale, protégée ou non, choisie parmi les chaines latérales des 22 acides aminés naturels, C ou N branché, racémique ou non, de stéréochimie L ou D, protégé ou non sur le résidu C ou N et sur la chaine latérale pour les acides aminés alpha concernés. De préférence, la chaine latérale est celle d'une cystéine, d'une lysine, d'un acide aspartique, d'un acide glutamique ou d'une arginine, plus préférentiellement une lysine.
   - G3 désigne un radical phényle substitué sur l'une quelconque des positions par un radical hydroxyle et éventuellement substitué par un groupement R₂₀, De préférence, G3 désigne un orthodiphénol.
   R₂₀ désigne un radical alkyle saturé ou non, linéaire ou ramifié, en C1 à C6, hydroxyalkyle en C1 à C6, un radical aryle (cyclique aromatique) en C5-C10, un radical -OH, -SH, -SO₃H, -COOH, -COOR, -NHC(O)R, NHC(O)OR, -NRR' et-N⁺RR'R" An-,
   R, R' et R" désignent indépendamment un atome d'hydrogène H ou un radical alkyle en C1-C6, notamment méthyle ;
   An- désigne un anion ou un mélange d'anions cosmétiquement acceptables.
   j est un nombre entier égal à 0 ou 1.
   De préférence, j = 1 quand G désigne un radical G1 et j = 0 quand G désigne un radical G2 ou G3.
   Lorsque G désigne un radical G1, et que G1 est relié à H par une liaison anomérique, celle-ci peut être α ou β.
   Les fonctions hydroxyles ou amines protégées de mono- ou de polysaccharides sont par exemple celles obtenues par les réactions de protection décrites dans Protective Groups in organic synthesis, T.W. Greene, P.G.M. Wuts, second edition, Wiley Interscience, 1991.
(vii) un radical de formule (A6) : -(CH₂)_{g}-(J)ₕ-(CH₂)ᵢ-(CHR₂₃)ₖ-CH₂R₂₄
   dans laquelle J désigne un groupement choisi parmi les groupements -S-, S(O), SO2, -NH- (ou =NH), -N= (trivalent), -O-, -C(O)-, -C(=NH)-, -N+(CH3)2- An-,un radical aryle (cyclique aromatique), en C6-C10, notamment C6-C8 , une double liaison carbone -carbone notamment -CH=CH-, une triple liaison carbone-carbone, ou leurs combinaisons.
   De préférence, J désigne un groupement -NH-C(O)- ;
   R₂₃ désigne un radical -OR₂₅, -SH, -SO₃H, -COOH, -COOR, -NHC(O)R, NHC(O)OR, -NRR' et -N⁺RR'R" An-, avec R, R' et R" = H ou alkyle en C1-C6, notamment méthyle. De préférence, R₂₃ désigne un radical -OR₂₅ ;
   R₂₄ désigne un atome d'hydrogène H, ou un radical hydroxyle -OH;
   R₂₅ désigne un atome d'hydrogène H, ou un monosaccharide ou un polysaccharide jusqu'à 20 unités sucre, sous forme pyranose et/ou furanose et de séries L et/ou D, ledit mono- ou polysaccharide présentant éventuellement une ou plusieurs fonctions aminés, les fonctions hydroxyles et amines étant éventuellement protégées ; et leurs isomères optiques et/ou géométriques. De préférence, R₂₅ représente un atome d'hydrogène ou un monosaccharide ou un polysaccharide contenant jusqu'à 6 unités sucre, sous forme pyranose et/ou furanose et de série L et/ou D. Avantageusement, les monosaccharides préférés sont choisis parmi la D-glucosamine, la D-galactosamine, le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, avec une préférence pour le glucose. Ces monosaccharides peuvent être protégés ou non sur les fonctions hydroxyles non engagées dans la liaison avec le radical -CH₂-R₂₄. Avantageusement encore, les polysaccharides préférés contenant jusqu'à 6 unités sucre sont choisis parmi le D-maltose, le D-lactose, le D-cellobiose, le D-maltotriose, un disaccharide associant un acide uronique choisi parmi l'acide D-iduronique, ou l'acide D-glucuronique avec une hexosamine choisi parmi la D-galactosamine, la D-glucosamine, la N-acétyl-D-galactosamine, la N-acétyl-D-glucosamine.
   An- désigne un anion ou un mélange d'anions cosmétiquement acceptables.
   g, h, i, k désignent un nombre entier
   g, h k = 1 à 10 ;
   i = 0 à 10.
   De préférence, g, h, k = 1 à 6 ; i = 0 à 3,
   Plus préférentiellement g = 4 à 6, h = 1, k = 4 à 6, i = 0.
   Dans le cas ou R₂₅ est relié à R₂₃ par une liaison anomérique, celle-ci peut être α ou β.
(viii) un radical de formule (A7) : -(CH₂)_{b}-(A)-(B)-(A)_{c}-(CH₂)_{d}-D
   dans lequel A désigne un groupement choisi parmi -NHC(O)-, -C(O)NH-,-NHC(O)NH-, -C(O)O-, -OC(O)-;
   B désigne une chaine hydrocarbonée, ramifiée ou non, éventuellement substituée par un radical choisi parmi -OH, -COOH, -NHC(O)R₂₁ ;
   R₂₁ désigne un radical alkyle linéaire ou ramifié, en C1 à C6;
   D désigne un radical -SH ou un radical alkyle linéaire ou ramifié, en C1 à C4.
   b, c et d sont des nombres entiers
   b = 2 à 10, de préférence 3 à 6 ;
   c = 0 ou 1 ;
   d = 0 à 10, de préférence 0 à 3 ;
(ix) un radical de formule (A8) : -Alk3-S-S-Alk4
   dans lequel Alk4 désigne un radical hydrocarboné linéaire ou ramifié, en C2 à C20, comprenant éventuellement une ou plusieurs doubles liaisons, et comprenant éventuellement un ou plusieurs hétéroatomes choisis parmi O ou N, et éventuellement substituée par un ou plusieurs radicaux identiques ou différents choisis parmi -OH, -SO₃H, -COOH, -COOR, -NHC(O)R, NHC(O)OR, -NRR' et -N⁺RR'R" An-.
   Alk3 désigne un radical hydrocarboné linéaire ou ramifié, en C2 à C20, comprenant éventuellement une ou plusieurs doubles liaisons, et comprenant éventuellement un ou plusieurs hétéroatomes choisis parmi O ou N, et éventuellement substituée par un ou plusieurs radicaux identiques ou différents choisis parmi -OH, -SO₃H, -COOH, -COOR, -NHC(O)R, -NRR' et -N⁺RR'R" An-.
   R, R' et R" désignent indépendamment un atome d'hydrogène H ou un radical alkyle linéaire ou ramifié en C1-C6, notamment tert-butyle ou méthyle.
   An- désigne un anion ou un mélange d'anions cosmétiquement acceptables.
   De préférence, Alk3 représente une chaine hydrocarbonée linéaire ou ramifiée, en C2 à C10, éventuellement interrompue par un groupement choisi parmi -NH-C(O)-O, -NH-C(O)-, -O-C(O)-.
   De façon encore plus préférée, Alk3 représente une chaine hydrocarbonée linéaire, en C2 à C6.
(x) un radical de formule (A9) : -CHL-COOR₂₂
   dans lequel L désigne la chaine latérale d'un acide alpha aminé, choisie parmi les chaines latérales des 22 acides aminés naturels ; la stéréochimie est L, D ou un mélange racémique. De préférence, la chaine latérale est celle d'une lysine (-(CH₂)₄-NH₂), d'un acide glutamique (-CH₂-CH₂-CO₂H) ou d'une glutamine (-CH₂-CH₂-CO-NH₂), protégée ou non.
   R₂₂ désigne un atome d'hydrogène H, ou un radical alkyle linéaire ou ramifié en C1 à C6, ou un radical benzyle.
   De préférence, R₂₂ désigne un atome d'hydrogène H ou un radical tertbutyle.
(xi) un radical -(CH₂)₂-SH
(xii) un radical -(CH₂)₄-NH2

Etant entendu que le composé (Ia) ne peut représenter les composés suivants : 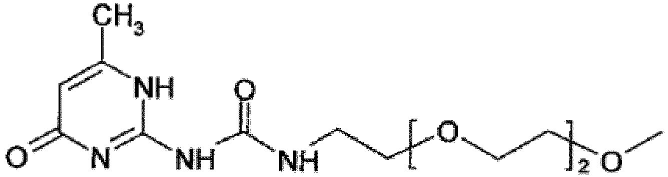 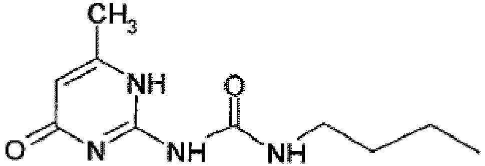 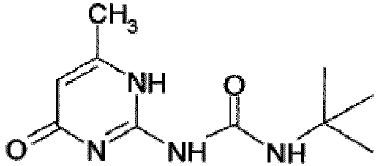 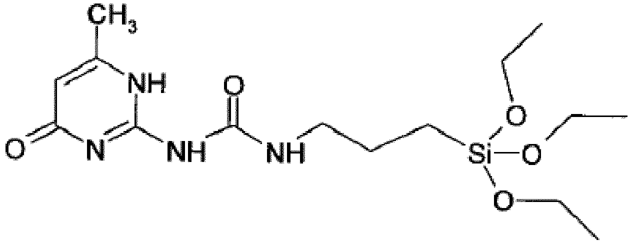 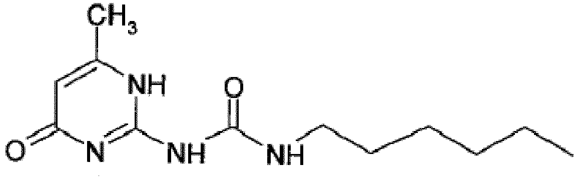 et

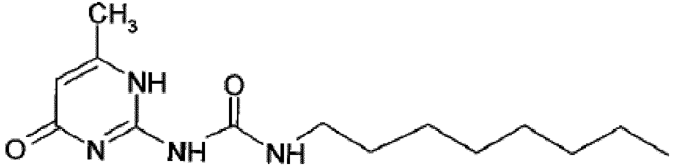

Selon une première variante, les composés de formule (Ia) sont tels que Z désigne :
un radical hydrocarboné non cyclique saturé, linéaire , en C1 à C20, éventuellement substitué par 1 à 5 radicaux hydroxyles -OH, éventuellement interrompu par 1 à 5 groupements identiques ou différents choisis parmi, -NH-CO-NH- , -NHCO- , CO-NH- N+(CH3)₂-, terminé par un groupement choisi parmi -NR₁R₂, -N+R₁R₂R₃ An-, -NHC(O)OR₄, -CH(NH₂)CO₂R₄, -NHC(O)CH(OH)(CH₂)₂OH,
dans lesquels :
   R₁, R₂, R₃, représentent indépendamment un radical méthyle ou éthyle ou un radical aminoalkyle, notamment -(CH₂)₄-NH₂.
   R₄ représente un atome d'hydrogène H, ou un radical méthyle, éthyle ou terbutyle; An- désigne un anion ou un mélange d'anions cosmétiquement acceptable.

Selon une seconde variante, les composés de formule (Ia) sont tels que Z désigne un radical (A1) dans lequel n représente 0 ou 1, X représente -NH-C(O)-NH-.. p+q = 4 à 10 étant entendu que p+ q ≠ 5.
Plus préférentiellement, selon cette variante, n = 0, p + q = 4.

Selon une troisième variante, les composés de formule (Ia) sont tels que Z désigne un radical (A2) dans lequel T représente un groupement -NH-C(O)-NH- ou-NH-C(O)-O-, u=0, s= 0 ou 1, r, t et v sont des nombres entiers avec r +t + v compris entre 1 et 10, plus préférentiellement entre 3 et 9.
R₁₀, R₁₁, R₁₂, sont choisis indépendamment parmi les radicaux OMe, OEt, OSiMe3, Me, Et,
étant entendu que lorsque s=u=0 et R₁₀=R₁₁=R₁₂= OR₁₃, alors r+t+v ≠ 3.

Selon une quatrième variante, les composés de formule (Ia) sont tels que Z désigne un radical (A3) dans lequel U désigne une double liaison carbone -carbone CH=CH, y est égal à 0 ou 1 et x+z =15.

Selon une cinquième variante, les composés de formule (Ia) sont tels que Z désigne un radical (A4) dans lequel Alk1 et Alk2 désignent indépendamment un radical alkyle saturé linéaire en C1 à C10.
V désigne un groupement choisi parmi les radicaux -NH-C(O)-, NH-C(O)-O-,-N+(CH3)2- An-.
An- désigne un anion ou un mélange d'anions cosmétiquement acceptables.
W représente un radical -S-CO-CH3 ou -SO₃H ou -S-C(=NH)NH2.
a1 = 0 ou 1.
a2 = 0 ou 1.

Selon une sixième variante, les composés de formule (Ia) sont tels que Z désigne un radical (A5) dans lequel Y désigne un radical alkyle saturé linéaire en C1 à C8, plus préférentiellement C2, C4, C6 ;
K désigne un groupement -CO- ou -NHC(O)-, plus préférentiellement -NHC(O)- ;
j est égal à 0 ou 1.
G désigne un groupement choisi parmi G1, G3, avec:
- G1 désigne un monosaccharide sous forme pyranose et/ou furanose et de séries L et/ou D, ledit monosaccharide présentant éventuellement une ou plusieurs fonctions amines, les fonctions hydroxyles et amines étant éventuellement protégées ; et leurs isomères optiques et/ou géométriques. Avantageusement, les monosaccharides préférés sont choisis parmi la D-glucosamine, la D-galactosamine, avec une préférence pour la D-glucosamine. Ces monosaccharides peuvent être protégés ou non sur les fonctions hydroxyles non engagées dans la liaison avec le radical H.
- G3 désigne un radical orthodiphénol.
De préférence, j = 1 quand G désigne un radical G1 et j = 0 quand G désigne un radical G3.
Lorsque G désigne un radical G1, et que G1 est relié à H par une liaison anomérique, celle-ci peut être α ou β.

Selon une septième variante, les composés de formule (Ia) sont tels que Z désigne un radical (A6) dans lequel J désigne un groupement -NH-C(O)- ;
R₂₃ désigne un radical -OR₂₅, -SH, -SO₃H, -COOH, -COOR, -NHC(O)R, NHC(O)OR, -NRR' et -N⁺RR'R" An-, avec R, R' et R" = H ou alkyle en C1-C6, notamment méthyle. R₂₃ désigne un radical -OR₂₅;
R₂₄ désigne un atome d'hydrogène H, ou un radical hydroxyle -OH, de préférence un radical hydroxyle -OH.
R₂₅ désigne un atome d'hydrogène H, ou un monosaccharide, sous forme pyranose et/ou furanose et de séries L et/ou D, ledit monosaccharide présentant éventuellement une ou plusieurs fonctions amines, les fonctions hydroxyles et amines étant éventuellement protégées ; et leurs isomères optiques et/ou géométriques. De préférence, R₂₅ représente un atome d'hydrogène ou un monosaccharide sous forme pyranose et/ou furanose et de série L et/ou D. Avantageusement, les monosaccharides préférés sont choisis parmi le D-glucose, le D-galactose, le D-mannose, le D-xylose, le D-lyxose, le L-fucose, le L-arabinose, le L-rhamnose. Ces monosaccharides peuvent être protégés ou non sur les fonctions hydroxyles non engagées dans la liaison avec le radical -CH₂-R₂₄.
Plus préférentiellement R₂₅ désigne un atome d'hydrogène ou le glucose.
g = 4 à 6, h = 1, k = 4 à 6, i = 0.
Dans le cas ou R₂₅ est relié à R₂₃ par une liaison anomérique, celle-ci peut être α ou β.

Selon une huitième variante, les composés de formule (Ia) sont tels que Z désigne un radical (A7) dans lequel A désigne un groupement -NHC(O)-, B désigne une chaine hydrocarbonée, ramifiée ou non, éventuellement substituée par un radical choisi parmi -OH, -COOH, -NHC(O)R₂₁ ;
R₂₁ désigne un radical alkyle linéaire ou ramifié, en C1 à C4, de préférence un radical méthyle;
D désigne un radical -SH ou un radical alkyle linéaire ou ramifié, en C1 à C4, de préférence un radical -SH ou un radical méthyle.
b = 3 à 6 ;
c = 0 ou 1 ; de préférence c = 0;
d = 0 à 3 ;

Selon une neuvième variante, les composés de formule (Ia) sont tels que Z désigne un radical (A8) dans lequel Alk4 désigne un radical hydrocarboné linéaire, en C2 à C10, éventuellement substitué par un ou plusieurs radicaux identiques ou différents choisis parmi -COOR, -NHC(O)R, NHC(O)OR, de préférence NH-C(O)-OR
Alk3 désigne un radical hydrocarboné linéaire, en C2 à C6.
R désigne un atome d'hydrogène H ou un radical alkyle linéaire ou ramifié en C1-C4, notamment tert-butyle ou méthyle.

Selon une dixième variante, les composés de formule (Ia) sont tels que Z désigne un radical (A9) dans lequel L désigne la chaine latérale d'une lysine (-(CH₂)₄-NH₂), d'un acide glutamique (-CH₂-CH₂-CO₂H) ou d'une glutamine (-CH₂-CH₂-CO-NH₂), protégée ou non, l'acide aminé précurseur étant D ou L ou racémique. R₂₂ désigne un atome d'hydrogène H, ou un radical alkyle linéaire ou ramifié en C1 à C6, ou un radical benzyle. De préférence, R₂₂ désigne un atome d'hydrogène H ou un radical tertbutyle.

De préférence, les composés de formule (Ia) sont les suivants :

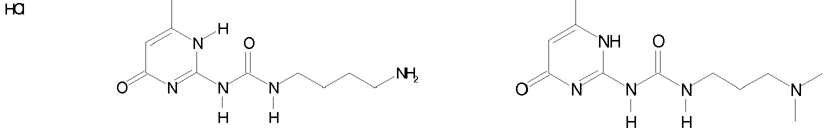

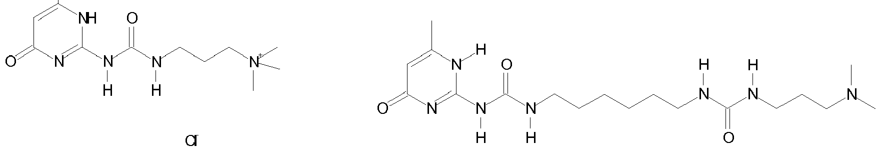

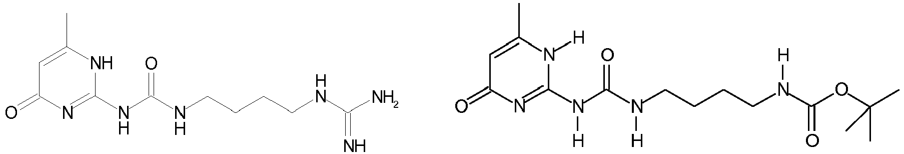

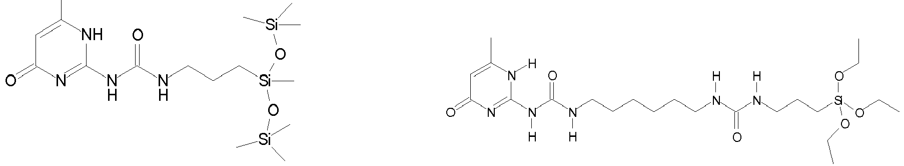

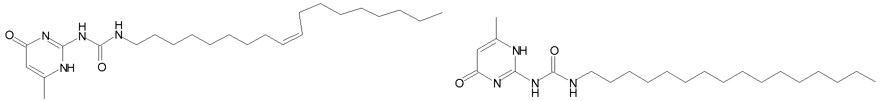

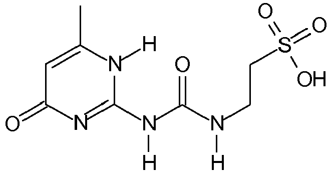

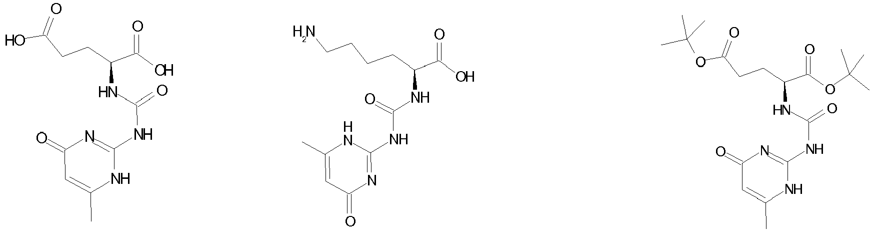

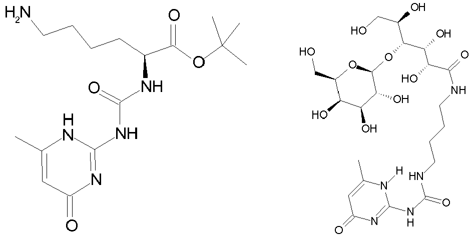

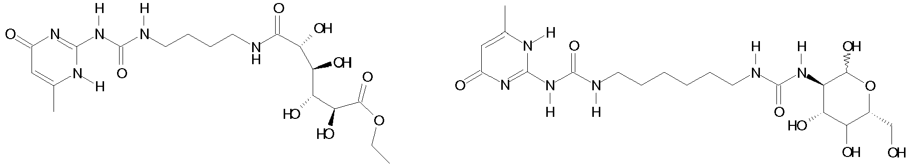

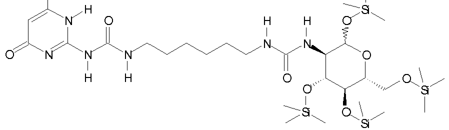

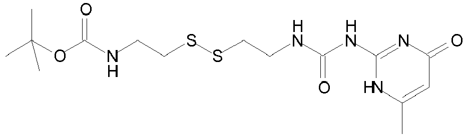

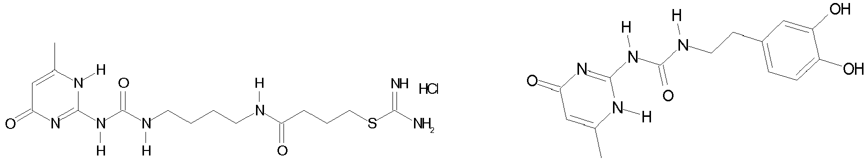

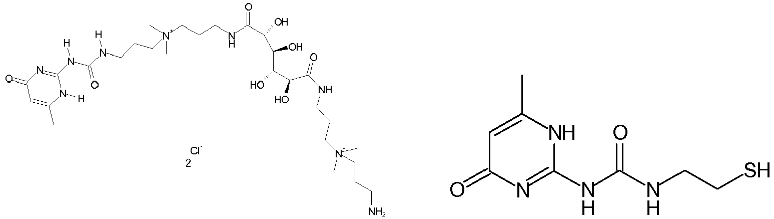

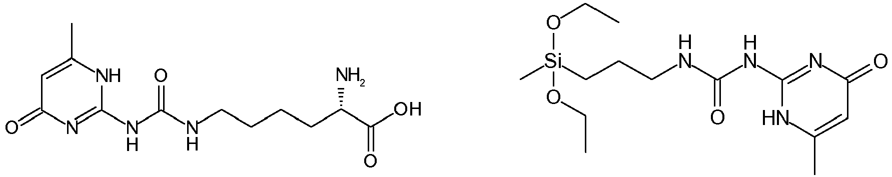

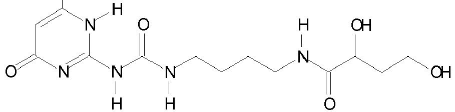

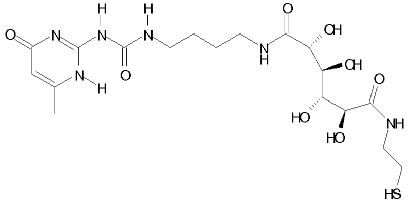

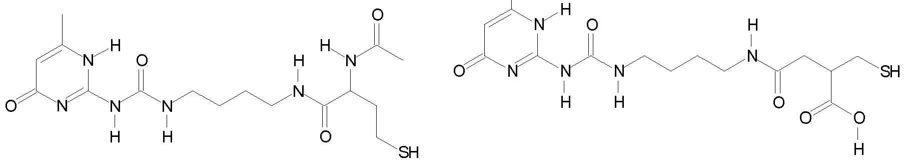

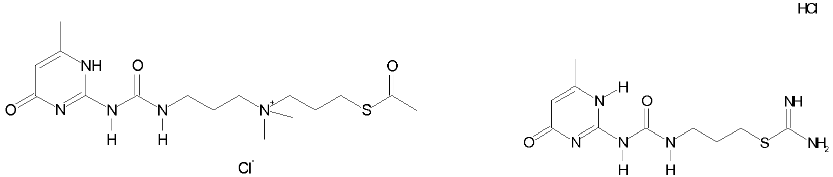

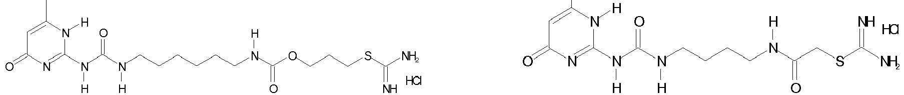

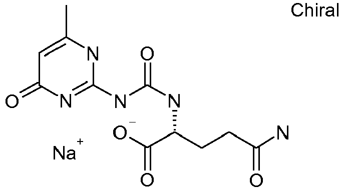

Définition d'un acide aminé N branché : -NH-CHL-COP, avec L représentant la chaine latérale et P égal à -OH ou un groupe protecteur d'acide tel qu'un alcool ou une amine.
Définition d'un acide aminé C branché : -C(0)-CHL-NHP, avec L représentant la chaine latérale et P égal à -H ou un groupe protecteur d'amine tel que le groupement -NHP représente une amide, une urée, un carbamate, une guanidine.

Les composés de formule (I) peuvent être préparés selon toutes les méthodes connues.
Si l'on appelle A, le groupement suivant : 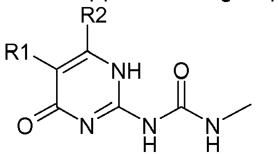 les composés selon l'invention peuvent être schématisés : A-Z.
Ils peuvent être obtenus lors de la réaction :
- entre une fonction réactive liée au groupe A avec une fonction réactive portée par le groupement Z; ou bien
- entre une fonction réactive liée à un précurseur du groupe A avec une fonction réactive portée par le groupement Z pour former simultanément le groupe A et l'entité A-Z;
les deux fonctions réactives étant bien entendu capables de réagir entre elles, et pouvant être liées directement ou par un segment divalent au groupe A, au groupement Z et/ou au précurseur dudit groupe A.

Les fonctions réactives peuvent de préférence être choisies parmi les fonctions :
- isocyanate -N=C=O ;
- isothiocyanate -N=C=S ;
- acide ou ester carboxylique -COORₐ avec Rₐ = H ou un radical alkyle, linéaire ou ramifié en C₁-C₁₂, de préférence en C₁-C₄ et mieux méthyle ou éthyle;
- ester activé COOR_{b} avec OR_{b} choisi parmi phénoxy, 4-nitrophénoxy, 2,4,5-trichlorophénoxy et les radicaux suivants : 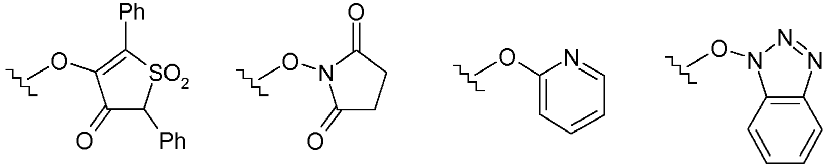
- halogénure d'acyle,
- acyl imidazole ou acyl benzotriazole de formule : 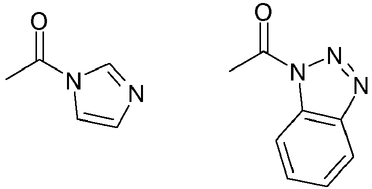
- anhydride d'acide,
- acide carbamique activé -NHCOX avec X= Cl, imidazole, ou OR_{b} avec OR_{b} tel que défini ci-dessus;
- hydroxyle (OH) ou hydroxyle activé par exemple sous forme O-tosylate,
- amine primaire ou secondaire -N(Rₐ)₂, où Rₐ, identique ou différent, est tel que défini ci-dessus;
- une fonction choisie parmi : 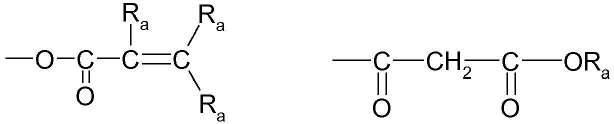 avec Rₐ, identique ou différent, est tel que défini ci-dessus.

De préférence, les fonctions réactives précurseur de la liaison entre Z et A sont choisies parmi les fonctions isocyanate, amine, hydroxyle ou de formule : 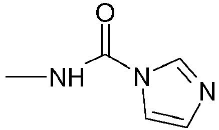 Une méthode d'obtention particulière des composés selon l'invention est décrite dans l'article de Katritzky et al., Comprehensive Organic Functional Group Transformations, Pergamon, Oxford, 1995, vol.6, pp.500-506 ou bien dans Arkiv der Pharmazie, 314(1), 34-41, 1981.

On peut notamment faire réagir :
- une isocytosine avec un acide carbamique activé : 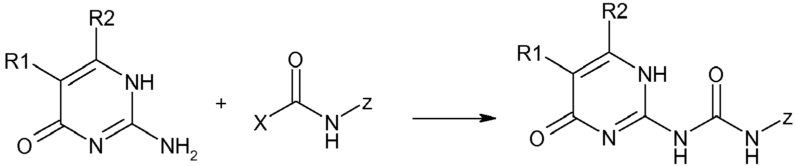
- une isocytosine avec un isocyanate dérivé d'amine : 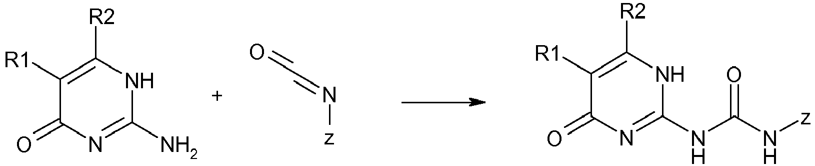
- une isocytosine à fonction carbamique activée avec une aminé : 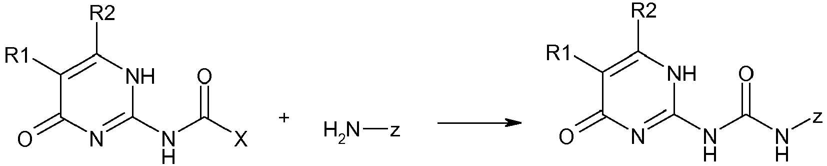
- un β-cétoester avec un dérivé guanylalkylurée :

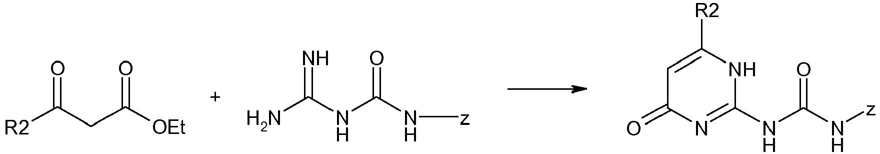

Un autre procédé de préparation des composés selon l'invention consiste à synthétiser un composé du type : 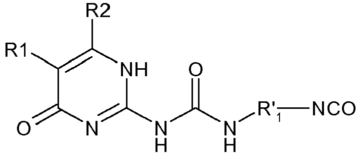 puis à le faire réagir sur un alcool ou une amine.
Dans cette formule, le groupement divalent R'1 représente par exemple un groupe choisi parmi 1,2-éthylène; 1,6-hexylène; 1,4-butylène, 1,6-(2,4,4-triméthyl hexylène); 1,4-(4-méthylpentylène), 1,5-(5-méthylhexylène), 1,6-(6-méthylheptylène); 1,5-(2,2,5-triméthylhexylène), 1,7-(3,7-diméthyloctylène); isophorone; 4,4'-méthylènebiscyclohexylène, tolylène, 2-méthyl,1,3-phénylène; 4-méthyl,1,3-phénylène; 4,4-biphénylèneméthylène; et de préférence isophorone; -(CH₂)₂-;-(CH₂)₆-; -CH₂CH(CH₃)-CH₂-C(CH₃)₂-CH₂-CH₂; 4,4'-méthylènebiscyclohexylène; 2-méthyl,1,3-phénylène. Encore mieux, R'₁ = isophorone ou 1,6-hexylène.
De préférence, R1=H et R2=méthyle.

De façon particulièrement préférée, les composés de formule (I) sont solubles ou dispersibles dans l'eau, ou solubles dans de l'eau basifiée à l'aide d'une solution d'ammoniaque à 28% (le pH de la solution est alors compris entre 8 et 10), ou solubles dans de l'eau acidifiée à l'aide d'acide citrique.
Par soluble, on entend que le composé forme une solution limpide, à une concentration de 0,1% en poids dans le milieu, à 25°C, 1 atm.

Par dispersible, on entend que le composé forme dans le milieu, à une concentration de 0,1% en poids à 25°C, 1 atm., une suspension ou dispersion stable de fines particules, généralement sphériques. Par stable, on entend que la suspension ne précipite pas et ne présente donc pas de dépôt visible. La taille moyenne des particules constituant la suspension ou la dispersion est de préférence inférieure à 1 µm et plus généralement varie entre 5 et 400 nm, de préférence 10 à 250 nm. Ces tailles de particules sont mesurées par toute méthode classique de diffusion de lumière.

Les composés selon l'invention trouvent une application toute particulière dans le domaine de la cosmétique, notamment dans le domaine capillaire.
La quantité de composé présent dans les compositions cosmétiques de l'invention dépend bien entendu du type de composition et des propriétés recherchées, et peut varier à l'intérieur d'une gamme très large, comprise généralement entre 0,001% et 30% en poids, de préférence entre 0,005% et 15% en poids, notamment entre 0,01% et 10% en poids, voire entre 0,1 et 5% en poids, par rapport au poids total de la composition.
Les compositions cosmétiques peuvent bien évidemment comprendre un mélange de composés de formule (I).

Dans un mode de réalisation particulier de l'invention, les compositions cosmétiques peuvent comprendre, outre les composés de formule (I), des composés de formule (II) tels que définis ci-après.
On a en effet constaté que lorsque l'on emploie un mélange de composés (I) et de composés (II), on observe une modification des propriétés mécaniques des cheveux, qui peut être une augmentation ou une diminution de leur rigidité, en fonction de la nature des composés employés.
De préférence, les compositions peuvent comprendre les composés (II), seuls ou en mélange, en une quantité de 0,001 % et 30% en poids, de préférence entre 0,005% et 15% en poids, notamment entre 0,01% et 10% en poids, voire entre 0,1 et 5% en poids, par rapport au poids total de la composition.

Les composés additionnels répondent donc à la formule (II) : 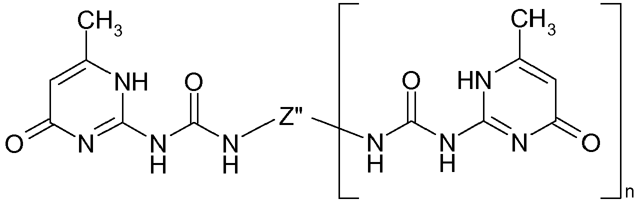 dans laquelle :
- n = 1 ou 2, et
- Z représente un radical divalent ou trivalent (selon la valeur de n), alkyle en C1-C32 ou alcényle en C2-C32, linéaire ou ramifié; de préférence en C2-C18, notamment en C3-C12;
   éventuellement substitué par 1 à 10, notamment 2 à 8, voire 3 à 6, radicaux choisis parmi -OH, -SO₃H, -COOH, -COOR et -N⁺RR'R", avec R, R' et R" = alkyle en C1-C12, notamment méthyle; et/ou
   éventuellement interrompu par 1 à 10, notamment 2 à 8, voire 3 à 6, groupements choisis parmi (i) les groupements divalents : -S-, -NH- (ou =NH), -O-, -C(O)-, ou de formule : 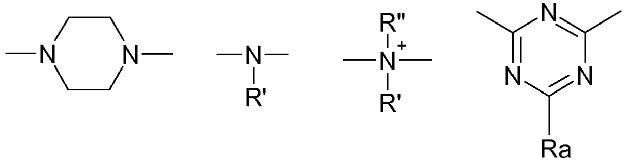 avec Ra = H ou halogène, notamment Cl, ou alkyle en C1-C6, ou liaison simple, et R' et R", identiques ou différents, représentant un atome d'hydrogène ou un radical alkyle en C1-C6, notamment méthyle; et
- (ii) les groupements trivalents de formule : 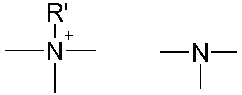 R' représentant un atome d'hydrogène ou un radical alkyle en C1-C6, notamment méthyle; lesdits groupes trivalents étant généralement présents à la jonction desdits radicaux.

Parmi les composés de formule (II) susceptibles d'être employés, on peut citer les composés suivants :

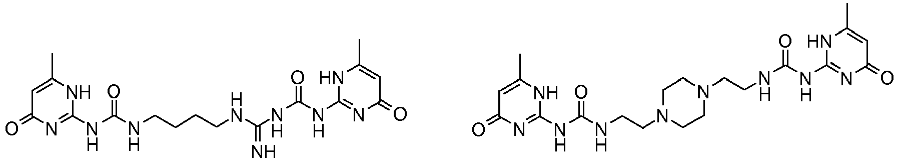

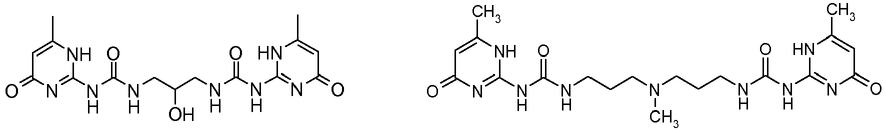

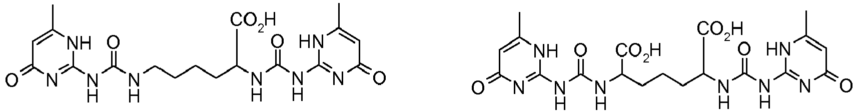

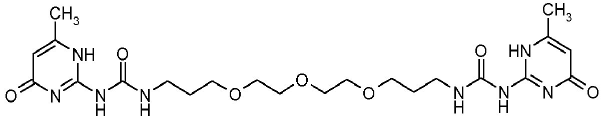

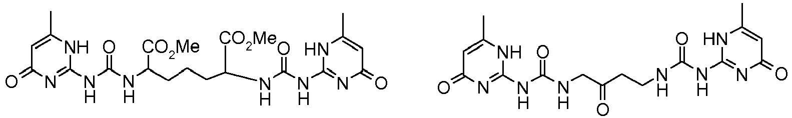

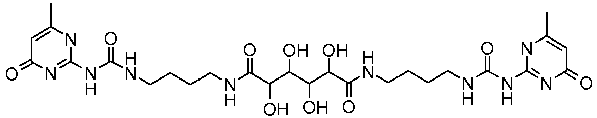

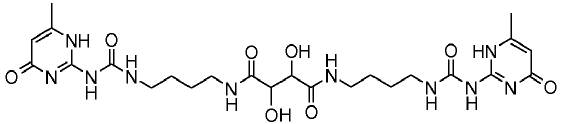

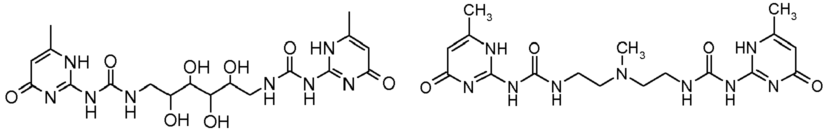

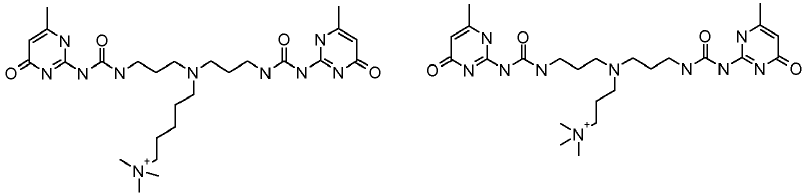

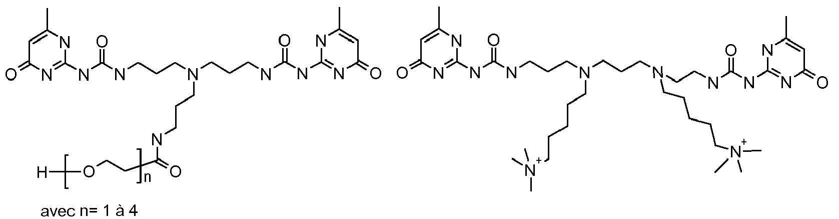

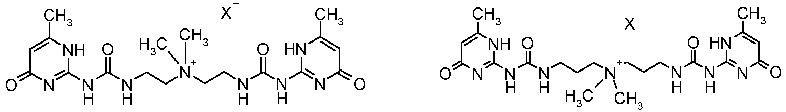

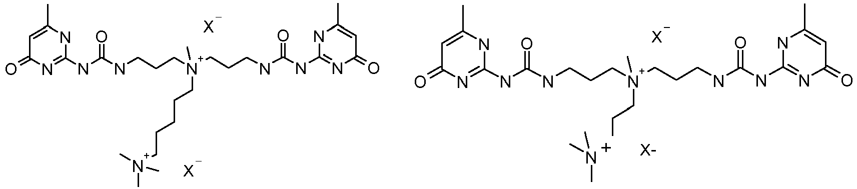

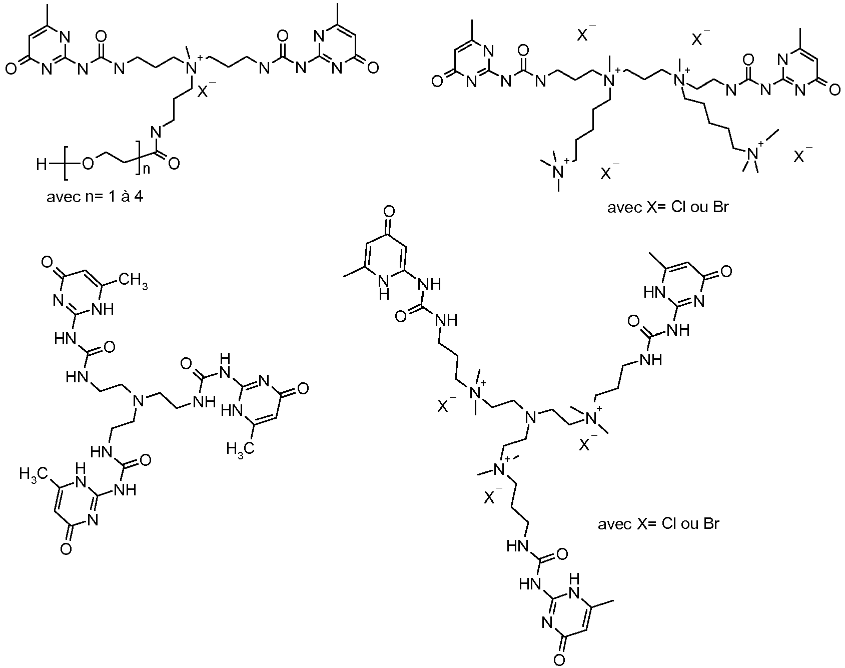

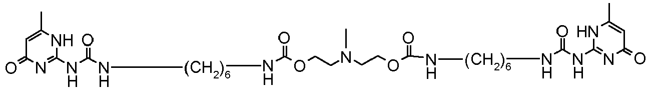

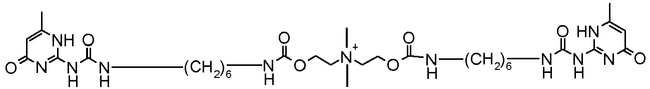

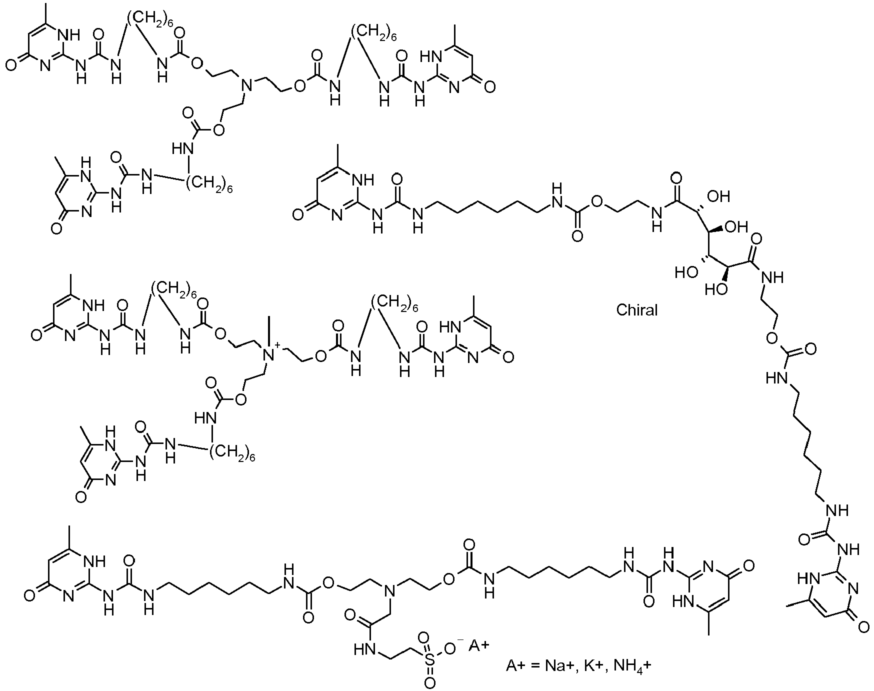

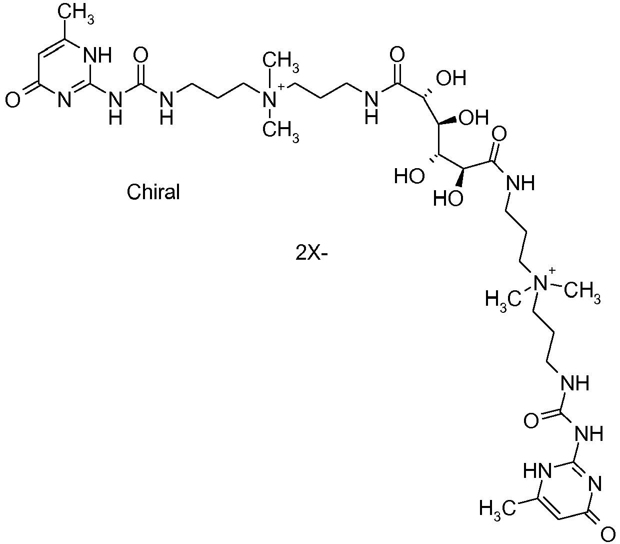

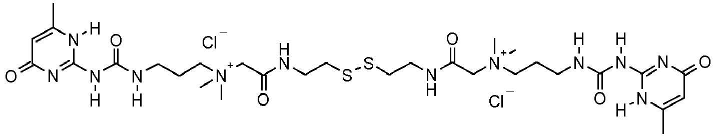

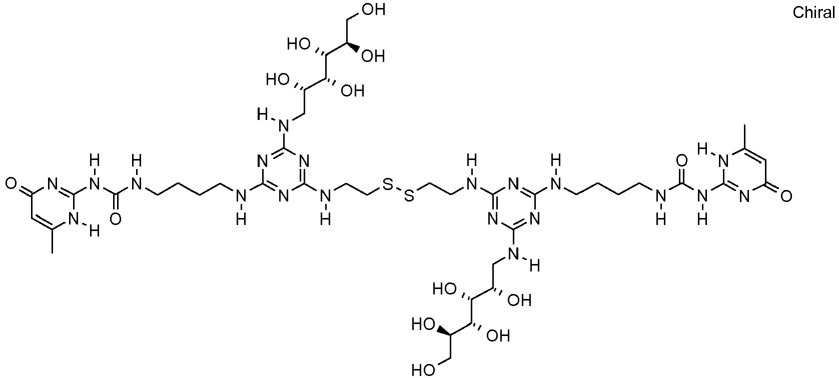

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique. De préférence, la composition se présente sous forme de lotion, sérum ou suspension aqueuse.
Ces compositions peuvent être conditionnées, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

Les compositions selon l'invention comprennent un milieu cosmétiquement acceptable, c'est-à-dire un milieu compatible avec les matières kératiniques, notamment la peau du visage ou du corps, les lèvres, les cheveux, les cils, les sourcils et les ongles.

Ledit milieu comprend au moins un ingrédient cosmétique usuel, notamment choisi parmi les agents propulseurs; les huiles carbonées; les huiles siliconées; les alcools en C8-C40, les esters en C8-C40, les acides en C8-C40; les tensioactifs non ioniques, les tensioactifs cationiques, les tensioactifs anioniques, les tensioactifs amphotères, les tensioactifs zwittérioniques; les filtres solaires; les agents antipelliculaires; les antioxydants; les agents réducteurs; les bases d'oxydation, les coupleurs, les agents oxydants, les colorants directs; les agents défrisants, les agents nacrants et opacifiants; les agents plastifiants ou de coalescence; les hydroxyacides; les pigments; les charges; les polyols; les cires; les épaississants, les émulsionnants; les polymères.
Ledit milieu peut également comprendre un ingrédient additionnel choisi parmi l'eau, les alcools en C1-C7, les cétones, les solvants organiques, les silicones; les cires; les agents de pH.
Ledit milieu peut bien évidemment comprendre plusieurs ingrédients cosmétiques figurant dans les listes ci-dessus.
Selon leur nature et la destination de la composition, les ingrédients peuvent être présents en des quantités usuelles, aisément déterminables par l'homme du métier, et qui peuvent être comprises, pour chaque ingrédient, entre 0,01 à 80% en poids.
Dans un mode de réalisation particulier, la composition selon l'invention peut comprendre un ou plusieurs agents réducteurs, qui peuvent être choisis parmi les thiols par exemple la cystéine, l'homocystéine, l'acide thiolactique, les sels de ces thiols; les phosphines, le bisulfite, les sulfites, l'acide thioglycolique ainsi que ses esters, notamment le monothioglycolate de glycérol et le thioglycerol. Cet agent réducteur peut aussi être choisi parmi les borohydrures et leurs dérivés, comme par exemple les sels de borohydrure, du cyanoborohydrure, du triacetoxyborohydrure, du trimethoxyborohydrure: sels de sodium, lithium, potassium, calcium, ammoniums quaternaires (tétraméthylammonium, tétraéthylammonium, tétra n-butylammonium, benzyltriéthylammonium); le catéchol-borane; et leurs mélanges.

La composition peut notamment comprendre de l'eau, un ou plusieurs alcools en C1-C7, seul ou en mélange avec de l'eau, et notamment un mélange eau/éthanol, eau/isopropanol ou eau/alcool benzylique.

Les huiles carbonées, notamment hydrocarbonées, et/ou les huiles siliconées peuvent être présentes à raison de 0,01 à 20% en poids par rapport au poids total de la composition. On peut notamment citer les huiles végétales, animales ou minérales, hydrogénées ou non, les huiles synthétiques hydrocarbonées, cycliques ou aliphatiques, linéaires ou ramifiées, saturées ou insaturées, telles que par exemple les polyalpha-oléfines, en particulier les polydécènes et polyisobutènes; les huiles de silicone, volatiles ou non, organo-modifiées ou non, hydrosolubles ou non; les huiles fluorées ou perfluorées; leurs mélanges.
Les alcools, les esters et les acides, ayant 8 à 40 atomes de carbone, peuvent être présents à raison de 0,01 à 50% en poids, notamment 0,1 à 20% en poids par rapport au poids total de la composition.
On peut notamment citer les alcools gras à chaînes linéaires ou ramifiées en C12-C32, notamment en C12-C26, et en particulier l'alcool cétylique, l'alcool stéarylique, l'alcool cétylstéarylique, l'alcool isostéarylique, l'octyldodécanol, le 2-butyloctanol, le 2-hexyldécanol, le 2-undécylpentadécanol, l'alcool oléique ou l'alcool linoléique. On peut également citer les alcools gras en C8-C40, notamment C16-C20, alcoxylés, notamment éthoxylés, de préférence comportant de 10 à 50 moles d'oxyde d'éthylène et/ou d'oxyde de propylène, tels que l'oleth-12, le ceteareth-12 et le ceteareth-20, l'alcool stéarylique oxypropyléné notamment comportant 15 moles d'oxyde de propylène, l'alcool laurylique oxyéthyléné, notamment comportant plus de 7 groupes oxyéthylénés, ainsi que leurs mélanges. On peut citer également les acides gras à chaînes linéaires ou ramifiées en C16-C40, et notamment l'acide méthyl-18 eicosanoïque, les acides d'huile de coprah ou d'huile de coprah hydrogénée; l'acide stéarique, l'acide laurique, l'acide palmitique et l'acide oléique, l'acide béhénique, et leurs mélanges. On peut encore citer les esters gras à chaînes linéaires ou ramifiées en C16-C40, tels que les esters de polyols dérivés d'acides gras comportant de 8 à 30 atomes de carbone, et leurs dérivés oxyalkylénés et notamment oxyéthylénés, les polyols étant préférentiellement choisis parmi les sucres, les C2-C6-alkylène glycols, le glycérol, les polyglycérols, le sorbitol, le sorbitan, les polyéthylèneglycols, les polypropylèneglycols et leurs mélanges.
Les silicones peuvent être volatiles ou non; on peut notamment citer les polyorganosiloxanes, modifiés ou non, à savoir les huiles, les gommes et les résines de polyorganosiloxanes, telles quelles ou sous forme de solutions dans des solvants organiques, ou sous forme d'émulsions ou de microémulsions.
L'homme de métier veillera à choisir les ingrédients entrant dans la composition, ainsi que leurs quantités, de manière à ce qu'ils ne nuisent pas aux propriétés des compositions de la présente invention.

La composition cosmétique selon l'invention peut se présenter sous la forme d'un produit de soin, de nettoyage et/ou de maquillage de la peau du corps ou du visage, des lèvres, des sourcils, des cils, des ongles et des cheveux, d'un produit solaire ou autobronzant, d'un produit d'hygiène corporelle, d'un produit capillaire, notamment de soin, de nettoyage, de conditionnement, de coloration des cheveux. Elle trouve notamment une application particulièrement intéressante dans le domaine capillaire, notamment pour le soin, le traitement cosmétique ou le nettoyage des cheveux.

Les compositions capillaires sont de préférence des shampooings, des après-shampooings, des gels, lotions ou crèmes de soin, des conditionneurs, des lotions restructurantes pour cheveux; des lotions ou gels antichute, des shampoings anti-parasitaires, des lotions ou shampoings antipelliculaires, des shampoings traitant antiséborrhéiques. Elles peuvent également être un produit de coloration capillaire, notamment coloration d'oxydation, éventuellement sous forme de shampoing colorant; une composition de permanente, de défrisage ou de décoloration, ou encore une composition à rincer ou non, à appliquer avant ou après une coloration, une décoloration, une permanente ou un défrisage ou encore entre les deux étapes d'une permanente ou d'un défrisage.

La composition selon l'invention trouve une application intéressante pour le soin et le traitement cosmétique, notamment la protection, des cheveux, en particulier des cheveux affaiblis et/ou abîmés, par exemple par des traitements chimiques ou mécaniques; on peut notamment utiliser les composés selon l'invention en post-traitement, après une étape de coloration, de décoloration ou de défrisage des cheveux.

L'invention a donc pour objet un procédé de traitement cosmétique, notamment de maquillage, de soin, de nettoyage, de coloration, des matières kératiniques, notamment de la peau du corps ou du visage, des lèvres, des ongles, des cheveux et/ou des cils, comprenant l'application sur lesdites matières d'une composition cosmétique comprenant au moins un composé selon l'invention.

De préférence, il s'agit d'un procédé de traitement cosmétique pour le conditionnement et/ou le soin des cheveux, en particulier pour leur apporter du corps et/ou de la nervosité, ou améliorer le démêlage, le lissage, la peignabilité, la réparation et la maniabilité de la chevelure. Il peut s'agir d'un procédé de réparation et/ou de protection des cheveux abîmés ou affaiblis.

Dans un mode de réalisation particulier du procédé de traitement, on peut appliquer la composition selon l'invention en une fois ou en plusieurs fois (multi-application).
Ainsi, on peut appliquer une composition comprenant les composés selon l'invention, en une quantité par exemple de 0,05 à 15%, notamment 0,1 à 5% en poids, une première fois sur les cheveux, laisser reposer pendant 2 à 20, notamment 5 à 15 minutes, éventuellement en chauffant à une température inférieure à 65°C, ou bien au fer à lisser ou à friser, par exemple pendant quelques secondes, puis éventuellement sécher les cheveux avant d'appliquer une seconde fois la composition, et à nouveau laisser reposer pendant 2 à 20 minutes, notamment 10 minutes, avant d'éventuellement chauffer à une température inférieure à 65°C, ou bien au fer à lisser ou à friser, par exemple pendant quelques secondes, puis sécher ou laisser sécher. Il est possible d'effectuer une troisième application de la composition. Cette application en plusieurs fois de la composition peut notamment permettre d'améliorer la pénétration des composés à l'intérieur du cheveu, et donc améliorer la réparation du cheveu in situ.

Ainsi qu'indiqué plus haut, les composés selon l'invention trouvent une utilisation toute particulière dans un procédé de traitement cosmétique des cheveux, avant, pendant ou après un traitement cosmétique de coloration, de décoloration, de permanente, de défrisage, ou de lissage des cheveux; ils trouvent également une application dans un procédé d'entretien des cheveux dégradés, notamment par les agressions extérieures telles que les UV, la pollution, les brossages répétés, l'eau chlorée.
Par l'expression "pendant un traitement", on entend une utilisation en mélange avec l'un ou l'autre des éléments du kit de traitement ou entre les différentes étapes d'un traitement, de permanente par exemple.

Ainsi, un autre objet de l'invention est un kit comprenant d'une part une composition de coloration, de décoloration, de permanente, de défrisage ou de lissage des cheveux et d'autre part une composition cosmétique comprenant au moins un composé de formule (I).
Les deux compositions peuvent être appliquées l'une après l'autre, avec rinçage éventuel entre les deux applications; elles peuvent également être mélangées juste avant emploi et application du mélange. Une étape postérieure de rinçage et/ou de séchage peut être envisagée.
Dans un mode de réalisation particulier, l'une des compositions du kit comprend au moins un agent réducteur, en particulier choisi dans la liste donnée plus haut; notamment l'agent réducteur peut être présent dans la composition de coloration, de décoloration, de permanente, de défrisage ou de lissage des cheveux, le composé de formule (I) étant présent dans l'autre composition du kit.

L'invention est illustrée plus en détail dans les exemples de réalisation suivants.

### Exemple 1 : N-(4-aminobutyl)-N'-(6-méthyl-4-oxo-1,4-dihydropyrimidin-2-yl) urée hydrochlorure (1)

### 1/ Préparation de tert-butyl [4-({[(6-methyl-4-oxo-1,4-dihydropyrimidin-2-yl)amino] carbonyl}amino)butyl]carbamate (1B) à partir d'isocytosine à fonction carbamique activée par du carbonyl diimidazole (1A) (mode opératoire n°1):

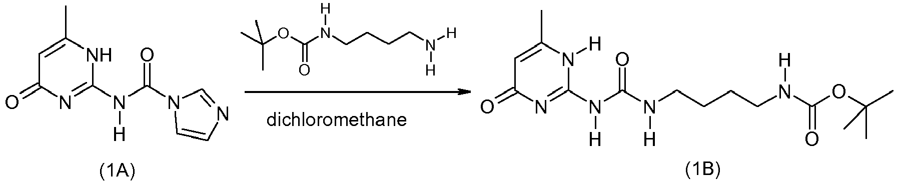

A une solution de 1,72 g de tert-butyl (4-aminobutyl)carbamate (9,1 mmol) dans 50 ml de dichlorométhane, on ajoute 2 g de 2-(1-imidazolylcarbonylamino)-6-méthyl-4[1H]-pyrimidinone 1A (9,1 mmol) préparé selon la procédure décrite par E.W. Meijer et al. J.Am.Chem.Soc. 2003, 125, p6860. La solution est agitée à reflux pendant 4 heures. Le produit final est obtenu par précipitation dans l'acétone. Après filtration et lavage avec de l'acétone, le produit final est séché sous pression réduite et on obtient 4,02 g (11,8 mmol) de produit 1B pur, sous forme d'une poudre blanche avec un rendement supérieur à 99% (produit hygroscopique).
¹H RMN (DMSO) : δ 1.37 ppm (s, 9H), 2.11 ppm (s, 3H), 2.88-2.96 ppm (m,2H), 3.09-3.16 ppm (m, 2H), 3.17-3.20 ppm (m, 4H), 4.07-4.13 ppm (m,1H)

### 2/ Préparation de N-(4-aminobutyl)-N'-(6-methyl-4-oxo-1,4-dihvdropyrimidin-2-yl) urée hydrochlorure (1) (mode opératoire n°2):

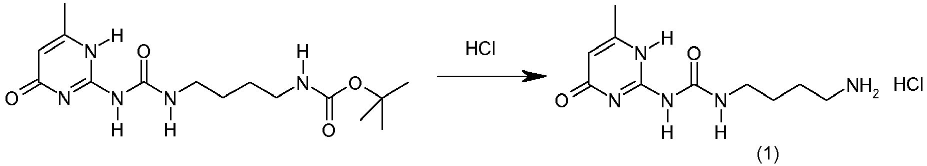

A une solution de 3,02 g de tert-butyl [4-({[(6-methyl-4-oxo-1,4-dihydropyrimidin-2-yl)amino]carbonyl}amino)butyl]carbamate (8,9 mmol) préparé ci-dessus, dans 10 ml d'acétate d'éthyle, on ajoute 9,28 g d'acide chlorhydrique 35% (89.5 mmol). La solution est agitée à 5°C pendant 1 heures. Le produit final est obtenu par précipitation dans l'acétone. Après filtration et lavage avec de l'acétone, le produit final est séché sous pression réduite et on obtient 2,28 g (9.5 mmol) de produit (1) recherché, sous forme d'une poudre blanche avec un rendement supérieur à 99% (produit hygroscopique).
Point de fusion : 131 °C
¹H RMN (DMSO): δ 1.30-1.78 ppm (m, 4H), 2.23 ppm (s,3H), 2.73-2.87 ppm (m,2H), 3.10-3.25 ppm (m, 2H), 6.04 ppm (m, 1H)

### Exemple 2 : N-[3-(dimethylamino)propyl]-N'-(6-méthyl-4-oxo-1,4-dihydro pyrimidin-2-yl) urée (2)

Préparation à partir de 6-méthylisocytosine (2A) et de carbonyl diimidazole (2B) :

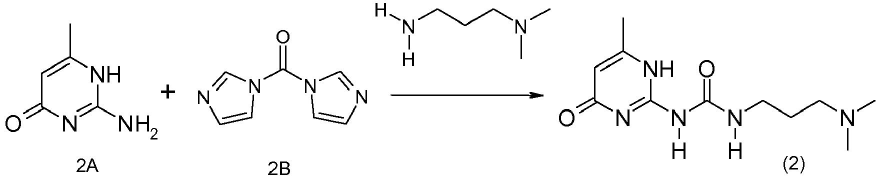

A une suspension de 5 g de 6-methylisocytosine (40 mmol) dans 100 ml d'acétate d'éthyle, on ajoute 8,42 g de carbonyldiimidazole (52 mmol). La solution est agitée pendant 12 heures au reflux, puis 8 heures à température ambiante. On ajoute 5,03 ml de diméthylaminopropylamine (40 mmol). La solution est agitée pendant 6 heures au reflux. Le produit final est obtenu par filtration sur Büchner. Le produit final est séché sous pression réduite et on obtient 9,6 g (38 mmol) de produit recherché, sous forme d'une poudre blanche avec un rendement de 95%.
¹H RMN (DMSO) : δ 1.77 ppm (t,2H), 2.19 ppm (s, 3h), 2.71 ppm (s, 6H), 3.28ppm (t, 2H), 5.84 ppm (s, 1H)

### Exemple 3 : triméthyl-{3-[3-(6-méthyl-4-oxo-1,4-dihydro-pyrimidin-2-yl)-ureido]-propyl}-ammonium chlorure (3)

Préparation à partir d'isocytosine à fonction carbamique activée par du carbonyldiimidazole :

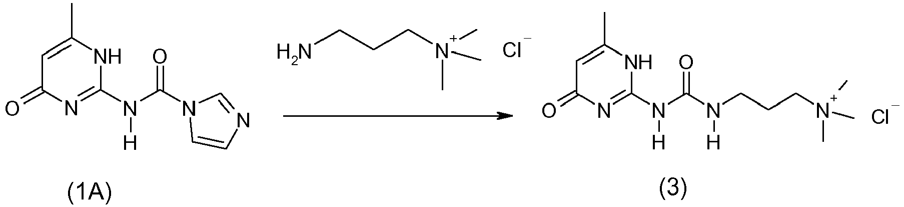

Le produit est préparé selon le mode opératoire n°1 décrit à l'exemple 1.
On obtient 6 g (20.2 mmol) de produit recherché pur, sous forme d'une poudre blanche avec un rendement de 69%.
Point de fusion 143.49°C
¹H RMN (D2O) : δ 1.97-2.07ppm (m, 2H), 2.17-2.22ppm (m, 3H), 3.05-3.08ppm (m, 9H), 3.10-3.13ppm (m, 2H), 3.26-3.32ppm (m, 3H), 3.33-3.37ppm (m, 2H), 5.86-5.98ppm (m, 2H).

### Exemple 4 : 1-[3-(dimethylamino)propyl]-3-(6-{[(6-méthyl-4-oxo-1,4-dihydro pyrimidin-2-yl)carbamoyl]amino}hexyl)urée (4)

Préparation à partir de d'isocytosine à fonction isocyanate :

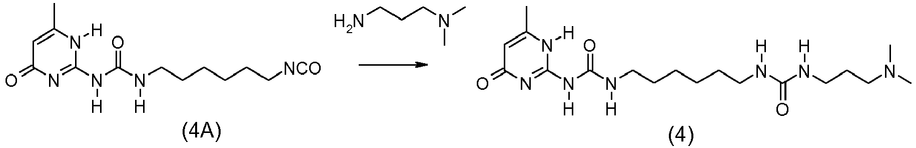

Le produit est préparé selon le mode opératoire n°1 décrit à l'exemple 1, à partir du 1-(6-isocyanatohexyl)-3-(6-methyl-4-oxo-1,4-dihydropyrimidin-2-yl) urée préparé selon la procédure décrite par E.W. Meijer et al. J.Am.Chem.Soc. 2003, 125, p6860.
On obtient 6,2 g (15.7 mmol) de produit recherché pur, sous forme d'une poudre blanche avec un rendement de 92%.
¹H RMN (DMSO+TFA): δ 1.37 ppm (m, 8H) ; 1.76 ppm (t, 2H) ; 2.25 ppm (s, 3H) ; 2.74 ppm (d, 6H) ; 3.07 ppm (m, 8H) ; 6.03 ppm (s, 1H).

### Exemple 5 : N-(4-{[amino(imino)methyl]amino}butyl)-N'-(6-methyl-4-oxo-1,4-dihydropyrimidin-2-yl)urée (5)

Préparation à partir d'isocytosine à fonction carbamique activée par du carbonyl diimidazole (1A) :

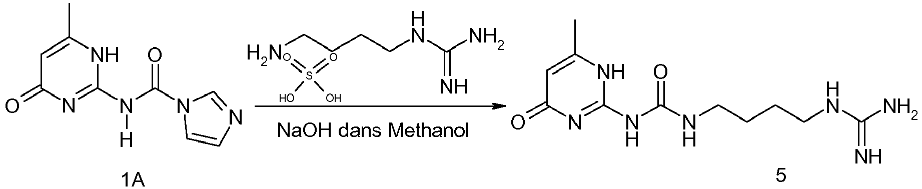

Le produit est préparé selon le mode opératoire n°1.
On obtient 5,8 g (20.6 mmol) de produit recherché pur, sous forme de poudre blanche avec un rendement de 47%.
Point de fusion 197.1 °C
¹H RMN (DMSO + CD₃COOD) : δ 1.48 ppm (m, 4H) ; 2.14 ppm (m, 3H) ; 3.11-3.17 ppm (m, 4H); 5.84 ppm (m, 1H)

### Exemple 6 : 1-(6-méthyl-4-oxo-1,4-dihydro-pyrimidin-2-yl)-3-octadec-9-enyl-urée (6)

Préparation à partir d'isocytosine à fonction carbamique activée par du carbonyl diimidazole 1A :

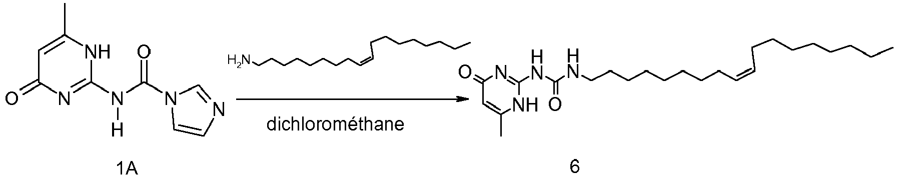

Le produit est préparé selon le mode opératoire n°1.
On obtient 4,2 g (10 mmol) de produit recherché pur, sous forme d'une poudre blanche avec un rendement de 73%.
Point de fusion 114.4°C
¹H RMN (DMSO) : δ 0.85-0.91 ppm (m, 3H), 1.18-1.39ppm (m, 24H), 1.54-1.64ppm (m, 2H), 1.94-2.05ppm (m, 2H), 2.14-2.39ppm (m, 3H), 3.15-3.43ppm (m, 2H), 5.20-5.55ppm (m, 2H), 5.67-5.97ppm (m, 1H).

### Exemple 7 : acide (2S)-6-amino-2-({[(6-méthyl-4-oxo-1,4-dihydropyrimidin-2-yl)amino]carbonyl}amino)hexanoïque (7)

### 1/ Préparation de tert-butvl (2S)-6-[(tert-butoxycarbonyl)amino]-2-({[(6-methyl-4-oxo-1,4-dihydropyrimidin-2-yl)amino]carbonyl}amino)hexanoate (7A) à partir d'isocytosine à fonction carbamique activée par du carbonyl diimidazole (1A) :

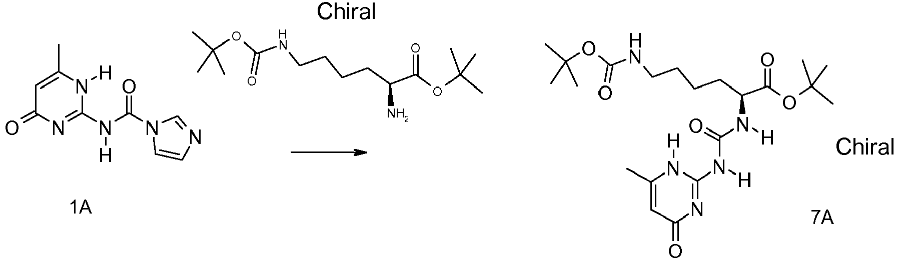

Le produit (7A) est préparé selon le mode opératoire n°1.
On obtient 4,6 g (10.1 mmol) de produit recherché pur, sous forme d'une poudre blanche avec un rendement de 69%.
¹H RMN (DMSO) : δ 1.26 ppm (m, 2H); 1.37 ppm (s, 9H); 1.42 ppm (s, 9H); 1.65 ppm (m, 2H); 2.13 ppm (s, 3H); 2.89-2.91 ppm (q, 2H); 4.14-4.15 ppm (m, 1H); 5.81 ppm (s, 1H).

### 2/ Préparation du produit recherché (7) à partir de (7A) :

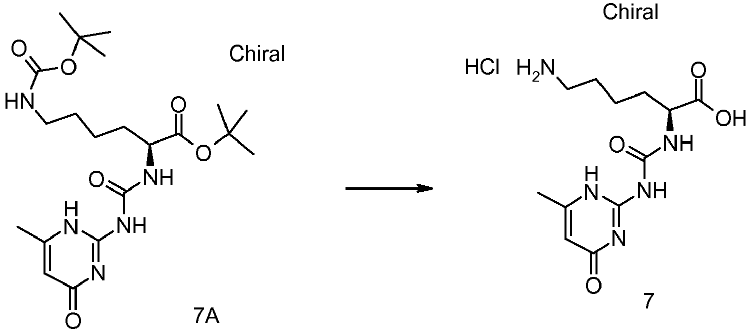

Le produit recherché (7) est préparé selon le mode opératoire n°2 décrit à l'exemple 2.
On obtient 4,5 g (15.2 mmol) de produit pur, sous forme de poudre blanche avec un rendement >99%.
¹H RMN (DMSO) : δ 1.39 ppm (m, 2H), 1.56-1.58 ppm (m, 2H) ; 1.90 ppm (m, 2H); 2.18 ppm (s, 3H); 2.75 ppm (m, 2H) ; 4.19-4.20 ppm (d, 1H) ; 5.93 ppm (s, 1H).

### Exemple 8 : acide (2S)-2-({[(6-méthyl-4-oxo-1,4-dihydropyrimidin-2-yl)amino] carbonyl}amino)pentanedioïque (8)

### 1/ Préparation de di-tert-butvl (2S)-2-({[(6-methyl-4-oxo-1,4-dihydropyrimidin-2-yl)amino]carbonyl}amino)pentanedioate (8A) à partir d'isocytosine à fonction carbamique activée par du carbonyl diimidazole (1A) :

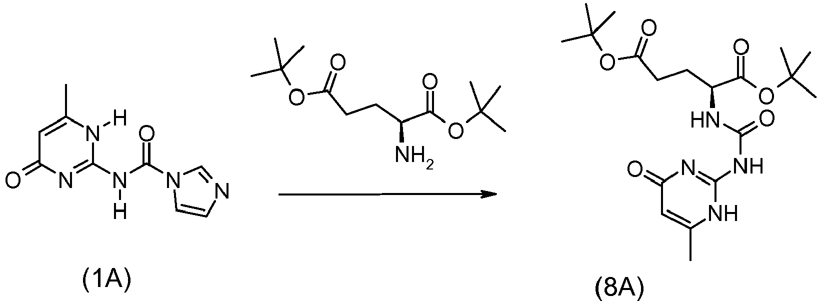

Le produit (8A) est préparé selon le mode opératoire n°1.
On obtient 11,7 g (28.5 mmol) de produit (8A) pur sous forme de poudre blanche avec un rendement >99%.
¹H RMN (DMSO) : δ 1.38-1.46 ppm (d, 18H); 1.78-1.86 ppm (m, 1H), 1.78-1.86 ppm (m, 1H) ; 2.14 ppm (s, 3H) ; 2.24-2.31 ppm (m, 2H) ; 4.17-4.27 ppm (m, 1H) ; 5.82 ppm (s, 1H).

### 2/ Préparation d'acide (2S)-2-({[(6-méthyl-4-oxo-1,4-dihydropyrimidin-2-yl)amino] carbonyl}amino)pentanedioïque (8) :

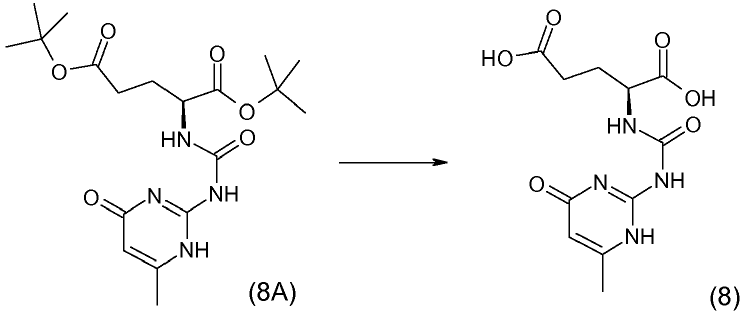

Le produit est préparé selon le mode opératoire n°2.
On obtient 9,8 g (32.9 mmol) de produit (8) pur sous forme de poudre blanche avec un rendement >99%.
¹H RMN (D2O) : δ 2.05 ppm (m, 1H), 2.24 ppm (m, 1H) ; 2.27 ppm (s, 3H) ; 2.5 ppm (m, 2H) ; 4.42-4.43 ppm (m, 1H) ; 6.04 ppm (s, 1H).

### Exemple 9 : (2R,3R,4R,5R)-2,3,5,6-tetrahydroxy-N-(4-{[(6-methyl-4-oxo-1,4-dihydropyrimidin-2-yl)carbamoyl]amino}butyl)-4-{[(2S,3R,4S,5R,6R)-3,4,5-trihydroxy-6-(hydroxymethyl)tetrahydro-2H-pyran-2-yl]oxy}hexanamide (10)

Préparation de (10) à partir de N-(4-aminobutyl)-N'-(6-methyl-4-oxo-1,4-dihydro-pyrimidin-2-yl)urée hydrochlorure (1) (mode opératoire n°3) :

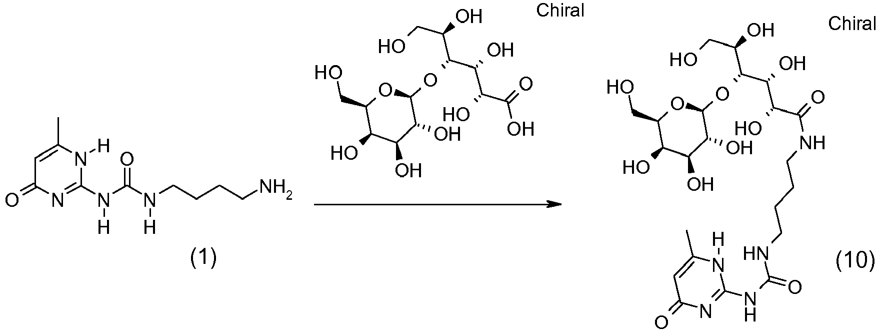

A une suspension de 0,5 g d'acide lactobionique (1,4 mmol) dans 40 ml d'éthanol sont ajoutés 0,35 g de N-(4-aminobutyl)-N'-(6-methyl-4-oxo-1,4-dihydropyrimidin-2-yl)urée hydrochlorure (1,27 mmol) et 0,32 g de diisopropyléthylamine (2,56 mmol). La solution est agitée pendant 6 heures au reflux. Le produit final est obtenu par purification sur phase inverse éluant acétonitrile/eau 5/5 avec 0,2% d'acide trifluoroacétique.
On obtient 0,45 g (0,78 mmol) de produit (10) pur sous forme de poudre blanche avec un rendement de 62%.
¹H RMN (DMSO) : δ 1.47 ppm (m, 4H); 2.12 ppm (s, 3H); 3.12 ppm (m, 4H); 3.25-4.36 ppm (m, 13H); 5.78 ppm (s, 1H)

### Exemple 10 : (2-{2-[3-(6-méthyl-4-oxo-1,4-dihydro-pyrimidin-2-yl)-ureido]-ethyldisulfanyl}-éthyl)-carbamic acide tert-butyl ester (11)

Préparation de (11) à partir d'isocytosine à fonction carbamique activée par du carbonyl diimidazole (1A) :

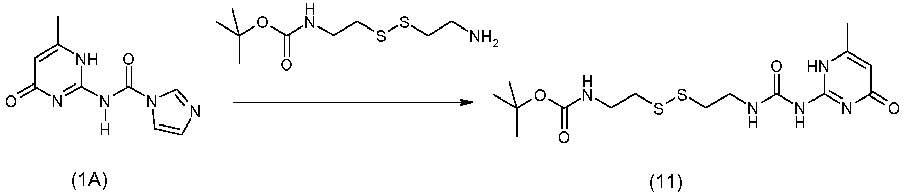

Le produit (11) est préparé selon le mode opératoire n°1.
On obtient 4,8 g (10 mmol) de produit (11) pur sous forme de poudre blanche avec un rendement de 86%.
Point de fusion : 182.15°C
¹H RMN (DMSO) : δ 1.31-1.45ppm (s, 9H), 2.11-2.14ppm (m, 3H), 2.73-2.81 ppm (m, 2H), 2.81-2.90ppm (m,2H), 3.15-3.26ppm (m, 2H), 3.41-3.51 ppm (m, 2H), 5.76-5.81 ppm (m, 1H).

### Exemple 11 : 4-[(4-{[(6-methyl-4-oxo-1,4-dihydropyrimidin-2-yl)carbamoyl] amino}butyl)amino]-4-oxobutyl imidothiocarbamate hydrochlorure (12)

### 1/ Préparation du 4-chloro-N-(4-{[(6-methyl-4-oxo-1,4-dihydropyrimidin-2-yl)carbamoyl]amino}butyl)butanamide (12A) à partir de N-(4-aminobutyl)-N'-(6-methyl-4-oxo-1,4-dihydropyrimidin-2-yl)urée hydrochlorure (1) :

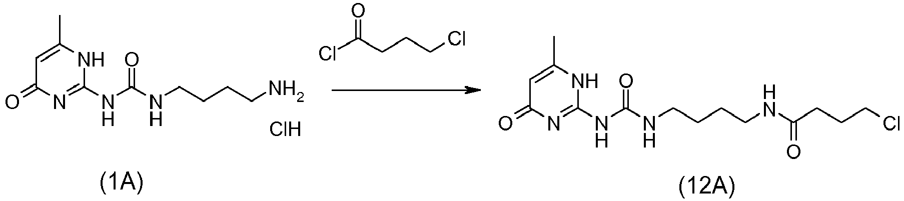

A une solution de 20 g de N-(4-aminobutyl)-N'-(6-méthyl-4-oxo-1,4-dihydropyrimidin-2-yl)urée hydrochlorure (1) (70 mmol) dans 200 ml d'eau refroidie à 0°C, on ajoute 11,6 g d'hydroxyde de sodium (0.29 mol) et 24,4 ml de chlorure de chlorobutyle (0.22 mol). La solution est agitée pendant 18 heures à température ambiante. Le produit obtenu est filtré puis séché sous pression réduite.
On obtient 14,2 g (41.4 mmol) de produit (12A) pur sous forme de poudre blanche avec un rendement de 54%.
¹H RMN (DMSO) : δ 1.41 ppm (s, 4H); 1.93 ppm (m, 2H); 2.10 ppm (s, 3H); 2.20 ppm (t, 2H); 3.09 ppm (m, 2H); 3.62 ppm (m, 2H); 5.79 ppm (s, 1H)

### 2/ Préparation de (12) à partir de 4-chloro-N-(4-{[(6-methyl-4-oxo-1,4-dihydropyrimidin-2-yl)carbamoyl]amino}butyl)butanamide (12A) :

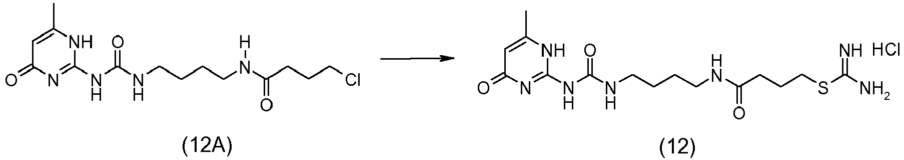

A une solution de 10,7 g de 4-chloro-N-(4-{[(6-methyl-4-oxo-1,4-dihydropyrimidin-2-yl)carbamoyl]amino}butyl)butanamide (12A) (3 mmol) dans 100 ml de 2-propanol, on ajoute 2,84 g de thiourée (4 mmol). La solution est agitée pendant 17 heures au reflux. Le produit obtenu est filtré puis séché sous pression réduite.
On obtient 10,7 g (25.5 mmol) de produit (12) pur sous forme de poudre jaune avec un rendement de 82%.
¹H RMN (DMSO) : δ 1.44 ppm (t, 4H); 1.83 ppm (q, 2H); 2.09 ppm (s, 3H); 2.24 ppm (t, 2H); 3.11 ppm (m, 6H); 5.76 ppm (s, 1H)

### Exemple 12 : 1-[2-(3,4-dihydroxyphenyl)ethyl]-3-(6-méthyl-4-oxo-1,4-dihydro pyrimidin-2-yl)urée (13)

Préparation de (13) à partir d'isocytosine à fonction carbamique activée par du carbonyl diimidazole (1A) :

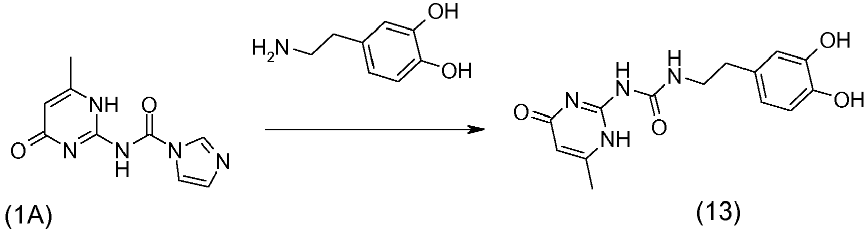

Le produit est préparé selon le mode opératoire n°1.
On obtient 11,1 g (36.5 mmol) de produit (13) pur sous forme de poudre blanche avec un rendement de 80%.
Point de fusion : 266.28 °C
¹H RMN (DMSO) : δ 1.95-2.15 ppm (s, 3H) ; 2.52-2.62 ppm (t, 2H) ; 5.76 ppm (s, 1H) ; 3.33 ppm (t, 2H); 6.46-6.66 ppm (m, 3H).

### Exemple 13 : amélioration du défrisage

On applique sur des cheveux (à l'origine frisés de frisure IV) défrisés par un produit de défrisage alcalin (Goldys - actif soude 2%), la composition suivante, avec un rapport quantité de produit/quantité de cheveux (g/g) de 2, sans rinçage :
- 5% en poids de composé (2) de l'exemple 2
- qsp 100% eau distillée
On effectue les observations à T0.
Les mèches sont ensuite lavées 1 fois ou 5 fois à l'aide d'un shampoing Ultra Doux (Garnier) puis séchées. On effectue les observations à T1 (après 1 shampoing) et à T5 (après 5 shampoings).
Les mèches témoins sont traitées avec une composition comprenant 100% d'eau distillée.

On constate qu'avec un traitement selon l'invention, à T0, le rendement de défrisage est amélioré par rapport à un défrisage ayant subi le post-traitement témoin (seulement constitué d'eau distillée). Cette amélioration du rendement de défrisage est rémanente à 1 et 5 shampooings.
Une mesure de porosité par sonde fluorescente des différentes mèches de cheveux montre que l'amélioration du rendement de défrisage à T0 ne s'accompagne pas d'une augmentation de la porosité interne du cheveu : en effet, le cheveu témoin et le cheveu post-traité selon l'invention présentent une porosité équivalente, après 5 shampooings.
Par un post-traitement selon l'invention, sur cheveux défrisés à la soude, il est possible d'obtenir des performances de défrisage supérieures à celles obtenues sans traitement, sans abîmer la fibre davantage.

### Exemple 14 : diminution de la concentration en agent alcalin de la formulation de défrisage

La composition de l'exemple 14 est appliquée en post-traitement d'une formule de défrisage contenant 1,75% en poids de soude (soit une diminution de 0,25g% de soude par rapport à une formule de défrisage usuelle à 2% de soude).

On observe des performances de défrisage identiques à T0 entre :
- la formule de défrisage usuelle (2% de soude), sans post-traitement et
- la formule de défrisage moins concentrée en soude (1,75% de soude), dont l'application est suivie du post-traitement avec la composition de l'exemple 14.

### Exemple 15

Application d'un mélange (14) + (1) sur mèches de cheveux sensibilisés. 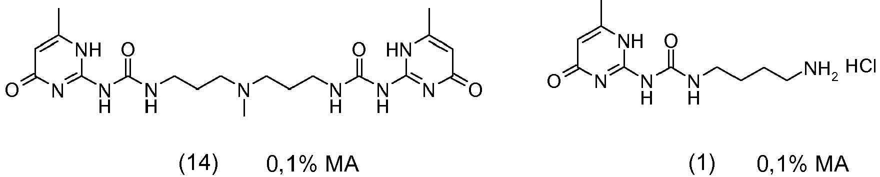 MA : matière active

### 1/ Préparation de la solution

On mélange :
- 30 mg de N,N"-[(methylimino)dipropane-3,1-diyl]bis[N'-(6-methyl-4-oxo-1,4-dihydropyrimidin-2-yl)urée] (14) (0.06 mmol) et
- 3 mg de 1-(4-aminobutyl)-3-(6-methyl-4-oxo-1,4-dihydropyrimidin-2-yl)urée hydrochlorure (0.12 mol) (1) préparé à l'exemple 1,
- dans 29,94 ml d'eau.

Le mélange est agité vigoureusement pendant 30 minutes à température ambiante. On obtient une solution translucide incolore.

### 2/ Application sur cheveu

Les essais sont réalisés sur des mèches de cheveu sensibilisé, de 1 gramme.

Les cheveux sont humidifiés à l'eau, shampooinés avec 0,4 g de shampooing constitué de 15% de sodium laurylsulfate dans l'eau, puis rincés.

Les cheveux sont préalablement traités avec 10 ml d'une solution réductrice d'acide thioglycolique à 0,2N pendant 15 minutes à 30°C. Les cheveux sont ensuite rincés à l'eau.

Les cheveux subissent le post-traitement suivant, répété trois fois : on applique 10 ml de solution préparée ci-dessus, sur la mèche de cheveu pendant 10 minutes à 60°C. La mèche est ensuite essorée puis laissée 10 minutes à température ambiante.

On observe une augmentation significative de la rigidité de la mèche post-traitée selon l'invention (environ 15% d'augmentation), en comparaison avec une mèche témoin, post-traitée avec de l'eau.

### Exemple 16

De façon comparable à l'exemple 15, on applique un post-traitement à l'aide d'une composition comprenant le composé (5) préparé à l'exemple 5, à la place du composé (1).

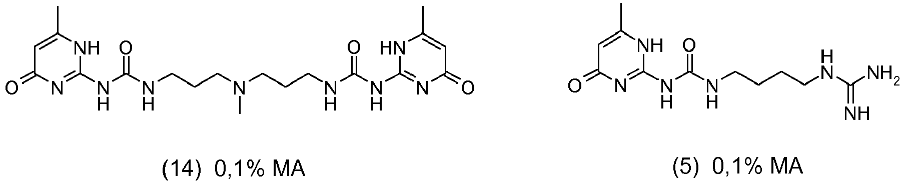

On observe une augmentation significative de la rigidité de la mèche post-traitée selon l'invention (environ 23% d'augmentation), en comparaison avec une mèche témoin, post-traitée avec de l'eau.

### Exemple 17

De façon comparable à l'exemple 15, on applique un post-traiteemnt à l'aide d'une composition comprenant le composé (15) à la place du composé (14).

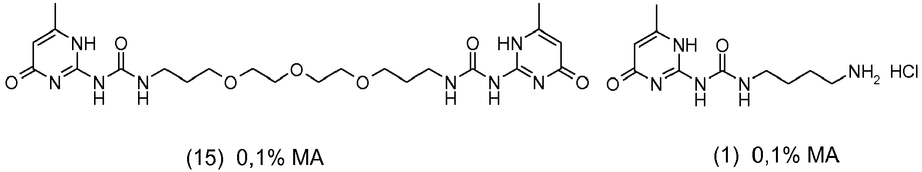

On observe une augmentation significative de la rigidité de la mèche post-traitée selon l'invention (environ 33% d'augmentation), en comparaison avec une mèche témoin, post-traitée avec de l'eau.

## Revendications

1. Composition cosmétique comprenant, dans un milieu physiologiquement acceptable, au moins un composé de formule (I), ses sels, ses sels d'addition, ses solvates notamment hydrates, les formes tautomères étant inclues : 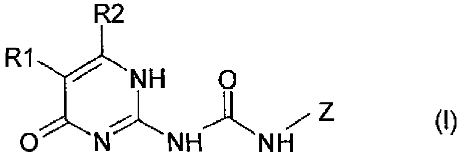 dans laquelle :
- R1 et R2, indépendamment l'un de l'autre, représentent H, -OH, -NRR' (avec R, R', identiques ou différents, étant H ou un radical alkyle, linéaire ou ramifié en C1-C12); ou un groupe carboné, notamment alkyle, linéaire, ramifié et/ou cyclique, en C1-C18, saturé ou non, éventuellement aromatique, pouvant contenir un ou plusieurs hétéroatomes O, N et/ou S, notamment OH, COOH et/ou NRR';
- Z représente un radical monovalent choisi parmi :
(i) un atome d'hydrogène,
(ii) un radical alkyle (saturé) linéaire ou ramifié, en C1 à C32;
(iii) un radical alcène (insaturé) linéaire ou ramifié, en C2 à C32, comprenant une ou deux doubles liaisons C=C;
(iv) un radical alkyle en C1-C32, notamment C2-C24, voire C3-C18, encore mieux C4-C12, substitué par un aryle en C6-C10, notamment C6-C8; en particulier phényle;
lesdits radicaux pouvant éventuellement être substitués par 1 à 8 groupements choisis parmi -OH, -OR, -SH, -SR, -SO₃H, -SO₃R, -SO₂NRR', -COOH, -COOR,-CONRR', -NR-C(O)-NRR', -NRR' et -N⁺RR'R", avec R, R' et R" = H ou alkyle en C1-C6; et les substituants de formule (a) à (h) ci-dessous : 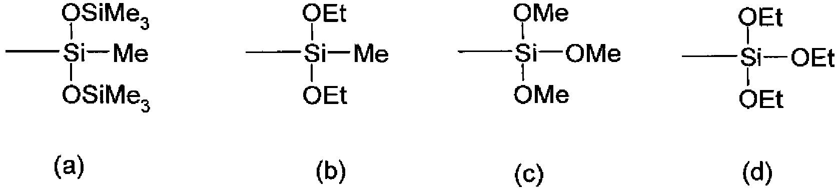 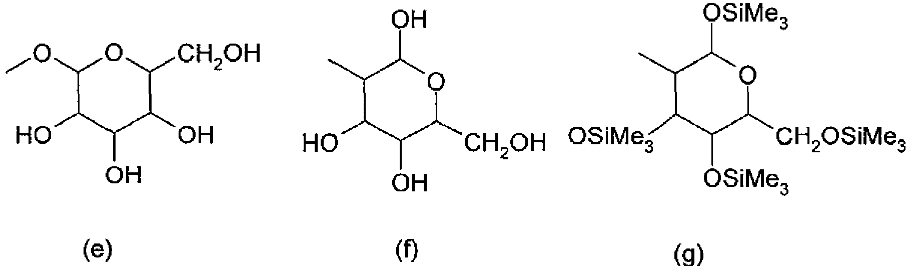 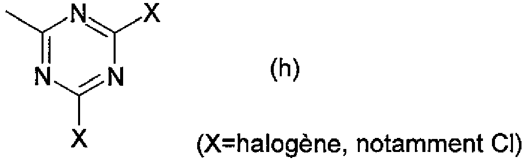 et/ou lesdits radicaux pouvant éventuellement comprendre 1 à 8 groupements choisis parmi, seuls ou en mélange, -S-, S(O), SO₂, -NH- (ou =NH), -N= (trivalent), -O-, -C(O)-, -C(=NH)-, -N⁺(CH₃)₂-An⁻ (An⁻ : anion),
le milieu cosmétiquement acceptable comprenant au moins un ingrédient choisi parmi les agents propulseur, les huiles carbonées ; les alcools en C8-C40, les esters en C8-C40, les acides en C8-C40 ; les tensioactifs non ioniques, les tensioactifs cationiques, les tensioactifs anioniques, les tensioactifs amphotères, les tensioactifs zwittérionigues; les filtres solaires; les agents antipelliculaires; les antioxydants; les agents réducteurs; les bases d'oxydation, les coupleurs, les agents oxydants, les colorants directs; les agents défrisants, les agents nacrants et opacifiants; les agents plastifiants ou de coalescence; les hvdroxyacides; les pigments; les charges; les polyols, les cires; les épaississants, les émulsionnants; les polymères.

2. Composition selon la revendication 1, dans laquelle R1 représente H et R2 représente H, CH₃, C₇H₁₅, C₁₃H₂₇ ou aryle.

3. Composition selon l'une quelconque des revendications précédentes, dans laquelle les composés de formule (I) sont choisis parmi les composés suivants :

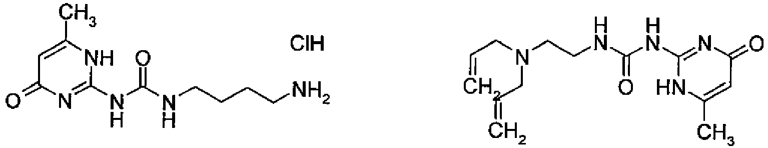

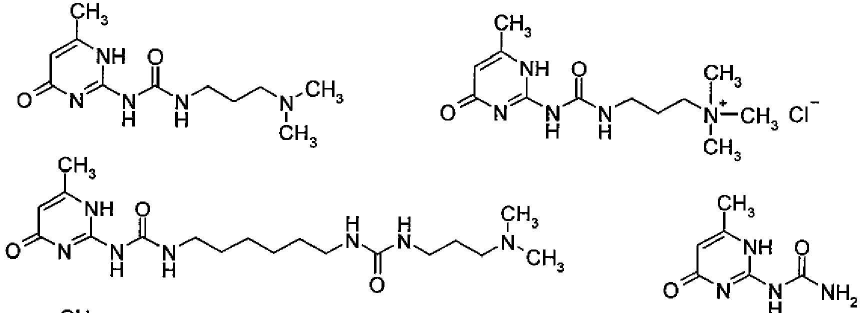

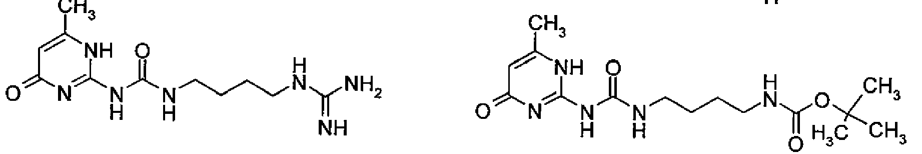

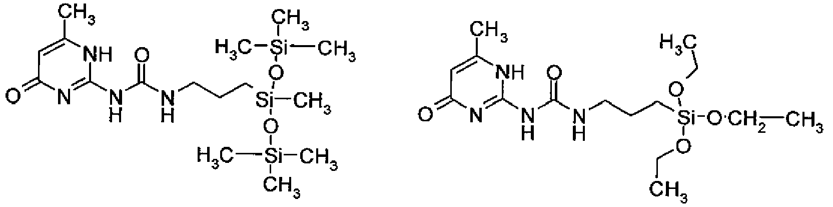

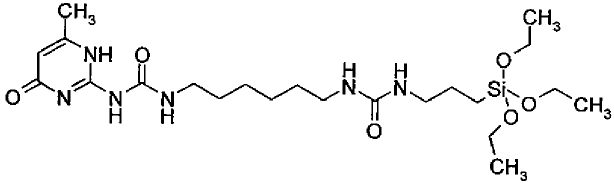

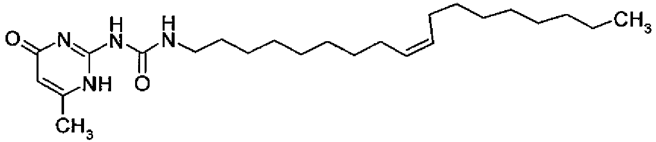

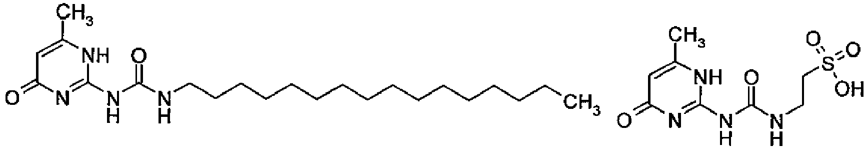

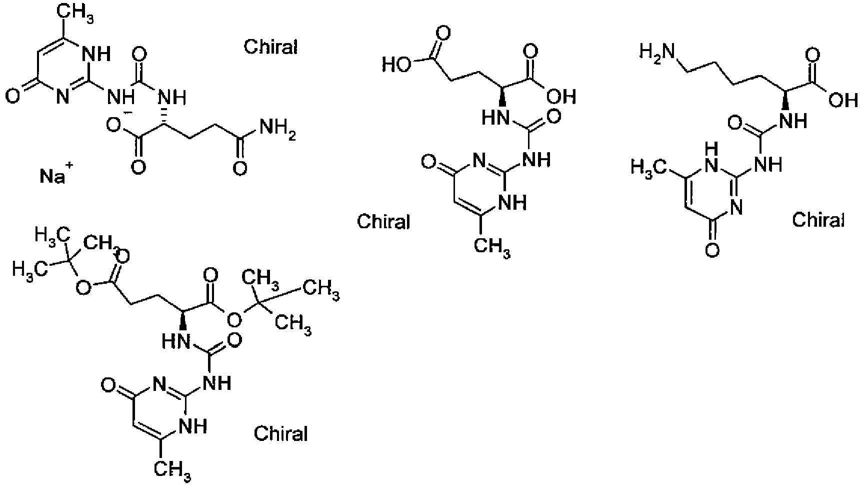

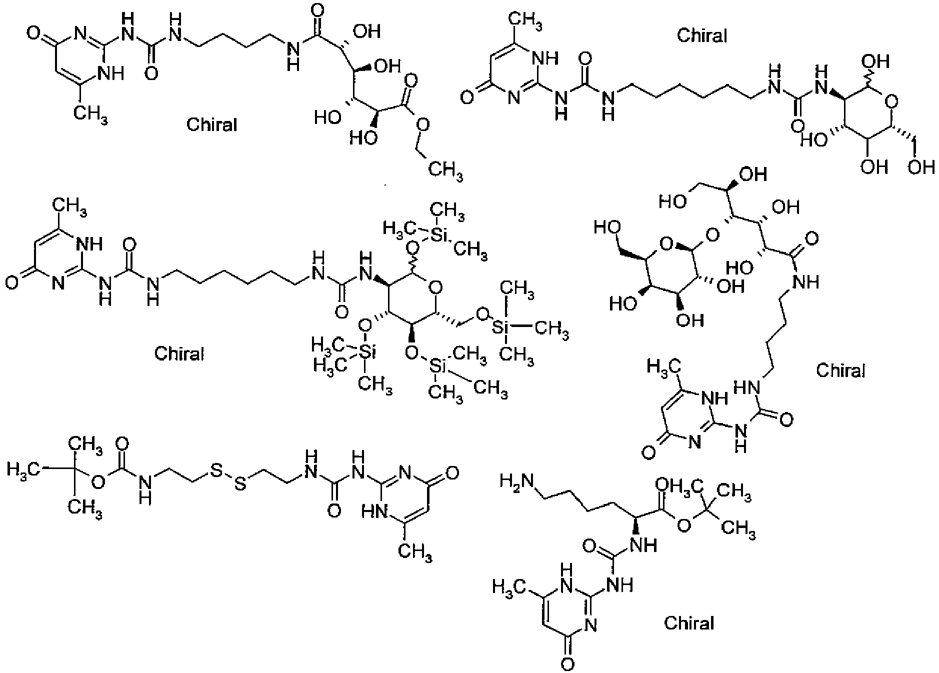

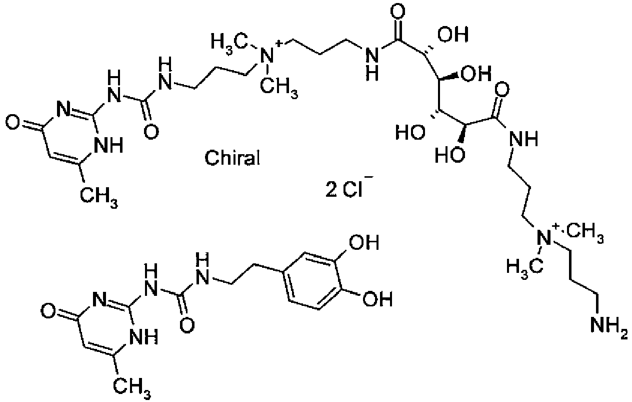

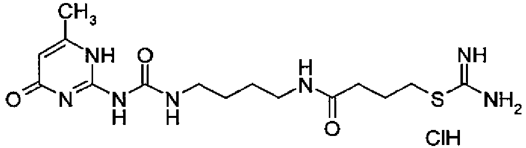

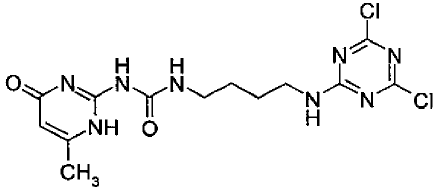

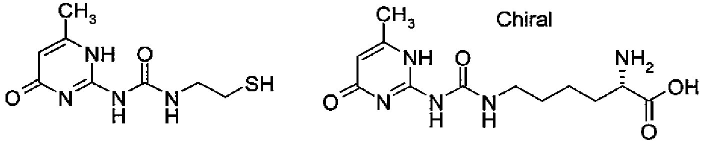

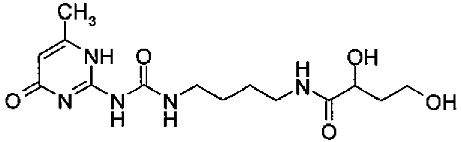

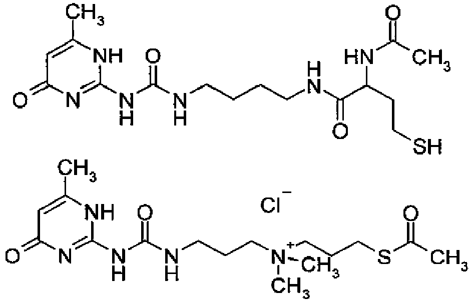

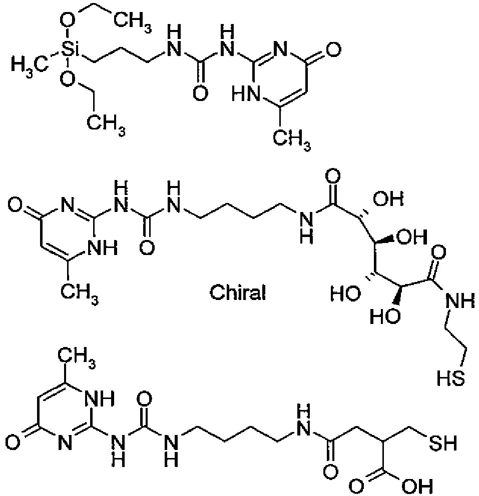

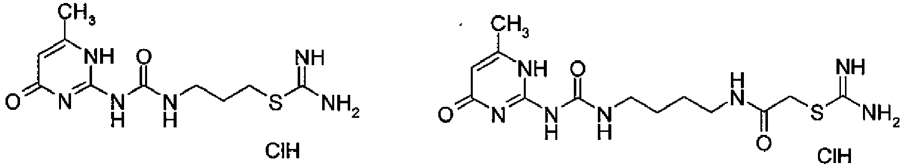

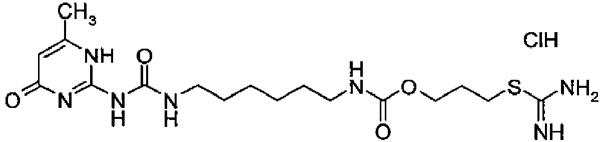

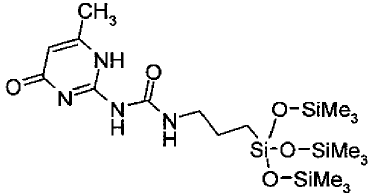

4. Composition selon l'une quelconque des revendications précédentes, dans laquelle le composé de formule (I), seul ou en mélange, est présent en une quantité comprise entre 0,001% et 30% en poids, de préférence entre 0,005% et 15% en poids, notamment entre 0,01% et 10% en poids, voire entre 0,1 et 5% en poids, par rapport au poids total de la composition.

5. Composition selon l'une quelconque des revendications précédentes, comprenant en outre au moins un composé additionnel de formule (II) : 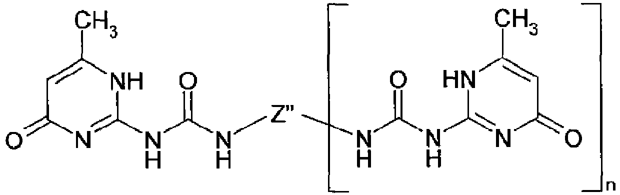 dans laquelle :
- n = 1 ou 2, et
- Z représente un radical divalent ou trivalent (selon la valeur de n), alkyle en C1-C32 ou alcényle en C2-C32, linéaire ou ramifié;
éventuellement substitué par 1 à 10 radicaux choisis parmi -OH, -SO₃H, -COOH,-COOR et -N⁺RR'R", avec R, R' et R" = alkyle en C1-C12, et/ou
éventuellement interrompu par 1 à 10 groupements choisis parmi (i) les groupements divalents : -S-, -NH- (ou =NH), -O-, -C(O)-, ou de formule : 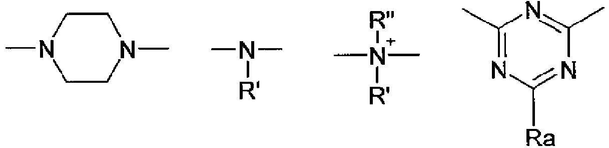
avec Ra = H ou halogène, ou alkyle en C1-C6, ou liaison simple, et
R' et R", identiques ou différents, représentant un atome d'hydrogène ou un radical alkyle en C1-C6; et
- (ii) les groupements trivalents de formule : 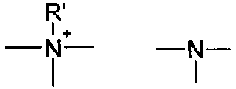 R' représentant un atome d'hydrogène ou un radical alkyle en C1-C6.

6. Composition selon la revendication 5, dans laquelle le composé de formule (II), seul ou en mélange, est présent en une quantité de 0,001% et 30% en poids, de préférence entre 0,005% et 15% en poids, notamment entre 0,01% et 10% en poids, par rapport au poids total de la composition.

7. Composition selon l'une quelconque des revendications précédentes, comprend au moins un ingrédient additionnel choisi parmi l'eau, les alcools en C1-C7, les cétones, les silicones.

8. Composition selon l'une quelconque des revendications précédentes, se présentant sous la forme d'un produit de soin, de nettoyage et/ou de maquillage de la peau du corps ou du visage, des lèvres, des sourcils, des cils, des ongles et des cheveux, d'un produit solaire ou autobronzant, d'un produit d'hygiène corporelle, d'un produit capillaire, notamment de soin, de nettoyage, de conditionnement, de coloration des cheveux.

9. Composition selon l'une quelconque des revendications précédentes, se présentant sous la forme d'une composition capillaire, pour le soin et le traitement cosmétique, notamment la protection, des cheveux, en particulier des cheveux affaiblis et/ou abîmés, par exemple par des traitements chimiques ou mécaniques.

10. Procédé de traitement cosmétique des matières kératiniques, comprenant l'application sur lesdites matières d'une composition cosmétique comprenant, dans un milieu physiologiquement acceptable, un composé de formule (I) tel que défini à l'une des revendications 1 à 4 et éventuellement un composé de formule (II) tel que défini à l'une des revendications 5 et 6.

11. Procédé selon la revendication précédente, dans lequel la composition cosmétique utilisée est une composition selon l'une des revendications 1 à 9.

12. Procédé selon la revendication 10 ou 11, pour le conditionnement et/ou le soin des cheveux, en particulier pour leur apporter du corps et/ou de la nervosité, ou améliorer le démêlage, le lissage, la peignabilité, la réparation et la maniabilité de la chevelure.

13. Kit comprenant d'une part une composition de coloration, de décoloration, de permanente, de défrisage ou de lissage des cheveux et d'autre part une composition cosmétique comprenant, dans un milieu physiologiquement acceptable, un composé de formule (I) tel que défini à l'une des revendications 1 à 4 et éventuellement un composé de formule (II) tel que défini à l'une des revendications 5 et 6.

14. Composé de formule (la), ses sels, ses sels d'addition, ses solvates notamment hydrates, les formes tautomères étant inclues : 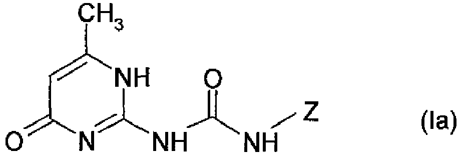 dans laquelle Z représente un radical monovalent choisi parmi :
(i) un radical hydrocarboné non cyclique saturé ou non, linéaire ou ramifié, en C1 à C32,
- éventuellement substituée par 1 à 20 radicaux hydroxyles -OH, et/ou
- éventuellement interrompu par 1 à 10 groupements identiques ou différents choisis parmi, seuls ou combinés entre eux, -S-, S(O), SO₂, -NH- (ou =NH), -N= (trivalent), -O-, -C(O)-,-CH(OH)-, -C(=NH)-, -N+(CH₃)₂-, radical aryle (cyclique aromatique), en C6-C10, notamment C6-C8.
- terminé par un groupement choisi parmi -NR₁R₂, -N+R₁R₂R₃ An-, -NHC(O)OR₄, -CH(NH₂)CO₂R₄, -NHC(O)CH(OH)(CH₂)₂OH, dans lesquels
R₁, R₃ représentent indépendamment un radical alkyle linéaire ou ramifié en C1-C6, un radical hydroxyalkyle ou un radical aminoalkyle;
R₂ représente un radical alkyle linéaire ou ramifié en C1-C6, ou un radical aminoalkyle;
R₄ représente un radical alkyle en C1-C6, linéaire ou ramifié, ou un radical hydroxyéthyle;
An- désigne un anion ou un mélange d'anions cosmétiquement acceptable.
(ii) un radical de formule (A1) : -(CH₂)ₚ-(X)ₙ-(CH₂)_{q}-NR₆-C(=NH)-NR₇R₈ dans laquelle X désigne un groupement choisi parmi -S-, S(O), SO₂, -NH- (ou =NH), -N= (trivalent), -O-, -C(O)-, -C(=NH)-, -C(O)O-,-OC(O)-,-NC(O) -, -NC(O)N-, NS02N-, -NR6-C(=NH)-NR7-, -N+(CH3)2- An-, un radical aryle (cyclique aromatique), en C6-C10, notamment C6-C8.
An- désigne un anion ou un mélange d'anions cosmétiquement acceptables.
n, p et q sont des nombres entiers et
n = 0 à 8 ;
p = 1 à 10 ;
q = 1 à 10,
p+q ≠ 5.
(iii) un radical de formule (A2) : -(CH₂)ᵣ(T)ₛ-(CH₂)ₜ-(CHR₉)ᵤ-(CH₂)ᵥ- Si R₁₀R₁₁R₁₂ dans lequel T représente un groupement choisi parmi les groupements, -S-, S(O), SO₂, -NH- (ou =NH), -N= (trivalent), -O-, -C(O)-, -C(=NH)-, -N+(CH₃)₂- An-; un radical aryle (cyclique aromatique) en C6-C10; une double liaison carbone-carbone notamment -CH=CH-, une triple liaison carbone-carbone, et leurs combinaisons; lorsque s, respectivement u, est non nul, les groupements T adjacents, respectivement -CHR₉-adjacents, sont identiques ou différents;
R₉ désigne un radical choisi parmi les radicaux -OH, -SH, -SO₃H, -COOH, -COOR, -NHC(O)R, NHC(O)OR, -NRR' et -N⁺RR'R" An-, dans lesquels R, R' et R", identiques ou différents représentent un atome d'hydrogène H ou un radical alkyle linéaire ou ramifié en C1-C6;
R₁₀, R₁₁, R₁₂ sont choisis indépendamment parmi les radicaux OR₁₃, alkyle linéaire ou ramifié en C1-C6 ;
R₁₃ désigne un radical alkyle linéaire ou ramifié en C1-C6, ou un radical -(CH₂-CH₂-O)_{w}R₁₄ ou un radical -SiR₁₅R₁₆R₁₇.
R₁₄ désigne un atome d'hydrogène H, ou un radical méthyle.
R₁₅, R₁₆, et R₁₇ désignent indépendamment un radical alkyle linéaire ou ramifié en C1-C4.
An- désigne un anion ou un mélange d'anions cosmétiquement acceptables.
r, s, t, u, v, w sont des nombres entiers et
r = 1 à 10 ;
s, t, u, v = 0 à 10 ;
w = 1 à 5 ;
étant entendu que lorsque s=u=0 et R₁₀=R₁₁=R₁₂= OR₁₃, alors r+t+v ≠ 3.
(iv) un radical de formule (A3) : -(CH₂)ₓ-(U)_{y}-(CH₂)_{z}-CH₃
dans laquelle U désigne un groupement choisi parmi les radicaux, -S-, S(O), SO₂, -NH- (ou =NH), -N= (trivalent), -O-, -C(O)-, -C(=NH)-, -N+(CH₃)₂- An-, un radical aryle (cyclique aromatique), en C6-C10, notamment C6-C8 , une double liaison carbone -carbone notamment -CH=CH-, une triple liaison carbone -carbone , ainsi que leurs combinaisons;
An- désigne un anion ou un mélange d'anions cosmétiquement acceptables.
x, y et z sont des nombres entiers, compris entre 0 et 10 inclus,
étant entendu que lorsque y = 0, x+z est supérieur ou égal à 10 et inférieur ou égal à 30 et différent de 18, notamment x+z est égal à 15 ;
et que lorsque y ≠ 0, x est supérieur ou égal à 2 et inférieur ou égal à 25 et z est supérieur ou égal à 3 et inférieur ou égal à 25, notamment x+z est égal à 15 avec U égal à C=C et y =1 ;
(v) un radical de formule (A4) : -Alk1-(V)ₐ₁-(Alk2)ₐ₂-CH₂-W
dans laquelle Alk1 et Alk2 désignent indépendamment un radical alkyle saturé ou non, linéaire ou ramifié, en C1 à C32;
V désigne un groupement choisi parmi les radicaux, -S-, S(O), SO₂, -NH- (ou =NH), -N= (trivalent), -O-, -C(O)-, -C(=NH)-, -N+(CH₃)₂- An-, radical aryle (cyclique aromatique), en C6-C10, notamment C6-C8 , double liaison carbone-carbone notamment -CH=CH-, triple liaison carbone-carbone, ou leurs combinaisons.
An- désigne un anion ou un mélange d'anions cosmétiquement acceptables.
W désigne un radical choisi parmi les radicaux -SR₁₅, -S(O)R₁₅, -SO₂R₁₅, -SO₃H,-OSO₃H.
R₁₅ désigne un (hétéro)cycle aromatique en C5-C10, dont 3 atomes de carbone peuvent être remplacés par des atomes d'azote; un radical carbonyle -C(O)R₁₆ ; un radical -C(=NR₁₇)-NR₁₈R₁₉
R₁₆ désigne un radical alkyle linéaire ou ramifié en C1-C6;
R₁₇, R₁₈, R₁₉ désignent indépendamment un atome d'hydrogène H, un radical alkyle linéaire ou ramifié en C1-C6,
R₁₇ et R₁₈ peuvent être reliés entre eux pour former avec les atomes d'azote auxquels ils sont rattachés un cycle ou un bicycle de 5 à 8 atomes, saturé ou non ;
a1 et a2 sont des nombres entiers
a1 = 0 à 8, de préférence 0 à 2.
a2 = 0 ou 1.
(vi) un radical de formule (A5) : -Y-(K)j-G
dans laquelle Y désigne un radical alkyle saturé ou non, linéaire ou ramifié, en C1 à C32, éventuellement interrompu par 1 à 8 groupements identiques ou non choisis parmi -S-, S(O), SO₂, -NH- (ou =NH), -N= (trivalent), -O-, -C(O)-, -C(=NH)-,-N+(CH₃)₂- An-, un radical aryle (cyclique aromatique) en C6-C10, ou leur combinaison.
K désigne un groupement choisi parmi -CO-, -NHC(O)- -CH(NRR')-, -CHR-,-C(=CHR)-;
G désigne un groupement choisi parmi G1, G2 ou G3, avec :
- G1 désigne un monosaccharide ou un polysaccharide jusqu'à 20 unités sucre, sous forme pyranose et/ou furanose et de séries L et/ou D, ledit mono- ou polysaccharide présentant éventuellement une ou plusieurs fonctions aminés, les fonctions hydroxyles et amines étant éventuellement protégées ; et leurs isomères optiques et/ou géométriques; ces monosaccharides pouvant être protégés ou non sur les fonctions hydroxyles non engagées dans la liaison avec le radical H;
- G2 désigne un radical acide aminé alpha avec une chaine latérale, protégée ou non, choisie parmi les chaines latérales des 22 acides aminés naturels, C ou N branché, racémique ou non, de stéréochimie L ou D, protégé ou non sur le résidu C ou N et sur la chaine latérale pour les acides aminés alpha concernés.
- G3 désigne un radical phényle substitué sur l'une quelconque des positions par un radical hydroxyle et éventuellement substitué par un groupement R_{20,} R₂₀ désigne un radical alkyle saturé ou non, linéaire ou ramifié, en C1 à C6, hydroxyalkyle en C1 à C6, un radical aryle (cyclique aromatique) en C5-C10, un radical -OH, -SH, -SO₃H, -COOH, -COOR, -NHC(O)R, NHC(O)OR, -NRR' et-N⁺RR'R" An-,
R, R' et R" désignent indépendamment un atome d'hydrogène H ou un radical alkyle en C1-C6;
An- désigne un anion ou un mélange d'anions cosmétiquement acceptables.
j est un nombre entier égal à 0 ou 1;
(vii) un radical de formule (A6) : -(CH₂)_{g}-(J)ₕ-(CH₂)ᵢ-(CFiR₂₃)ₖ-CH₂R₂₄
dans laquelle J désigne un groupement choisi parmi les groupements -S-, S(O), SO2, -NH- (ou =NH), -N= (trivalent), -O-, -C(O)-, -C(=NH)-, -N+(CH3)2- An-,un radical aryle (cyclique aromatique), en C6-C10, notamment C6-C8 , une double liaison carbone -carbone notamment -CH=CH-, une triple liaison carbone-carbone, ou leurs combinaisons.
R₂₃ désigne un radical -OR₂₅, -SH, -SO₃H, -COOH, -COOR, -NHC(O)R, NHC(O)OR, -NRR' et -N⁺RR'R" An-, avec R, R' et R" = H ou alkyle en C1-C6, notamment méthyle;
R₂₄ désigne un atome d'hydrogène H ou un radical hydroxyle -OH;
R₂₅ désigne un atome d'hydrogène H, ou un monosaccharide ou un polysaccharide jusqu'à 20 unités sucre, sous forme pyranose et/ou furanose et de séries L et/ou D, ledit mono- ou polysaccharide présentant éventuellement une ou plusieurs fonctions aminés, les fonctions hydroxyles et amines étant éventuellement protégées ; et leurs isomères optiques et/ou géométriques.
An- désigne un anion ou un mélange d'anions cosmétiquement acceptables.
g, h, i, k désignent un nombre entier
g, h k = 1 à 10
i = 0 à 10.
(viii) un radical de formule (A7) : -(CH₂)_{b}-(A)-(B)-(A)_{c}-(CH₂)_{d}-D
dans lequel A désigne un groupement choisi parmi -NHC(O)-, -C(O)NH-,-NHC(O)NH-, -C(O)O-, -OC(O)-;
B désigne une chaine hydrocarbonée, ramifiée ou non, éventuellement substituée par un radical choisi parmi -OH, -COOH, -NHC(O)R₂₁ ;
R₂₁ désigne un radical alkyle linéaire ou ramifié, en C1 à C6;
D désigne un radical -SH ou un radical alkyle linéaire ou ramifié, en C1 à C4.
b, c et d sont des nombres entiers
b = 2 à 10, de préférence 3 à 6 ;
c = 0 ou 1 ;
d = 0 à 10, de préférence 0 à 3 ;
(ix) un radical de formule (A8) : -Alk3-S-S-Alk4
dans lequel Alk4 désigne un radical hydrocarboné linéaire ou ramifié, en C2 à C20, comprenant éventuellement une ou plusieurs doubles liaisons, et comprenant éventuellement un ou plusieurs hétéroatomes choisis parmi O ou N, et éventuellement substituée par un ou plusieurs radicaux identiques ou différents choisis parmi -OH, -SO₃H, -COOH, -COOR, -NHC(O)R, NHC(O)OR, -NRR' et -N⁺RR'R" An-.
Alk3 désigne un radical hydrocarboné linéaire ou ramifié, en C2 à C20, comprenant éventuellement une ou plusieurs doubles liaisons, et comprenant éventuellement un ou plusieurs hétéroatomes choisis parmi O ou N, et éventuellement substituée par un ou plusieurs radicaux identiques ou différents choisis parmi -OH, -SO₃H, -COOH, -COOR, -NHC(O)R, -NRR' et -N⁺RR'R" An-.
R, R' et R" désignent indépendamment un atome d'hydrogène H ou un radical alkyle linéaire ou ramifié en C1-C6;
An- désigne un anion ou un mélange d'anions cosmétiquement acceptables.
(x) un radical de formule (A9) : -CHL-COOR₂₂
dans lequel L désigne la chaine latérale protégée ou non d'un acide alpha aminé, choisie parmi les chaines latérales des 22 acides aminés naturels ; la stéréochimie est L, D ou un mélange racémique;
R₂₂ désigne un atome d'hydrogène H, ou un radical alkyle linéaire ou ramifié en C1 à C6, ou un radical benzyle.
(xi) un radical -(CH₂)₂-SH;
(xii) un radical -(CH₂)₄-NH₂.
Etant entendu que le composé (Ia) ne peut représenter les composés suivants :

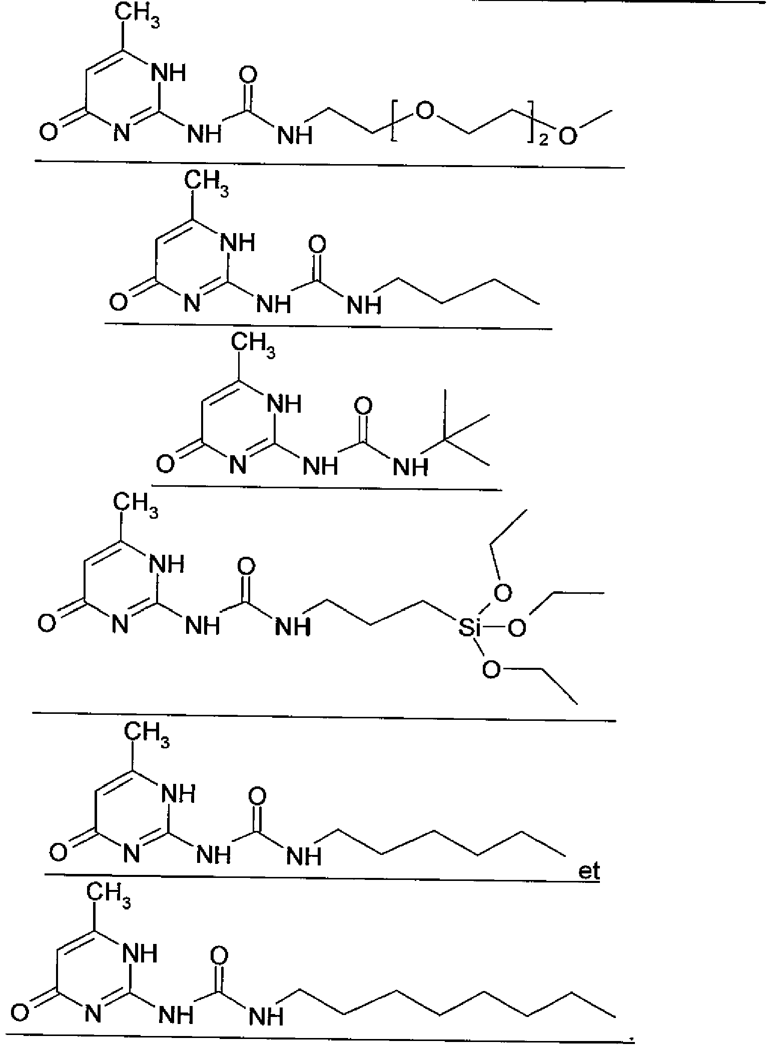

## Patentansprüche

1. Kosmetische Zusammensetzung, umfassend in einem physiologisch verträglichen Medium mindestens eine Verbindung der Formel (I), ihre Salze, ihre Additionssalze, ihre Solvate, insbesondere Hydrate, wobei die tautomeren Formen enthalten sind: 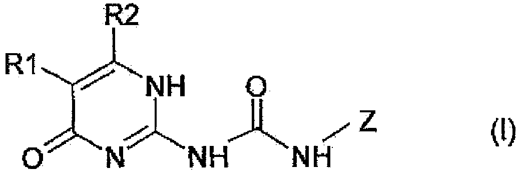 wobei:
- R1 und R2 unabhängig voneinander für H, -OH, -NRR' stehen (wobei R, R', gleich oder verschieden, H oder ein linearer oder verzweigter C1-C12-Alkylrest sind); oder eine Kohlenstoffgruppe, insbesondere ein gesättigtes oder nicht gesättigtes, gegebenenfalls aromatisches, lineares, verzweigtes und/oder zyklisches C1-C18-Alkyl, das ein oder mehrere Heteroatome O, N und/oder S, insbesondere OH, COOH und/oder NRR' enthalten kann;
- Z für einen einwertigen Rest steht, ausgewählt aus:
(i) einem Wasserstoffatom,
(ii) einem (gesättigten) linearen oder verzweigten C1-bis C32-Alkylrest;
(iii) einem linearen oder verzweigten (ungesättigten) C2- bis C32-Alkenrest, umfassend eine oder zwei C=C-Doppelbindungen;
(iv) einem C1-C32-, insbesondere C2-C24- oder sogar C3-C18-, bevorzugter C4-C12-Alkylrest, substituiert mit einem C6-C10-, insbesondere C6-C8-Aryl; insbesondere Phenyl;
wobei die Reste gegebenenfalls mit 1 bis 8 Gruppen, ausgewählt aus -OH, -OR, -SH, -SR, -SO₃H, -SO₃R, -SO₂NRR', -COOH, -COOR, -CONRR', -NR-C(O)-NRR', -NRR' und -N⁺RR'R" substituiert sein können, wobei R, R' und R" = H oder C1-C6-Alkyl sind; und die Substituenten der nachfolgenden Formeln (a) bis (h): 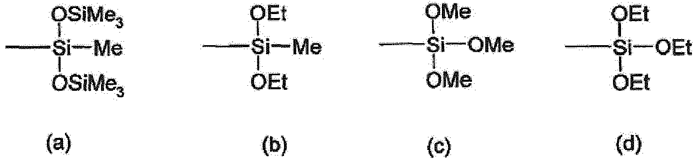 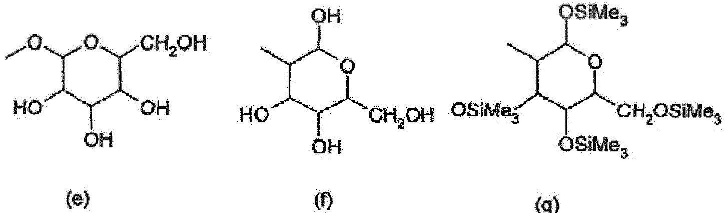 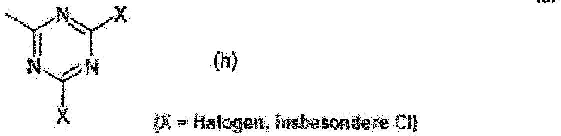
und/oder die Reste gegebenenfalls 1 bis 8 Gruppen umfassen können, allein oder im Gemisch, ausgewählt aus -S-, S(O), SO₂, -NH- (oder =NH), -N= (dreiwertig), -O-, -C(O)-, -C(=NH)-, -N⁺(CH₃)₂-An⁻ (An⁻: Anion),
wobei das kosmetisch verträgliche Medium mindestens einen Bestandteil umfasst, ausgewählt aus Treibmitteln, kohlenstoffhaltigen Ölen; C8-C40-Alkoholen, C8-C4Q-Estern, C8-C40-Säuren; nichtionischen Tensiden, kationischen Tensiden, anionischen Tensiden, amphoteren Tensiden, zwitterionischen Tensiden; UV-Filtern; Antischuppenmitteln; Antioxidantien; Reduktionsmitteln; Oxidationsbasen, Kopplungsmitteln, Oxidationsmitteln, Direktfarbstoffen; Haarglättungsmitteln, Perlglanzmitteln und Trübungsmitteln; Weichmachern oder Koaleszenzmitteln; Hydroxysäuren; Pigmenten; Füllstoffen; Polyolen; Wachsen; Verdickungsmitteln, Emulgatoren; Polymeren.

2. Zusammensetzung nach Anspruch 1, wobei R1 für H steht und R2 für H, CH₃, C₇H₁₅, C₁₃H₂₇ oder Aryl steht.

3. Zusammensetzung nach einem der vorhergehenden Ansprüche, wobei die Verbindungen der Formel (I) ausgewählt sind aus den folgenden Verbindungen:

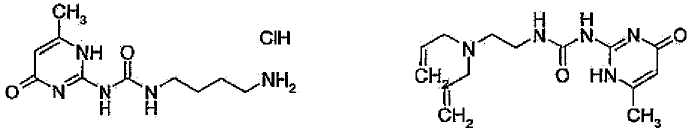

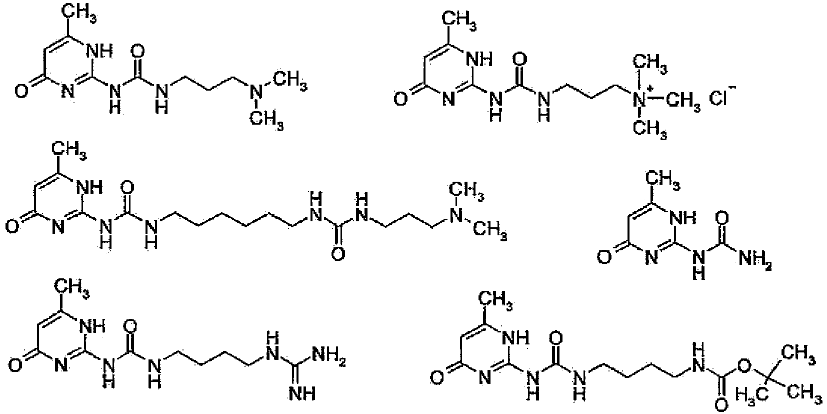

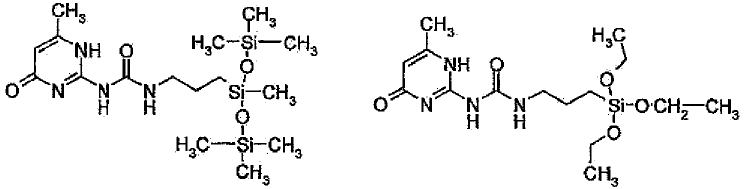

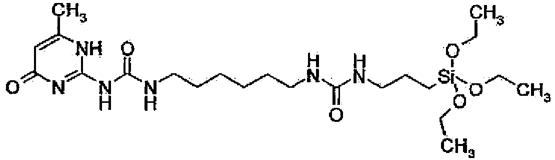

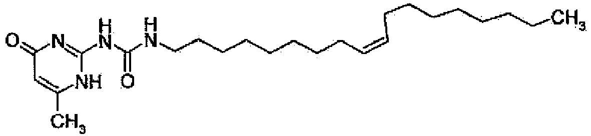

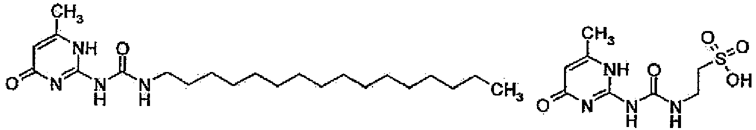

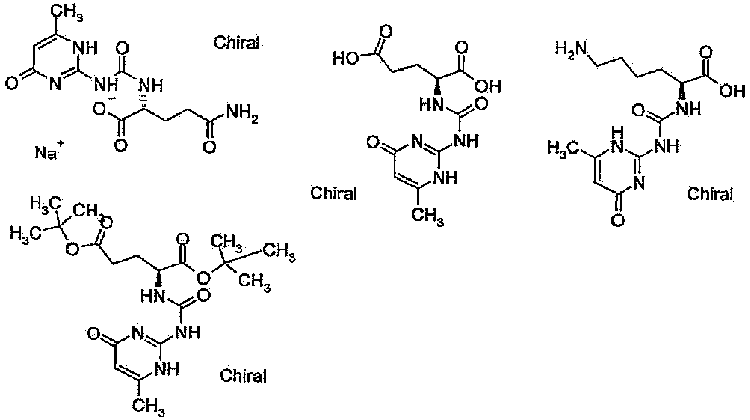

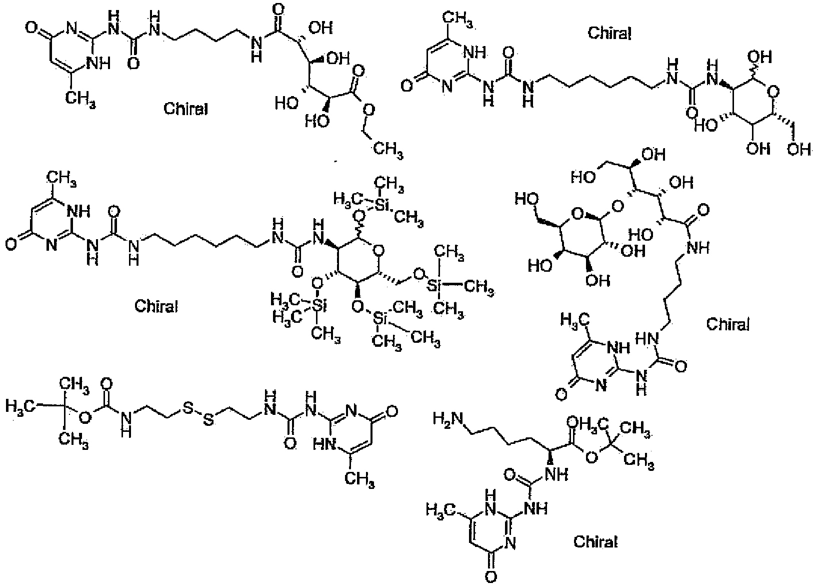

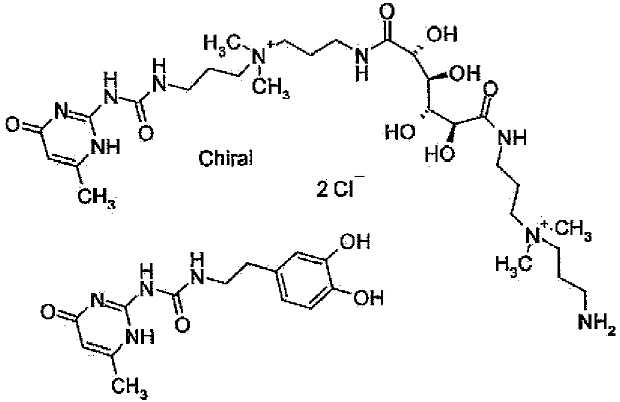

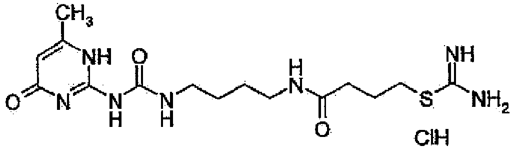

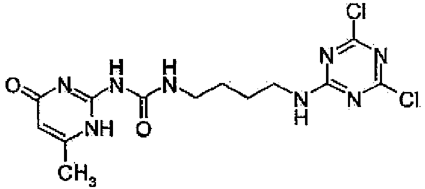

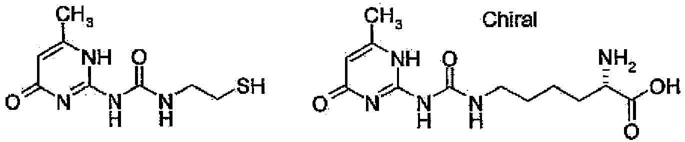

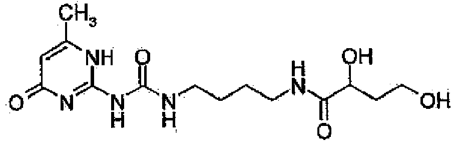

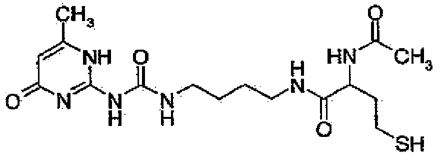

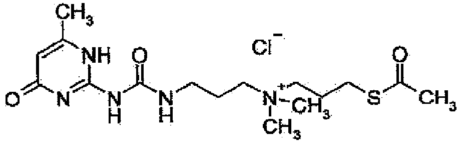

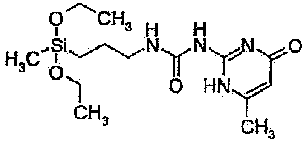

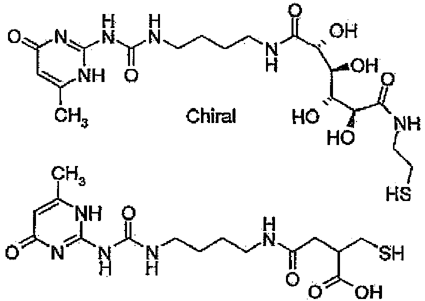

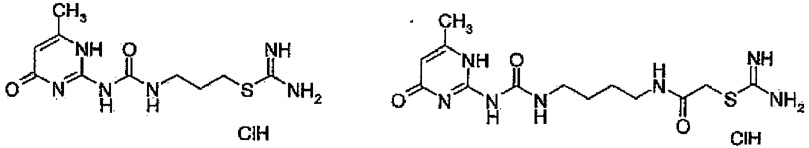

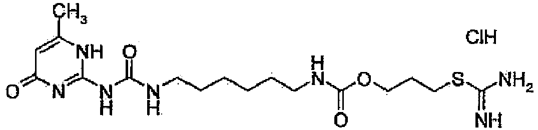

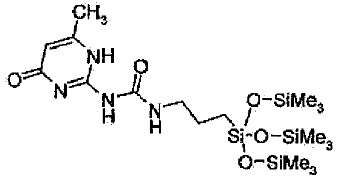

4. Zusammensetzung nach einem der vorhergehenden Ansprüche, wobei die Verbindung der Formel (I), allein oder als Gemisch, in einer Menge im Bereich zwischen 0,001 Gew.-% und 30 Gew.-%, vorzugsweise zwischen 0,005 Gew.-% und 15 Gew.-%, insbesondere zwischen 0,01 Gew.-% und 10 Gew.-% oder sogar zwischen 0,1 und 5 Gew.-% vorhanden ist, bezogen auf das Gesamtgewicht der Zusammensetzung.

5. Zusammensetzung nach einem der vorhergehenden Ansprüche, ferner umfassend mindestens eine zusätzliche Verbindung der Formel (II): 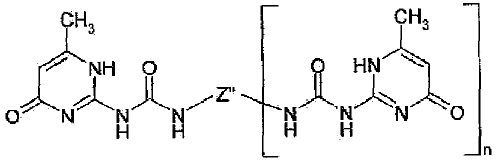 wobei:
- n = 1 oder 2 ist und
- Z für einen (je nach dem Wert von n) zweiwertigen oder dreiwertigen Rest, ein lineares oder verzweigtes C1-C32-Alkyl oder ein C2-C32-Alkerryl steht; gegebenenfalls substituiert mit 1 bis 10 Resten, ausgewählt aus -OH, -SO₃H, -COOH, -COOR und -N⁺RR'R", wobei R, R' und R" = C1-C12-Alkyl sind, und/oder gegebenenfalls unterbrochen durch 1 bis 10 Gruppen, ausgewählt aus (i) den zweiwertigen Gruppen: -S-, -NH-(oder =NH), -O-, -C(O)-, oder der Formel: 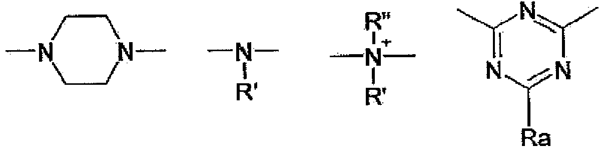
wobei Ra = H oder Halogen oder C1-C6-Alkyl oder eine Einfachbindung ist, und
R' und R", gleich oder verschieden, für ein Wasserstoffatom oder einen C1-C6-Alkylrest stehen; und
- (ii) den dreiwertigen Gruppen der Formel: 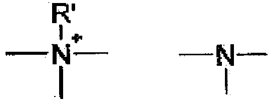 wobei R' für ein Wasserstoffatom oder einen C1-C6-Alkylrest steht.

6. Zusammensetzung nach Anspruch 5, wobei die Verbindung der Formel (II), allein öder als Gemisch, in einer Menge zwischen 0,001 Gew.-% und 30 Gew.-%, vorzugsweise zwischen 0,005 Gew.-% und 15 Gew.-%, insbesondere zwischen 0,01 Gew.-% und 10 Gew.-% vorhanden ist, bezogen auf das Gesamtgewicht der Zusammensetzung.

7. Zusammensetzung nach einem der vorhergehenden Ansprüche, umfassend mindestens einen zusätzlichen Bestandteil, ausgewählt aus Wasser, C1-C7-Alkoholen, Ketonen, Silikonen.

8. Zusammensetzung nach einem der vorhergehenden Ansprüche, in Form eines Produkts zur Pflege, Reinigung und/oder zum Schminken der Haut des Körpers oder des Gesichts, der Lippen, der Augenbrauen, der Wimpern, der Nägel und der Haare, eines Produkts zur Bräunung oder Selbstbräunung, eines Produkts zu Körperpflege, Haarpflege, insbesondere zur Pflege, Reinigung, Konditionierung, Färbung der Haare.

9. Zusammensetzung nach einem der vorhergehenden Ansprüche, in Form einer Haarzusammensetzung zur Pflege und zur kosmetischen Behandlung, insbesondere zum Schutz der Haare, insbesondere der beispielsweise durch chemische oder mechanische Behandlungen geschwächten und/oder geschädigten Haare.

10. Verfahren zur kosmetischen Behandlung von Keratinmaterialien, umfassend das Aufbringen auf diese Materialien einer kosmetischen Zusammensetzung, umfassend, in einem physiologisch verträglichen Medium, eine Verbindung der Formel (I) nach einem der Ansprüche 1 bis 4 und gegebenenfalls eine Verbindung der Formel (II) nach einem der Ansprüche 5 und 6.

11. Verfahren nach dem vorhergehenden Anspruch, wobei die verwendete kosmetische Zusammensetzung eine Zusammensetzung nach einem der Ansprüche 1 bis 9 ist.

12. Verfahren nach Anspruch 10 oder 11 zum Konditionieren und/oder Pflegen der Haare, insbesondere um ihnen Fülle und/oder Lebendigkeit zu verleihen, oder um die Entwirrbarkeit, Glättung, das Kämmen, die Reparatur und die Geschmeidigkeit der Haare zu verbessern.

13. Kit, umfassend einerseits eine Zusammensetzung zum Färben, Bleichen, Dauerwellen, Geschmeidigmachen oder Glätten der Haare und andererseits eine kosmetische Zusammensetzung, umfassend, in einem physiologisch verträglichen Medium, eine Verbindung der Formel (I) nach einem der Ansprüche 1 bis 4 und gegebenenfalls eine Verbindung der Formel (II) nach einem der Ansprüche 5 und 6.

14. Verbindung der Formel (Ia), ihre Salze, ihre Additionssalze, ihre Solvate, insbesondere Hydrate, wobei die tautomeren Formen enthalten sind: 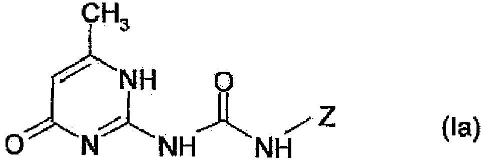 wobei Z für einen einwertigen Rest steht, ausgewählt aus:
(i) einem gesättigten oder ungesättigten, linearen oder verzweigten, nicht zyklischen C1- bis C32-Kohlenwasserstoffrest,
- gegebenenfalls substituiert mit 1 bis 20 Hydroxylresten -OH, und/oder
- gegebenenfalls unterbrochen durch 1 bis 10 Gruppen, gleich oder verschieden, ausgewählt aus, allein oder miteinander kombiniert, -S-, S(O), SO₂, -NH-(oder =NH), -N= (dreiwertig), -O-, -C(O)-, -CH(OH)-, -C(=NH)-, -N+(CH₃)₂-, C6-C10-, insbesondere C6-C8-Arylrest (zyklisch aromatisch),
- terminiert von einer Gruppe, ausgewählt aus -NR₁R₂, -N⁺R₁R₂R₃ An-, -NHC(O)OR₄, -CH(NH₂)CO₂R₄, -NHC(O)CH(OH)(CH₂)₂OH, wobei R₁, R₃ unabhängig für einen linearen oder verzweigten C1-C6-Alkylrest, einen Hydroxyalkylrest oder einen Aminoalkylrest stehen;
R₂ für einen linearen oder verzweigten C1-C6-Alkylrest oder einen Aminoalkylrest steht;
R₄ für einen linearen oder verzweigten C1-C6-Alkylrest oder einen Hydroxyethylrest steht;
An- ein Anion oder ein kosmetisch verträgliches Anionengemisch bezeichnet;
(ii) einem Rest der Formel (A1): -(CH₂)ₚ-(X)ₙ-(CH₂)_{q}-NR₆-C(=NH)-NR₇R₈,
wobei X eine Gruppe bezeichnet, ausgewählt aus -S-, S(O), SO₂, -NH- (oder = NH), -N= (dreiwertig), -O-, -C(O)-, -C(=NH)-, -C(O)O-, -OC(O)-, -NC(O)-, -NC(O)N-, NSO2N-, -NR6-C(=NH)-NR7-, -N+(CH3)2- An-, einem C6-C10-, insbesondere C6-C8-Arylrest (zyklisch aromatisch),
An- ein Anion oder ein Gemisch aus kosmetisch verträglichen Anionen bezeichnet;
n, p und q ganze Zahlen sind, und
n = 0 bis 8 ist;
p = 1 bis 10 ist;
q = 1 bis 10 ist;
p + q ≠ 5 ist;
(iii) einem Rest der Formel (A2): -(CH₂)ᵣ-{T)ₛ-(CH₂)ₜ-(CHR₉)ᵤ-(CH₂)ᵥ- Si R₁₀R₁₁R₁₂, wobei T für eine Gruppe steht, ausgewählt aus den Gruppen -S-, S(O), SO₂, -NH-(oder =NH), -N= (dreiwertig), -O-, -C(O)-, -C(=NH)-, -N+(CH₃)₂- An-; einen C6-C10-Arylrest (zyklisch aromatisch); eine Kohlenstoff-Kohlenstoff-Doppelbindung, insbesondere -CH=CH-, eine Kohlenstoff-Kohlenstoff-Dreifachbindung und Kombinationen davon; wenn s beziehungsweise u nicht null ist, sind die T-benachbarten, beziehungsweise -CHR₉-benachbarten Gruppen gleich oder verschieden;
R₉ einen Rest bezeichnet, ausgewählt aus den Resten -OH, -SH, -SO₃H, -COOH, -COOR, -NHC(O)R, NHC(O)OR, -NRR' und -N⁺RR'R" An-, wobei R, R' und R", gleich oder verschieden, für ein Wasserstoffatom H oder einen linearen oder verzweigten C1-C6 Alkylrest stehen;
R₁₀, R₁₁, R₁₂ unabhängig ausgewählt sind aus den Resten OR₁₃, linearem oder verzweigtem C1-C6-Alkyl;
R₁₃ einen linearen oder verzweigten C1-C6-Alkylrest oder einen Rest -(CH₂-CH₂-O)_{w}R₁₄ oder einen Rest -SiR₁₅R₁₅R₁₇ bezeichnet;
R₁₄ ein Wasserstoffatom H oder einen Methylrest bezeichnet;
R₁₅, R₁₆ und R₁₇ unabhängig einen linearen oder verzweigten C1-C4-Alkylrest bezeichnen;
An- ein Anion oder ein Gemisch aus kosmetisch verträglichen Anionen bezeichnet;
r, s, t, u, v, w ganze Zahlen sind und
r = 1 bis 10 ist;
s, t, u, v = 0 bis 10 sind;
w = 1 bis 5 ist;
unter der Maßgabe, dass wenn s=u=0 ist und R₁₀=R₁₁=R₁₂=OR₁₃ ist, dann r+t+v ≠ 3 gilt;
(iv) einem Rest der Formel (A3): - (CH₂)ₓ-(U)_{y}-(CH₂)_{z}-CH₃ wobei U eine Gruppe bezeichnet, ausgewählt aus den Resten -S-, S(O), SO2, -NH- (oder =NH), -N= (dreiwertig), -O-, -C(O)-, -C(=NH)-, -N+(CH₃)₂- An-, einem C6-C10-, insbesondere C6-C8-Arylrest (zyklisch aromatisch), einer Kohlenstoff-Kohlenstoff-Doppelbindung, insbesondere -CH=CH-, einer Kohlenstoff-Kohlenstoff-Dreifachbindung, sowie Kombinationen davon;
An- ein Anion oder ein Gemisch aus kosmetisch verträglichen Anionen bezeichnet;
x, y und z ganze Zahlen im Bereich zwischen einschließlich 0 und 10 sind;
unter der Maßgabe, dass wenn y = 0 ist, dann x+z größer oder gleich 10 und kleiner oder gleich 30 und verschieden von 18 ist, insbesondere x+z gleich 15 ist; und wenn y ≠ 0, x größer oder gleich 2 und kleiner oder gleich 25 ist und z größer oder gleich 3 und kleiner oder gleich 25 ist, insbesondere x+z gleich 15 ist, wobei U gleich C=C und y =1 ist;
(v) einem Rest der Formel (A4): -Alk1-(V)ₐ₁-(Alk2)ₐ₂-CH₂-W,
wobei Alkl und Alk2 unabhängig einen gesättigten oder ungesättigten, linearen oder verzweigten C1- bis C32-Alkylrest bezeichnen;
V eine Gruppe bezeichnet, ausgewählt aus den Resten -S-, S(O), SO₂, -NH- (oder =NH), -N= (dreiwertig), -O-, -C(O)-, -C(=NH)-, -N+(CH₃)₂- An-, C6-C10-, insbesondere einem C6-C8-Arylrest (zyklisch aromatisch), einer Kohlenstoff-Kohlenstoff-Doppelbindung, insbesondere einer -CH=CH-, Köhlenstoff-Kohlenstoff-Dreifachbindung oder Kombinationen davon;
An- ein Anion oder ein Gemisch aus kosmetisch verträglichen Anionen bezeichnet;
W einen Rest bezeichnet, ausgewählt aus den Resten -SR₁₅ , -S(O)R₁₅, -SO₂R₁₅, -SO₃H, -OSO₃H;
R₁₅ einen aromatischen C5-C10-Heterozyklus bezeichnet, dessen 3 Kohlenstoffatome durch Stickstoffatome; einen Carbonylrest -C(O)R₁₆; einen Rest -C(=NR₁₇)-NR₁₈R₁₉ ersetzt werden können;
R₁₆ einen linearen oder verzweigten C1-C6-Alkylrest bezeichnet;
R₁₇, R₁₈, R₁₉ unabhängig ein Wasserstoffatom H, einen linearen oder verzweigten C1-C6-Alkylrest bezeichnen; R₁₇ und R₁₈ miteinander verbunden sein können, um mit den Stickstoffatomen, an die sie gebunden sind, einen Zyklus oder einen Bizyklus mit 5 bis 8 gesättigten oder ungesättigten Atomen zu bilden;
a1 und a2 ganze Zahlen sind;
a1 = 0 bis 8, vorzugsweise 0 bis 2 ist;
a2 = 0 oder 1 ist;
(vi) einem Rest der Formel (A5): -Y-(K)j-G,
wobei Y einen gesättigten oder ungesättigten, linearen oder verzweigten C1- bis C32-Alkylrest bezeichnet, gegebenenfalls unterbrochen durch 1 bis 8 Gruppen, gleich oder nicht, ausgewählt aus -S-, S(O), SO₂, -NH-(oder =NH), -N= (dreiwertig), -O-, -C(O)-, -C(=NH)-, -N+(CH₃)₂- An-, einen C6-C10-Arylrest (zyklisch aromatisch) oder eine Kombination davon;
K eine Gruppe bezeichnet, ausgewählt aus -CO-, -NHC(O)--CH(NRR')-, -CHR-, -C(=CHR)-;
G eine Gruppe bezeichnet, ausgewählt aus G1, G2 oder G3, wobei:
- G1 ein Monosaccharid oder ein Polysaccharid mit bis zu 20 Zuckereinheiten in Pyranose- und/oder Furanoseform und der Reihe L und/oder D bezeichnet, wobei das Mono- oder Polysaccharid gegebenenfalls eine oder mehrere Aminfunktionen aufweist, wobei die Hydroxyl- und Aminfunktionen gegebenenfalls geschützt sind; und optische und/oder geometrische Isomere davon; wobei diese Monosaccharide an ihren Hydroxylfunkionen, die nicht an der Bindung mit dem Rest H beteiligt sind, geschützt sein können oder nicht;
- G2 einen alpha-Aminosäurerest mit einer geschützten oder ungeschützten Seitenkette bezeichnet, ausgewählt aus den Seitenketten der 22 natürlichen Aminosäuren, C oder N, verzweigt, racemisch oder nicht, mit der Stereochemie L oder D, geschützt oder nicht an dem C- oder N-Rest und an der Seitenkette für die betreffenden alpha-Aminosäuren;
- G3 einen Phenylrest bezeichnet, substituiert an einer beliebigen Position mit einem Hydroxylrest und gegebenenfalls substituiert mit einer Gruppe R₂₀;
R₂₀ einen gesättigten oder ungesättigten, linearen oder verzweigten C1- bis C6-Alkylrest, einen C1- bis C6-Hydroxyalkylrest, einen C5-C10-Arylrest (zyklisch aromatisch), einen Rest -OH, -SH, -SO₃H, -COOH, -COOR, -NHC(O)R, NHC(O)OR, -NRR' und -N⁺RR'R" An- bezeichnet;
R, R' und R" unabhängig ein Wasserstoffatom H oder einen C1-C6-Alkylrest bezeichnen;
An- ein Anion oder ein Gemisch von kosmetisch verträglichen Anionen bezeichnet;
j eine ganze Zahl gleich 0 oder 1 ist;
(vii) einem Rest der Formel (A6): -(CH₂)_{g}-(J)ₕ-(CH₂)ᵢ-(CHR₂₃)ₖ-CH₂R₂₄,
wobei J eine Gruppe bezeichnet, ausgewählt aus den Gruppen -S-, S(O), SO2, -NH- (oder =NH), -N= (dreiwertig), -O-, -C(O)-, -C(=NH)-, -N+(CH₃)₂- An-, einem C6-C10-, insbesondere C6-C8-Arylrest (zyklisch aromatisch), einer Kohlenstoff-Kohlenstoff-Doppelbindung, insbesondere -CH=CH-, einer Kohlenstoff-Kohlenstoff-Dreifachbindung oder Kombinationen davon; R₂₃ einen Rest -OR₂₅, -SH, -SO₃H, -COOH, -COOR, -NHC(O)R, NHC(O)OR, -NRR' und -N⁺RR'R" An- bezeichnet, wobei R, R' und R" = H oder C1-C6-Alkyl, insbesondere Methyl sind; R₂₄ ein Wasserstoffatom H oder einen Hydroxylrest -OH bezeichnet;
R₂₅ ein Wasserstoffatom H oder ein Monosaccharid oder ein Polysaccharid mit bis zu 20 Zuckereinheiten in Pyranose- und/oder Furanoseform und der Reihen L und/oder D bezeichnet, wobei das Mono- oder Polysaccharid gegebenenfalls eine oder mehrere Aminfunktionen aufweist, wobei die Hydroxyl- und Aminfunktionen gegebenenfalls geschützt sind; und deren optische und/oder geometrische Isomere;
An- ein Anion oder ein Gemisch aus kosmetisch verträglichen Anionen bezeichnet;
g, h, i, k eine ganze Zahl bezeichnen;
g, h, k = 1 bis 10 sind;
i = 0 bis 10 ist;
(viii) einem Rest der Formel (A7): -(CH₂)_{b}-(A)-(B)-(A)_{c}-(CH₂)_{d}-D,
wobei A eine Gruppe bezeichnet, ausgewählt aus -NHC(O)-, -C(O)NH-, -NHC(O)NH-, -C(O)O-, -C)C(O)-;
B eine verzweigte oder unverzweigte Kohlenwasserstoffkette bezeichnet, gegebenenfalls substituiert mit einem Rest, ausgewählt aus -OH, -COOH, -NHC(O)R₂₁;
R₂₁ einen linearen oder verzweigten C1- bis C6-Alkylrest bezeichnet;
D einen Rest -SH oder einen linearen oder verzweigten C1- bis C4-Alkylrest bezeichnet;
b, c und d ganze Zahlen sind;
b = 2 bis 10, vorzugsweise 3 bis 6 ist;
c = 0 oder 1 ist;
d = 0 bis 10, vorzugsweise 0 bis 3 ist;
(ix) einem Rest der Formel (A8): -Alk3-S-S-Alk4,
wobei Alk4 einen linearen oder verzweigten C2- bis C20-Kohlenwasserstoffrest bezeichnet, umfassend gegebenenfalls eine oder mehrere Doppelbindungen, und umfassend gegebenenfalls ein oder mehrere Heteroatome, ausgewählt aus O oder N, und gegebenenfalls substituiert mit einem oder mehreren Resten, gleich oder verschieden, ausgewählt aus -OH, -SO₃H, -COOH, -COOR, -NHC(O)R, NHC(O)OR, -NRR' und -N⁺RR'R" An-;
Alk3 einen linearen oder verzweigten C2- bis C20-Kohlenwasserstoffrest bezeichnet, umfassend gegebenenfalls eine oder mehrere Doppelbindungen, und umfassend gegebenenfalls ein oder mehrere Heteroatome, ausgewählt aus O oder N, und gegebenenfalls substituiert mit einem oder mehreren Resten, gleich oder verschieden, ausgewählt aus -OH, -SO₃H, -COOH, -COOR, -NHC(O)R, -NRR' und -N⁺RR'R" An-;
R, R' und R" unabhängig ein Wasserstoffatom H oder einen linearen oder verzweigten C1-C6-Alkylrest bezeichnen;
An- ein Anion oder ein Gemisch aus kosmetisch verträglichen Anionen bezeichnet;
(x) einem Rest der Formel (A9): -CHL-COOR₂₂,
wobei L die geschützte oder ungeschützte Seitenkette einer alpha-Aminosäure bezeichnet, ausgewählt aus den Seitenketten der 22 natürlichen Aminosäuren; wobei die Stereochemie L, D oder ein racemisches Gemisch ist;
R₂₂ ein Wasserstoffatom H oder einen linearen oder verzweigten C1- bis C6-Alkylrest oder einen Benzylrest bezeichnet;
(xi) einem Rest -(CH₂)₂-SH;
(xii) einem Rest -(CH₂)₄-NH₂,
unter der Maßgabe, dass die Verbindung (Ia) nicht für die folgenden Verbindungen stehen kann:

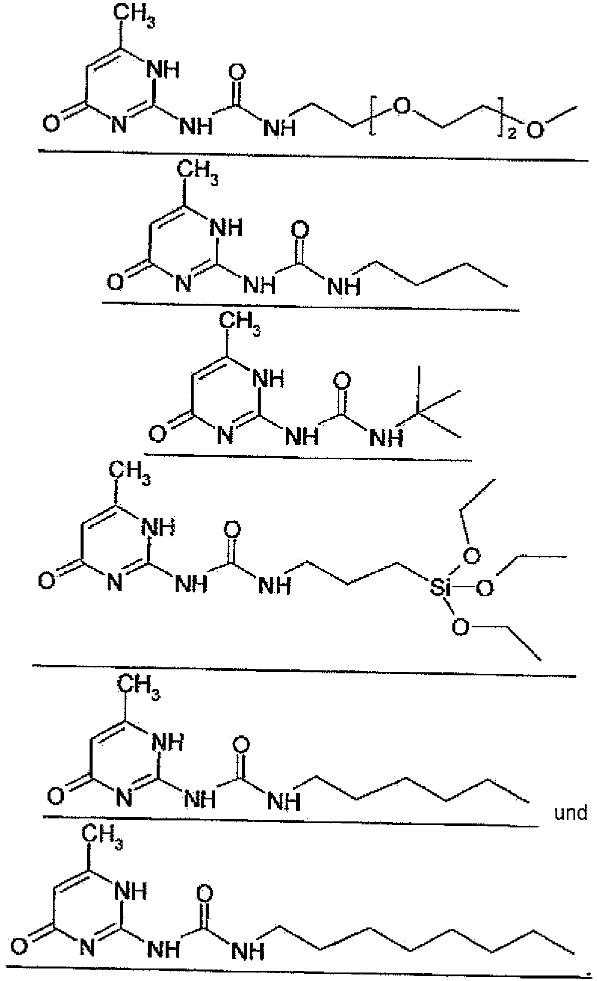

## Claims

1. Cosmetic composition comprising, in a physiologically acceptable medium, at least one compound of formula (I), salts thereof, addition salts thereof, solvates thereof, especially hydrates thereof, the tautomeric forms being included: 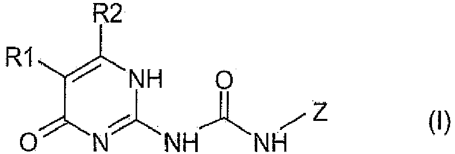 in which:
- R1 and R2, independently of one another, represent H, -OH, -NRR' (with R, R', which may be identical or different, being H or a linear or branched C1-C12 alkyl radical); or a carbon-based group, especially an optionally aromatic, saturated or unsaturated, linear, branched and/or cyclic C1-C18 alkyl group, which may contain one or more heteroatoms O, N and/or S, especially OH, COOH and/or NRR';
- Z represents a monovalent radical chosen from:
(i) a hydrogen atom,
(ii) a linear or branched C1 to C32 (saturated) alkyl radical;
(iii) a linear or branched C2 to C32 (unsaturated) alkene radical, comprising one or two C=C double bonds;
(iv) a C1-C32, especially C2-C24, or even C3-C18, better still C4-C12 alkyl radical, substituted with a C6-C10, especially C6-C8 aryl; in particular phenyl;
it being possible for said radicals to optionally be substituted with 1 to 8 groups chosen from -OH, -OR, -SH, -SR, -SO₃H, -SO₃R, -SO₂NRR',-COOH, -COOR,
-CONRR', -NR-C(O)-NRR', -NRR' and -N⁺RR'R", with R, R' and R" = H or C1-C6 alkyl; and the substituents of formula (a) to (h) below: 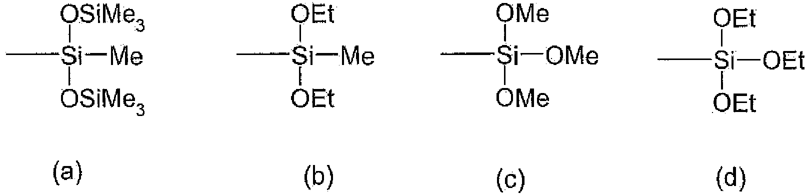 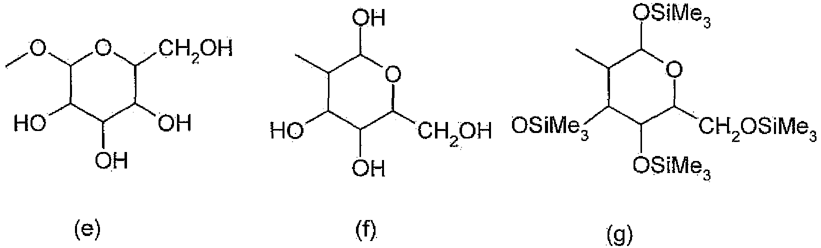 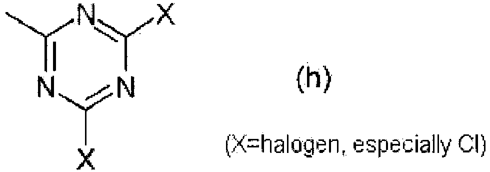 and/or it being possible for said radicals to optionally comprise 1 to 8 groups chosen from, alone or as a mixture, -S-, S(O), SO₂, -NH- (or =NH), -N= (trivalent), -O-, -C(O)-, -C(=NH)-, -N⁺(CH₃)₂-An⁻ (An⁻: anion),
the cosmetically acceptable medium comprising at least one ingredient chosen from propellants, carbon-based oils; C8-C40 alcohols, C8-C40 esters, C8-C40 acids; nonionic surfactants, cationic surfactants, anionic surfactants, amphoteric surfactants, zwitterionic surfactants; sunscreens; antidandruff agents; antioxidants; reducing agents; oxidation bases, couplers, oxidizing agents, direct dyes; hair straightening agents, pearlescent agents and opacifying agents; plasticizers or coalescence agents; hydroxy acids; pigments; fillers; polyols; waxes; thickeners, emulsifiers; polymers.

2. Composition according to Claim 1, in which R1 represents H and R2 represents H, CH₃, C₇H₁₅, C₁₃H₂₇ or aryl.

3. Composition according to either one of the preceding claims, in which the compounds of formula (I) are chosen from the following compounds:

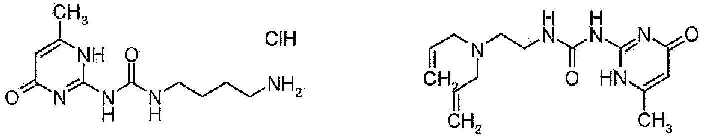

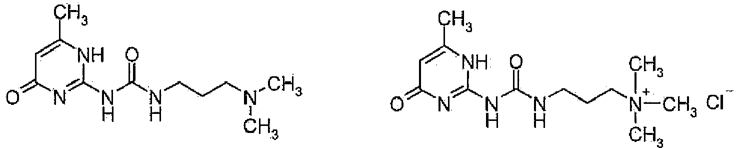

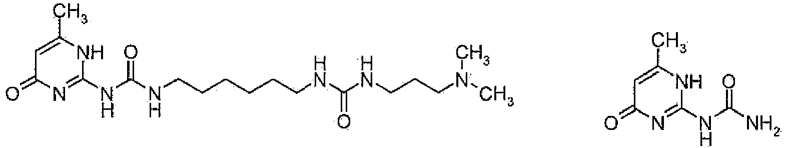

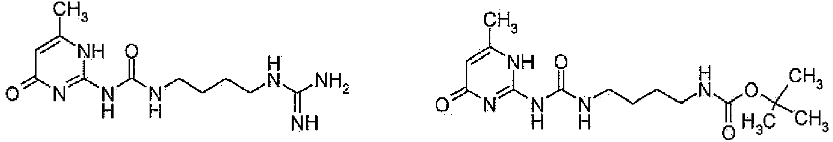

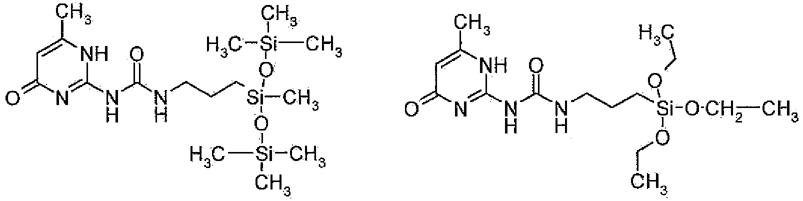

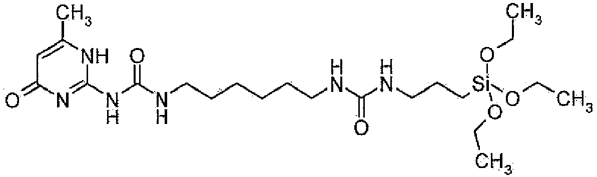

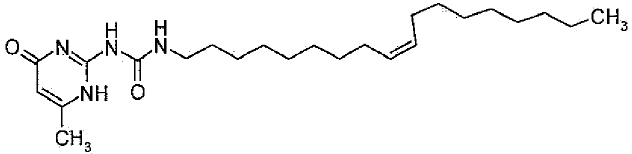

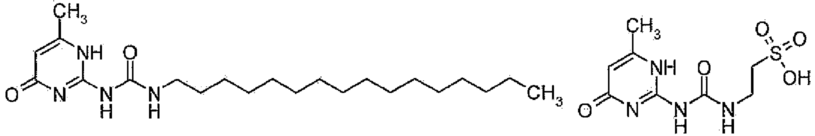

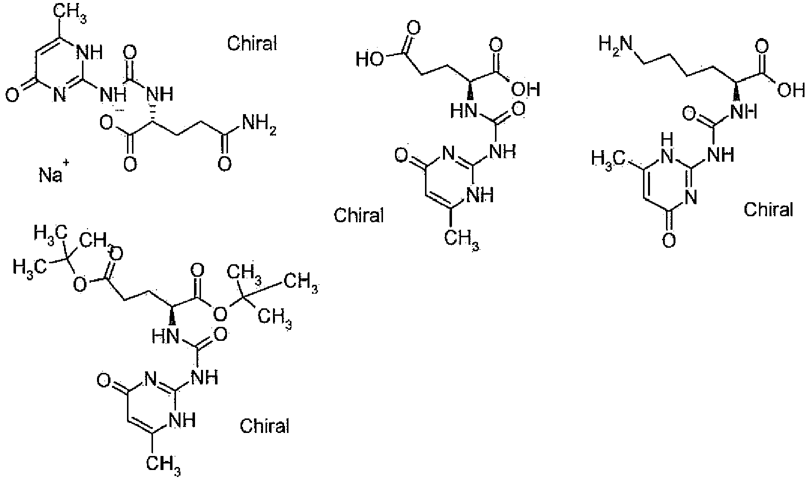

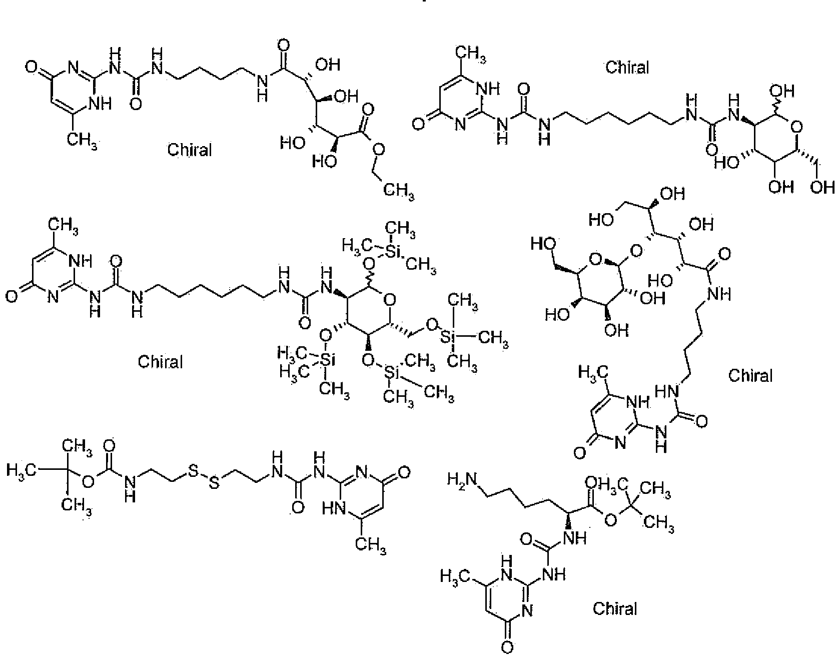

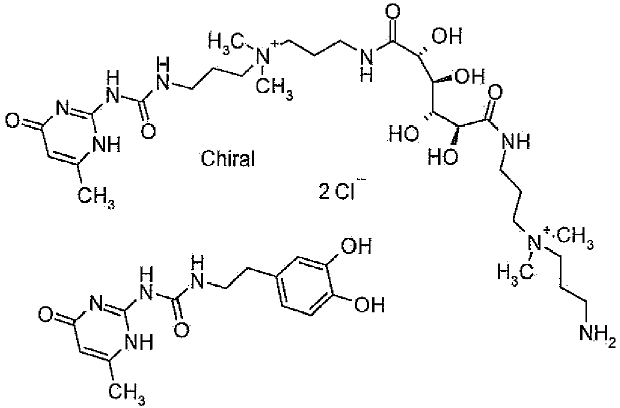

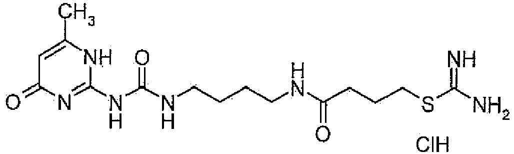

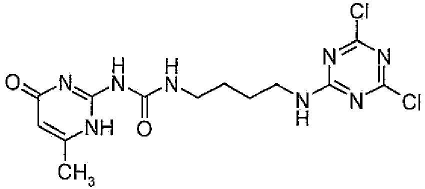

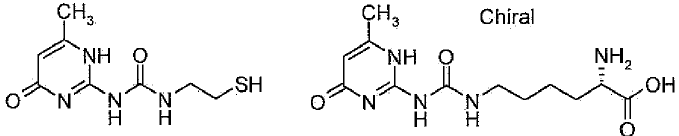

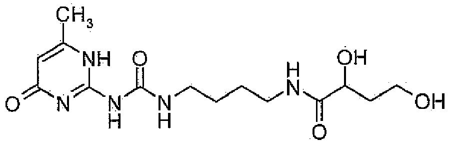

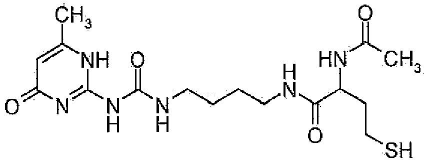

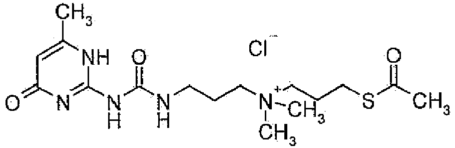

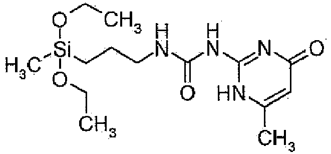

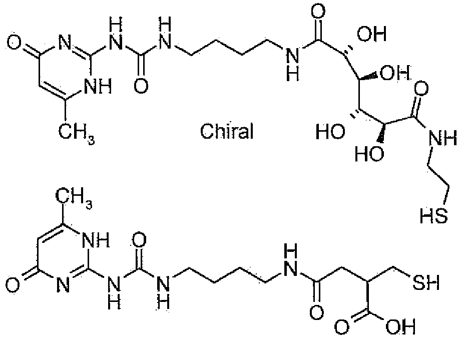

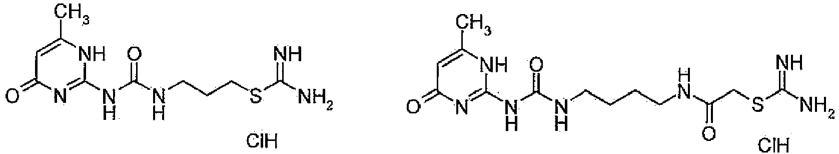

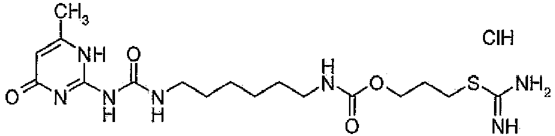

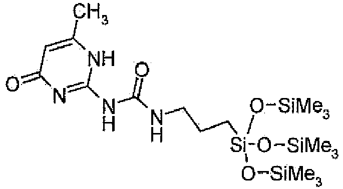

4. Composition according to any one of the preceding claims, in which the compound of formula (I), alone or as a mixture, is present in an amount between 0.001% and 30% by weight, preferably between 0.005% and 15% by weight, especially between 0.01% and 10% by weight, or even between 0.1% and 5% by weight, relative to the total weight of the composition.

5. Composition according to any one of the preceding claims, comprising moreover at least one additional compound of formula (II): 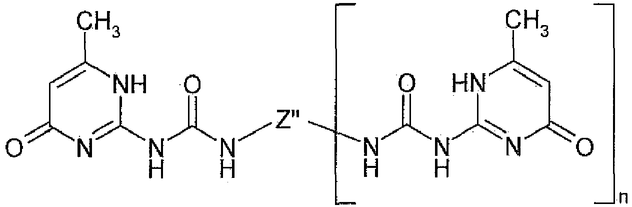 in which:
- n = 1 or 2, and
- Z represents a divalent or trivalent (depending on the value of n), linear or branched C1-C32 alkyl or C2-C32 alkenyl radical;
optionally substituted with 1 to 10 radicals chosen from -OH, -SO₃H, -COOH, -COOR and -N⁺RR'R", with R, R' and R" = C1-C12 alkyl, and/or
optionally interrupted by 1 to 10 groups chosen from (i) the divalent groups: -S-, -NH- (or =NH), -O-,-C(O)-, or of formula: 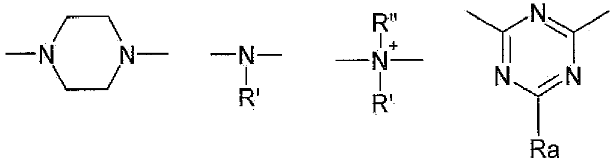
with Ra = H or halogen, or C1-C6 alkyl, or single bond, and
R' and R", which may be identical or different, representing a hydrogen atom or a C1-C6 alkyl radical; and
- (ii) the trivalent groups of formula: 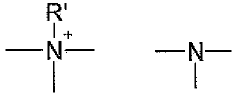 R' representing a hydrogen atom or a C1-C6 alkyl radical.

6. Composition according to Claim 5, in which the compound of formula (II), alone or as a mixture, is present in an amount between 0.001% and 30% by weight, preferably between 0.005% and 15% by weight and especially between 0.01% and 10% by weight, relative to the total weight of the composition.

7. Composition according to any one of the preceding claims, comprises at least one additional ingredient chosen from C1-C7 alcohols, ketones, silicones.

8. Composition according to any one of the preceding claims, which is in the form of a product for caring for, cleansing and/or making up bodily or facial skin, the lips, the eyebrows, the eyelashes, the nails and the hair, an antisun or self-tanning product, a body hygiene product, or a haircare product, especially for caring for, cleansing, conditioning or colouring the hair.

9. Composition according to any one of the preceding claims, which is in the form of a haircare composition, for the care and cosmetic treatment, especially protection, of the hair, in particular of hair that has been weakened and/or damaged, for example by chemical or mechanical treatments.

10. Method for the cosmetic treatment of keratin materials, comprising the application to said materials of a cosmetic composition comprising, in a physiologically acceptable medium, a compound of formula (I) as defined in one of Claims 1 to 4 and optionally a compound of formula (II) as defined in either of Claims 5 and 6.

11. Method according to the preceding claim, in which the cosmetic composition used in a composition according to one of Claims 1 to 9.

12. Method according to Claim 10 or 11, for conditioning and/or caring for the hair, in particular for imparting body and/or liveliness thereto, or improving the disentangling, smoothing, combability, repair and manageability of the head of hair.

13. Kit comprising, on the one hand, a composition for dyeing, bleaching, permanent waving, straightening or smoothing the hair and, on the other hand, a cosmetic composition comprising, in a physiologically acceptable medium, a compound of formula (I) as defined in one of Claims 1 to 4 and optionally a compound of formula (II) as defined in either of Claims 5 and 6.

14. Compound of formula (Ia), salts thereof, addition salts thereof, solvates thereof, especially hydrates thereof, the tautomeric forms being included: 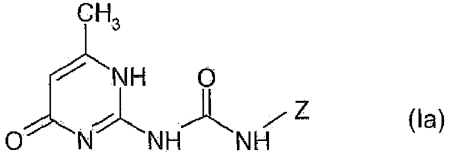 in which Z represents a monovalent radical chosen from:
(i) a non-cyclic, saturated or unsaturated, linear or branched, C1 to C32 hydrocarbon-based radical,
- optionally substituted with 1 to 20 hydroxyl radicals -OH, and/or
- optionally interrupted by 1 to 10 identical or different groups chosen from, alone or combined with one another, -S-, S(O), SO₂, -NH- (or =NH), -N= (trivalent), -O-,
-C(O)-, -CH(OH)-, -C(=NH)-, -N⁺(CH₃)₂-, C6-C10, especially C6-C8, aryl (cyclic aromatic) radical, - terminated with a group chosen from -NR₁R₂,-N⁺R₁R₂R₃ An⁻, -NHC(O)OR₄,
-CH(NH₂)CO₂R₄, -NHC(O)CH(OH)(CH₂)₂OH, in which:
R₁, R₃, independently represent a linear or branched C1-C6 alkyl radical, a hydroxyalkyl radical or an aminoalkyl radical;
R₂ represents a linear or branched C1-C6 alkyl radical, or an aminoalkyl radical;
R₄ represents a linear or branched C1-C6 alkyl radical, or a hydroxyethyl radical;
An⁻ denotes a cosmetically acceptable anion or a mixture of cosmetically acceptable anions;
(ii) a radical of formula (A1): -(CH₂)ₚ-(X)ₙ-(CH₂)_{q}-NR₆-C(=NH)-NR₇R₈
in which X denotes a group chosen from -S-, S(O), SO₂,-NH- (or =NH), -N= (trivalent), -O-, -C(O)-, -C(=NH)-,-C(O)O-, -OC(O)-, -NC(O)-, -NC(O)N-, NSO₂N-, -NR₆-C(=NH)-NR₇-, -N⁺(CH₃)₂- An⁻, a C6-C10, especially C6-C8, aryl (cyclic aromatic) radical;
An⁻ denotes a cosmetically acceptable anion or a mixture of cosmetically acceptable anions;
n, p and q are integers and
n = 0 to 8;
p = 1 to 10;
q = 1 to 10;
p+g ≠ 5.
(iii) a radical of formula (A2): -(CH₂)ᵣ-(T)ₛ-(CH₂)ₜ-(CHR₉)ᵤ-(CH₂)ᵥ- Si R₁₀R₁₁R₁₂
in which T represents a group chosen from the groups:-S-, S(O), SO₂, -NH- (or =NH), -N= (trivalent), -O-,-C(O)-, -C(=NH)-, -N⁺(CH₃)₂- An⁻; a C6-C10 aryl (cyclic aromatic) radical; a carbon-carbon double bond, especially -CH=CH-, a carbon-carbon triple bond, and combinations thereof;
when s, respectively u, is not zero, the adjacent T groups, respectively adjacent -CHR₉- groups, are identical or different;
Rg denotes a radical chosen from the radicals: -OH, -SH, -SO₃H, -COOH, -COOR, -NHC(O)R, NHC(O)OR, -NRR' and-N⁺RR'R" An⁻, in which R, R' and R", which may be identical or different, represent a hydrogen atom H or a linear or branched C1-C6 alkyl radical;
R₁₀, R₁₁, R₁₂ are chosen independently from OR₁₃ radicals or linear or branched C1-C6 alkyl radicals;
R₁₃ denotes a linear or branched C1-C6 alkyl radical or a radical -(CH₂-CH₂-O)_{w}R₁₄ or a radical -SiR₁₅R₁₆R₁₇;
R₁₄ denotes a hydrogen atom H, or a methyl radical.
R₁₅, R₁₆, and R₁₇ independently denote a linear or branched C1-C4 alkyl radical.
An⁻ denotes a cosmetically acceptable anion or a mixture of cosmetically acceptable anions;
r, s, t, u, v, w are integers and
r = 1 to 10;
s, t, u, v = 0 to 10;
w = 1 to 5;
it being understood that when s=u=0 and R₁₀=R₁₁=R₁₂= OR₁₃, then r+t+v ≠ 3;
(iv) a radical of formula (A3): -(CH₂)ₓ-(U)_{y}-(CH₂)_{z}-CH₃ in which U denotes a group chosen from the radicals:-S-, S(O), SO₂, -NH- (or =NH), -N= (trivalent), -O-,-C(O)-, -C(=NH)-, -N⁺(CH₃)₂-An⁻, a C6-C10, especially C6-C8, aryl (cyclic aromatic) radical, a carbon-carbon double bond, especially -CH=CH-, a carbon-carbon triple bond, and also combinations thereof;
An⁻ denotes a cosmetically acceptable anion or a mixture of cosmetically acceptable anions;
x, y and z are integers, between 0 and 10 inclusive,
it being understood that when y = 0, x+z is greater than or equal to 10 and less than or equal to 30 and other than 18, in particular x+z is equal to 15;
and that when y ≠ 0, x is greater than or equal to 2 and less than or equal to 25 and z is greater than or equal to 3 and less than or equal to 25, in particular x+z is equal to 15 with U equal to C=C and y =1;
(v) a radical of formula (A4): -Alk1-(V)ₐ₁-(Alk2)ₐ₂-CH₂-W in which Alk1 and Alk2 independently denote a saturated or unsaturated, linear or branched, C1 to C32 alkyl radical;
V denotes a group chosen from the radicals: -S-, S(O), SO₂, -NH- (or -NH), -N= (trivalent), -O-, -C(O)-,-C(=NH)-, -N⁺(CH₃)₂- An⁻, a C6-C10, especially C6-C8, aryl (cyclic aromatic) radical, a carbon-carbon double bond, especially -CH=CH-, a carbon-carbon triple bond, or combinations thereof;
An⁻ denotes a cosmetically acceptable anion or a mixture of cosmetically acceptable anions;
W denotes a radical chosen from the radicals: -SR₁₅,-S(O)R₁₅, -SO₂R₁₅, -SO₃H, -OSO₃H;
R₁₅ denotes an aromatic C5-C10 (hetero)cycle, 3 carbon atoms of which can be replaced by nitrogen atoms; a carbonyl radical -C(O)R₁₆; a radical -C(=NR₁₇)-NR₁₈R₁₉;
R₁₆ denotes a linear or branched C1-C6 alkyl radical; R₁₇, R₁₈, R₁₉ independently denote a hydrogen atom H or a linear or branched C1-C6 alkyl radical;
R₁₇ and R₁₈ may be connected to one another in order to form, with the nitrogen atoms to which they are attached, a saturated or unsaturated ring or bicyclic ring containing from 5 to 8 atoms;
a1 and a2 are integers;
a1 = 0 to 8, preferably 0 to 2;
a2 = 0 or 1;
(vi) a radical of formula (A5): -Y-(K)j-G
in which Y denotes a saturated or unsaturated, linear or branched C1 to C32 alkyl radical, optionally interrupted by 1 to 8 identical or different groups chosen from -S-, S(O), SO₂, -NH- (or =NH),-N= (trivalent), -O-, -C(O)-, -C(=NH)-,
-N⁺(CH₃)₂- An⁻, a C6-C10 aryl (cyclic aromatic) radical, or combination thereof;
K denotes a group chosen from -CO-, -NHC(O)-,-CH(NRR')-, -CHR-, -C(=CHR)-;
G denotes a group chosen from G1, G2 or G3, where:
- G1 denotes a monosaccharide or a polysaccharide containing up to 20 sugar units, in pyranose and/or furanose form and of L and/or D series, said monosaccharide or polysaccharide optionally having one or more amine functions, the hydroxyl and amine functions being optionally protected; and the optical and/or geometric isomers thereof; it being possible for these monosaccharides to be protected or unprotected at the hydroxyl functions not involved in bonding with the radical H;
- G2 denotes an alpha amino acid radical with a protected or unprotected side chain, chosen from the side chains of the 22 natural amino acids, this radical being C or N branched, racemic or non-racemic, of L or D stereochemistry, protected or unprotected at the C or N residue and at the side chain for the alpha amino acids in question;
- G3 denotes a phenyl radical substituted at any one of the positions with a hydroxyl radical and optionally substituted with a group R₂₀;
R₂₀ denotes a saturated or unsaturated, linear or branched C1 to C6 alkyl radical, a C1 to C6 hydroxyalkyl radical, a C5-C10 aryl (cyclic aromatic) radical, or a radical -OH, -SH, -SO₃H, -COOH,-COOR, -NHC(O)R, NHC(O)OR, -NRR' and
-N⁺RR'R" An⁻;
R, R' and R" independently denote a hydrogen atom H or a C1-C6 alkyl radical;
An⁻ denotes a cosmetically acceptable anion or a mixture of cosmetically acceptable anions;
j is an integer equal to 0 or 1;
(vii) a radical of formula (A6): -(CH₂)_{g}-(J)ₕ-(CH₂)ᵢ-(CHR₂₃)ₖ-CH₂R₂₄
in which J denotes a group chosen from the groups: -S-, S(O), SO₂, -NH- (or =NH), -N= (trivalent), -O-, -C(O)-, -C(=NH)-, -N⁺(CH₃)₂- An⁻, a C6-C10, especially C6-C8, aryl (cyclic aromatic) radical, a carbon-carbon double bond, especially -CH=CH-, a carbon-carbon triple bond, or combinations thereof;
R₂₃ denotes a radical -OR₂₅, -SH, -SO₃H, -COOH, -COOR,-NHC(O)R, NHC(O)OR, -NRR' and -N⁺RR'R" An⁻, with R, R' and R" = H or C1-C6 alkyl, especially methyl;
R₂₄ denotes a hydrogen atom H, or a hydroxyl radical-OH;
R₂₅ denotes a hydrogen atom H, or a monosaccharide or a polysaccharide containing up to 20 sugar units, in pyranose and/or furanose form and of L and/or D series, said monosaccharide or polysaccharide optionally having one or more amine functions, the hydroxyl and amine functions being optionally protected; and the optical and/or geometric isomers thereof.
An⁻ denotes a cosmetically acceptable anion or a mixture of cosmetically acceptable anions;
g, h, i, k denote an integer;
g, h, k = 1 to 10;
i = 0 to 10;
(viii) a radical of formula (A7): -(CH₂)_{b}-(A)-(B)-(A)_{c}-(CH₂)_{d}-D
in which A denotes a group chosen from -NHC(O)-,-C(O)NH-, -NHC(O)NH-,
-C(O)O-, -OC(O)-;
B denotes a branched or unbranched hydrocarbon-based chain, optionally substituted with a radical chosen from -OH, -COOH, -NHC(O)R₂₁;
R₂₁ denotes a linear or branched C1-C6 alkyl radical;
D denotes a radical -SH or a linear or branched C1 to C4 alkyl radical;
b, c and d are integers;
b = 2 to 10, preferably 3 to 6;
c = 0 or 1;
d = 0 to 10, preferably 0 to 3;
(ix) a radical of formula (A8): -Alk3-S-S-Alk4
in which Alk4 denotes a linear or branched C2 to C20 hydrocarbon-based radical, optionally comprising one or more double bonds, and optionally comprising one or more heteroatoms chosen from O or N, and optionally substituted with one or more identical or different radicals chosen from -OH, -SO₃H, -COOH, -COOR, -NHC(O)R, NHC(O)OR, -NRR' and -N⁺RR'R" An⁻.
Alk3 denotes a linear or branched C2 to C20 hydrocarbon-based radical, optionally comprising one or more double bonds, and optionally comprising one or more heteroatoms chosen from O or N, and optionally substituted with one or more identical or different radicals chosen from -OH, -SO₃H, -COOH, -COOR, -NHC(O)R, -NRR' and -N⁺RR'R" An⁻.
R, R' and R" independently denote a hydrogen atom H or a linear or branched C1-C6 alkyl radical;
An⁻ denotes a cosmetically acceptable anion or a mixture of cosmetically acceptable anions;
(x) a radical of formula (A9): -CHL-COOR₂₂
in which L denotes the protected or unprotected side chain of an alpha amino acid, chosen from the side chains of the 22 natural amino acids; the stereochemistry is L, D or a racemic mixture;
R₂₂ denotes a hydrogen atom H, or a linear or branched C1 to C6 alkyl radical, or a benzyl radical;
(xi) a radical -(CH₂)₂-SH;
(xii) a radical - (CH₂)₄-NH₂;
it being understood that the compound (Ia) cannot represent the following compounds:
